# FASCICULE DE BREVET EUROPEEN

(11) **EP 1 115 720 B1**
(45) Date de publication et mention de la délivrance du brevet: **02.07.2008**
(21) Numéro de dépôt: 99943025.9
(22) Date de dépôt: 22.09.1999
(51) Int. Cl.: C07D 333/38, A61K 31/38, C07D 409/12, C07D 417/12

(54) **DERIVES DE N-(IMINOMETHYL)AMINES, LEUR PREPARATIONLEUR APPLICATION A TITRE DE MEDICAMENTS ET LES COMPOSITI ONS PHARMACEUTIQUES LES CONTENANT**
N-(IMINOMETHYL)AMIN-DERIVATE, IHRE HERSTELLUNG, IHRE VERWENDUNG IN MEDIKAMENTEN UND SIE ENTHALTENDE PHARMAZEUTISCHE ZUBEREITUNGEN
NOVEL N-(IMINOMETHYL)AMINE DERIVATIVES, THEIR PREPARATION, THEIR USE AS MEDICINES AND COMPOSITIONS CONTAINING THEM

(30) Priorité: 23.09.1998 FR 9811867
(43) Date de publication de la demande: 18.07.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif-sur-Yvette (FR); CHABRIER DE LASSAUNIERE, Pierre-Etienne, F-75016 Paris (FR); AUVIN, Serge, F-91370 Mauchamps (FR); HARNETT, Jeremiah, F-91190 Gif-sur-Yvette (FR); ULIBARRI, Gérard, F-91440 Bures-sur-Yvette (FR)
(74) Mandataire: Bourgouin, André
(86) Numéro de dépôt international: PCT/FR1999/002251
(87) Numéro de publication internationale: WO 2000/017191

(56) Documents cités:
- EP-A- 0 798 292
- WO-A-95/00505
- WO-A-95/05363
- WO-A-98/42696
- OBRENOVITCH, A. ET AL: "Sensitive fluorometric determination of plasminogen activator in cell lysates and supernatants" FEBS LETT. (1983), 157(2), 265-70 CODEN: FEBLAL;ISSN: 0014-5793, XP002107910
- PH. E. BROWN ET AL.: "Studies of Chromenes. Part 10. Oxiranes of Nitrochromenes" JOURNAL OF THE CHEMICAL SOCIETY, PERKIN TRANSACTIONS 1, 1992, pages 573-577, XP002143910 LETCHWORTH GB

## Description

La présente invention a pour objet des nouveaux dérivés de N-(iminométhyl)amines comportant dans leur squelette le motif aminodiphénylamine, oxodiphénylamine, carbazole, phénazine, phénothiazine, phénoxazine ou oxodiphényle. Ces dérivés présentent une activité inhibitrice des enzymes NO-synthases produisant le monoxyde d'azote NO et une activité piégeuse des formes réactives de l'oxygène (ROS pour *"reactive oxygen species* "). L'invention concerne les dérivés correspondant à la formule générale **(I)** définie ci-après, leurs méthodes de préparation, les préparations pharmaceutiques les contenant et leur utilisation à des fins thérapeutiques, en particulier leur utilisation en tant qu'inhibiteurs des NO-synthases et piégeurs de formes réactives de l'oxygène de manière sélective ou non.

Compte tenu du rôle potentiel du NO et des ROS en physiopathologie, les nouveaux dérivés décrits répondant à la formule générale (I) peuvent produire des effets bénéfiques ou favorables dans le traitement de pathologies où ces espèces chimiques sont impliquées. Notamment :
• Les maladies inflammatoires et prolifératives comme par exemple l'athérosclérose, l'hypertension pulmonaire, la détresse respiratoire, la glomérulonéphrite, l'hypertension portale, le psoriasis, l'arthrose et l'arthrite rhumatoïde, les fibroses, l'angiogenèse, les amyloïdoses, les inflammations du système gastro-intestinal (colite ulcérative ou non, maladie de Crohn), les diarrhées.
• Les maladies affectant le système pulmonaire ou les voies aériennes (asthme, sinusites, rhinites).
• Les maladies cardio-vasculaires et cérébro-vasculaires comprenant par exemple la migraine, l'hypertension artérielle, le choc septique, les infarctus cardiaques ou cérébraux d'origine ischémique ou hémorragiques, les ischémies et les thromboses.
• Les troubles du système nerveux central ou périphérique comme par exemple les maladies neurodégénératives où l'on peut notamment citer les infarctus cérébraux, l'hémorragie sub arachnoïde, le vieillissement, les démences séniles, y compris la maladie d' Alzheimer, la chorée de Huntington, la maladie de Parkinson, la maladie de Creutzfeld Jacob et les maladies à prions, la sclérose latérale amyotrophique ; les neuropathies oculaires comme le glaucome mais aussi la douleur, les traumatismes cérébraux ou de la moelle épinière, l'addiction aux opiacées, à l'alcool et aux substances induisant une accoutumance, les désordres cognitifs, les encéphalopathies, les encéphalopathies d'origine virale ou toxique.
• Les troubles du muscle squelettique et des jonctions neuromusculaires (myopathie, myosite) ainsi que les maladies cutanées.
• La cataracte.
• Les transplantations d'organes.
• Les maladies auto-immunes et virales comme par exemple le lupus, le sida, les infections parasitaires et virales, le diabète et ses complications, la sclérose en plaques.
• Le cancer.
• Les maladies neurologiques associées à des intoxications (empoisonnement au Cadmium, inhalation de n-hexane, pesticide, herbicide), à des traitements (radiothérapie) ou à des désordres d'origine génétique (maladie de Wilson).
• Toutes les pathologies caractérisées par une production excessive ou un dysfonctionnement de NO et/ou des ROS.

Dans l'ensemble de ces pathologies, il existe des évidences expérimentales démontrant l'implication du NO ou des ROS (J. Med. Chem. (1995) 38, 4343-4362 ; Free Radic. Biol. Med. (1996) 20, 675-705 ; The Neuroscientist (1997) 3, 327-333).

Par ailleurs les inventeurs ont déjà décrit dans des brevets antérieurs des inhibiteurs de NO Synthases et leur utilisation (brevets US 5,081,148 ; US 5,360,925), et plus récemment l'association de ces inhibiteurs avec des produits possédant des propriétés antioxydantes ou antiradicalaires (demande de brevet PCT WO 98/09653). Ils ont aussi décrit dans des demandes non encore publiées d'autres dérivés d'amidines ou, plus récemment, des dérivés d'aminopyridines. Ces dérivés d'amidines ou d'aminopyridines présentent la particularité d'être à la fois des inhibiteurs de NO Synthases et des inhibiteurs de ROS.

La demande de brevet PCT WO 95/05363 décrit des composés inhibiteurs de NO Synthases de formule générale **(A1)** 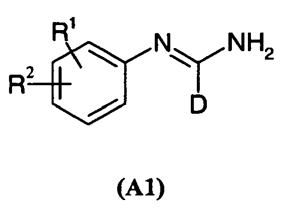 dans laquelle :
D représente phényle, pyridinyle ou un hétérocycle aromatique à 5 chaînons contenant de 1 à 4 hétéroatomes choisis parmi O N et S, ces trois groupes étant éventuellement substitués par un ou des groupes choisis parmi (C₁-C₆)alkyle, (C₁-C₆)alkoxy, halogène, (C₁-C₆)perfluoroalkyle, ou D représente (C₁-C₆)perfluoroalkyle ;
R¹ représente hydrogène, (C₁-C₆)alkyle ou halogène ;
R² représente -X(CH₂)ₙZCONR³R⁴, -X(CH₂)ₙNHCO(CH₂)ₛNR³R⁴, -X(CH₂)ₚNR³R⁴, -X(CH₂)ₙNHCOR⁵ ou -(CH₂)_{q}NHC(NH)R⁶,
R³ et R⁴ représentent indépendamment hydrogène, (C₁-C₆)alkyle, -(CH₂)ᵣ-A, -(CH₂)ₘOA, ou -CH(CH₃)(CH₂)ₜA ;
ou l'ensemble NR³R⁴ représente 1-indanyle, pipéronylamino, pipéridynyle, morpholinyle, pyrrolidinyle, 1,2,3,4-tétrahydroisoquinolinyle ou pipérazinyle éventuellement substitué en position 4 par (C₁-C₆)alkyle ;
R⁵ représente (C₁-C₆)alkyle, (C₁-C₆)perfluoroalkyle, -(CH₂)ᵣ-A ou -O(CH₂)_{w}A ;
A représente phényle, pyridinyle, pyrimidinyle ou hétérocycle aromatique à 5 chaînons contenant de 1 à 4 hétéroatomes choisis parmi O, N et S, ces 4 groupes étant éventuellement substitués par un ou des groupes choisis parmi (C₁-C₆)alkyle, halogène, nitro, cyano et trifluorométhyle ;
R⁶ représente phényle, pyridinyle ou un hétérocycle aromatique à 5 chaînons contenant de 1 à 4 hétéroatomes choisis parmi O, N et S, ces trois groupes étant éventuellement substitués par un ou des groupes choisis parmi (C₁-C₆)alkyle, (C₁-C₆)alkoxy, halogène, (C₁-C₆)perfluoroalkyle, ou R⁶ représente (C₁-C₆)perfluoroalkyle ;
n et r représentent indépendamment des entiers de 0 à 6 ;
p et w représentent indépendamment des entiers de 1 à 5 ;
m représente un entier de 2 à 5 ;
q et t représentent indépendamment des entiers de 0 à 5 ;
s représente un entier de 1 à 3 ;
X représente O ou une liaison ;
Z représente O, NR⁷ ou une liaison ;
R⁷ représente hydrogène ou (C₁-C₆)alkyle ;
étant entendu que :
   (a) D, lorsque qu'il contient un hétéroatome, n'est pas relié au reste du composé de formule **(A1)** par l'hétéroatome ;
   (b) lorsque R² représente -X(CH₂)ₙZCONR³R⁴ et ni X ni Z ne représentent une liaison, alors n représente un entier de 2 à 6 ;
   (c) lorsque R² représente -X(CH₂)ₙNHCO(CH₂)ₛNR³R⁴ ou -X(CH₂)ₙNHCOR⁵ et X représente O, alors n représente un entier de 2 à 6 ;
   (d) lorsque R² représente -X(CH₂)ₚNR³R⁴ et X représente O, alors p représente un entier de 2 à 5 ;
   (e) lorsque R² représente -(CH₂)_{q}NHC(NH)R⁶, R¹ représente hydrogène, D représente phényle et R⁶ représente phényle, alors q ne représente pas 0 ;
   (f) lorsque R² représente -(CH₂)_{q}NHC(NH)R⁶, R¹ représente hydrogène, D et R⁶ représentent 2-chlorophényle, alors q ne représente pas 0 ;
   (g) lorsque R² représente -(CH₂)_{q}NHC(NH)R⁶, R¹ représente hydrogène, D et R⁶ représentent 3-pyridinyle, alors q ne représente pas 0 ; et
   (h) lorsque R² représente -(CH₂)_{q}NHC(NH)R⁶, R¹ représente hydrogène, D et R⁶ représentent 4-pyridinyle, alors q ne représente pas 0.

La présente invention a pour objet de nouveaux dérivés d'amidines, leur préparation et leur application en thérapeutique.

Les composés de l'invention répondent à la formule générale **(I):** 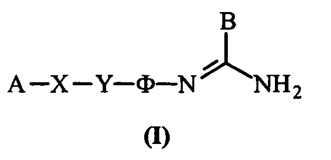 dans laquelle
Φ représente un radical phénylène pouvant compter, outre les deux chaînes déjà représentées dans la formule générale (I), jusqu'à deux substituants choisis parmi un atome d'hydrogène, un halogène, un groupe OH, et un radical alkyle ou alkoxy linéaire
ou ramifié ayant de 1 à 6 atomes de carbone ;
A représente un radical 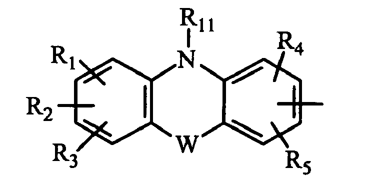 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 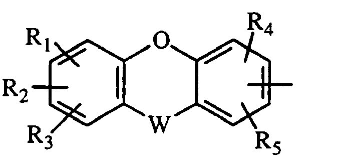 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire
ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone ;
B représente -CH₂-NO₂, un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
ou B représente un radical NR₁₃R₁₄, dans lequel R₁₃ et R₁₄ représentant, indépendamment, un atome d'hydrogène ou un radical alkyle linéaire ou ramifié ayant de 1 à 6 atomes de carbone ou un radical cyano ou nitro, ou R₁₃ et R₁₄ forment avec l'atome d'azote un hétérocycle non aromatique de cinq à six chaînons, les éléments de la chaîne étant choisis parmi un groupe composé de -CH₂-, -NH-, -O- ou -S- ;
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
X représente une liaison ou un radical (CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-,
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine,
m et n étant des entiers de 0 à 6 ;
R₁₆, R₁₇ et R₁₈ représentent, indépendamment, un atome d'hydrogène ou un radical alkyle linéaire ou ramifié ayant de 1 à 6 atomes de carbone ;
ou sont des sels des produits précédemment mentionnés.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par alkoxy linéaire ou ramifié ayant de 1 à 6 atomes de carbone, on entend les radicaux dont le radical alkyle a la signification indiquée précédemment. Enfin, par halogène, on entend les atomes de fluor, de chlore, de brome ou d'iode.

De préférence, les composés selon l'invention seront des composés de formule générale (I) tels que :
A représente un radical 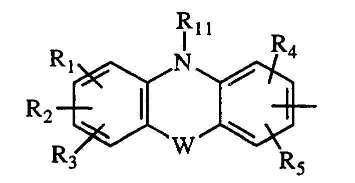 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone,
   R₁₁ représentant un atome d'hydrogène ou un radical alkyle linéaire ou ramifié ayant de 1 à 6 atomes de carbone,
   ou un radical 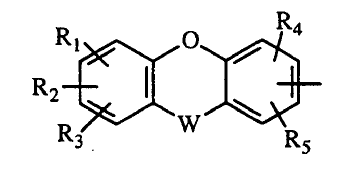 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone ;
B représente un radical aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;
   W n'existe pas, ou représente une liaison, S ou NR₁₅, dans lequel R₁₅ représente un atome d'hydrogène ou un radical alkyle linéaire ou ramifié ayant de 1 à 6 atomes de carbone ;
   X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-, k représentant 0 ou 1;
   Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-. -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-, Q représentant pipérazine, homopipérazine, 2-méthylpipérazine, 2,5-diméthylpipérazine, 4-oxypipéridine ou 4-aminopipéridine,
   m et n étant des entiers de 0 à 6 ;
   ou sont des sels des produits précédemment mentionnés.

Plus préférentiellement, les composés selon l'invention seront des composés de formule générale (I) tels que :
A représente un radical 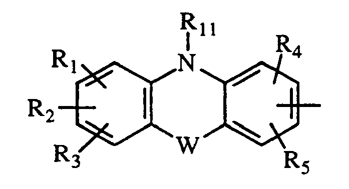 dans lequel R₁, R₂, R₃, R₄ et R₅ représentent, indépendamment, un atome d'hydrogène, le groupe OH ou un radical alkyle ou alkoxy linéaire ou ramifié ayant de 1 à 6 atomes de carbone,
   R₁₁ représentant un atome d'hydrogène ou un radical méthyle,
   ou un radical 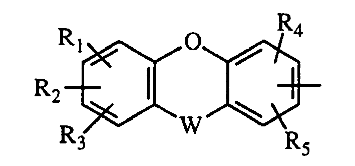 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone ;
B représente l'un des radicaux phényle, thiophène, furanne, pyrrole ou thiazole éventuellement substitués par un ou plusieurs groupes choisis parmi les radicaux alkyle, alkényle ou alkoxy linéaires ou ramifiés ayant de 1 à 6 atomes de carbone ;
   W n'existe pas, ou représente une liaison, S ou NR₁₅, dans lequel R₁₅ représente un atome d'hydrogène ou un radical alkyle linéaire ou ramifié ayant de 1 à 6 atomes de carbone ;
   X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-, k représentant 0 ou 1 ;
   Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-, Q représentant pipérazine, homopipérazine, 2-méthylpipérazine, 2,5-diméthylpipérazine, 4-oxypipéridine ou 4-aminopipéridine, m et n étant des entiers de 0 à 6 ;
   ou sont des sels des produits précédemment mentionnés.

Encore plus préférentiellement, les composés selon l'invention seront des composés de formule générale (I) tels que :
A représente un radical 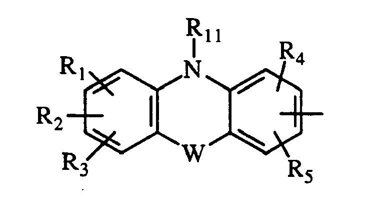 dans lequel R₁, R₂, R₃, R₄, R₅ représentent, indépendamment, un atome d'hydrogène ou un radical méthyle,
   R₁₁ représentant un atome d'hydrogène ou un radical méthyle ;
B représente le radical thiophène ;
   W n'existe pas, représente une simple liaison ou S ;
   X n'existe pas ou représente un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NP₁₇-, k représentant 0 ou 1 ;
   Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
   Q représentant pipérazine,
   m et n étant des entiers de 0 à 6 ;
   R₁₆, R₁₇ et R₁₈ représentent un atome d'hydrogène ;
   ou sont des sels des produits précédemment mentionnés.

Seront tout particulièrement préférés les composés suivants décrits dans les exemples :
-4-{[2-thiényl(imino)méthyl]amino}-N-[4-(phénylamino)phényl]-benzèneacétamide;
- {4-{[2-thiényl(imino)méthyl]amino}phénoxy}-N-[4-(phénylamino)phényl]-acétamide ;
- 4-{[2-thiényl(imino)méthyl]amino}-N-[2-(phénylamino)phényl]-benzènebutanamide ;
- 4-{[2-thiényl(imino)méthyl]amino}-N-[4-(phénylamino)phényl]-benzènebutanamide ;
- 4- {[2-thiényl(imino)méthyl]amino}-N-[4-(4-méthoxyphénylamino)phényl]-benzénebutanamide ;
- [4-(phénylamino)phényl]-carbamate de 2-{4-{[2-thiényl(imino)méthyl]amino}phényl}-éthyle ;
- N- {2-{4-{[2-thiényl(imino)méthyl]amino}phényl}éthyl}-N'-[4-(phénylamino)phényl]-urée ;
- 4-{4-{[2-thiényl(imino)méthyl]amino}phényl}-N-[4-(phénylamino)phényl]-1-pipérazine acétamide ;
- 1-{[(4-phénylamino)phénylamino]carbonyl}-4-{4-{[2-thiényl(imino)méthyl] amino}phényl}-pipérazine ;
- 4-{[2-thiényl(imino)méthyl]amino}-N-[4-(phénylamino)phényl]-benzènebutanamine ;
- 3-{[2-thiényl(imino)méthyl]amino}-N-[4-(phénylamino)phényl]-benzènepropanamide ;
- 4-(4-{[amino(2-thiényl)méthylidène]amino}phényl)-N-[2-(4-toluidino)phényl]butanamide ;
- 4-anilinophényl-4-(4-{[amino(2-thiényl)méthylidène]amino}-phényl)butanoate ;
- 4-(4-{[amino(2-thiényl)méthylidène]amino}phényl)-N-[2-(4-toluidino)phényl]butanamide ;
- N'-{4-[4-(3-anilinophénoxy)butyl]phényl}-2-thiophènecarboximidamide ;
- N'-(9*H*-carbazol-3-yl)-2-thiophènecarboximidamide ;
- 4-(4-{[amino(2-thiényl)méthylidène]amino}phényl)-N-(9*H*-carbazol-3-yl)butanamide ;
- N'-[4-(10*H*-phénothiazin-2-yloxy)phényl]-2-thiophènecarboximidamide ;
- N'-{4-[(10-méthyl-10*H*-phénothiazin-2-yl)oxy]phényl}-2-thiophènecarboximidamide ;
- 4-(4- {[amino(2-thiényl)méthylidène]amino}phényl)-N-(10*H*-phénothiazin-3-yl)butanamide ;
- N'-(4-{4-[2-(10*H*-phénothiazin-2-yloxy)éthyl]-1-pipérazinyl}phényl)-2-thiophènecarboximidamide ;
- 4-(4- {[amino(2-thiényl)méthylidène]amino} phényl)-N-[4-(4-toluidino)phényl]butanamide ;
- 3-anilinophényl 4-(4-{[amino(2-thiényl)méthylidène]amino}-phényl)butanoate ;
- 2-(4-{[amino(2-thiényl)méthylidène]amino}phényl)-N-[2-(9H-carbazol-4-yloxy)éthyl]acétamide ;
- N-(4-{[amino(2-thiényl)méthylidène]amino}phenéthyl)-2-anilinobenzamide ;
- N-(4-{[amino(2-thiényl)méthylidène]amino}phénéthyl)-2-(2,3-diméthylanilino)benzamide ;
- N'-{4-[4-(2-anilinobenzoyl)-1-pipérazinyl]phényl}-2-thiophènecarboximidamide ;
- N'-(4- {4-[2-(2,3-diméthylanilino)benzoyl]-1-pipérazinyl}phényl)-2-thiophènecarboximidamide ;
- 4-(4-{[amino(2-thiényl)méthylidène]amino}phényl)-N-(4-phénoxyphényl)butanamide ;
- N-(4- {[amino(2-thiényl)méthylidène]amino}phénéthyl)-4-(4-hydroxyphénoxy)benzamide ;
- N-{4-[4-(10*H*-phénothiazin-2-yloxy)butyl]phényl}-2-thiophènecarboximidamide ;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidamide ;
- N-(4-{[amino(2-thiényl)méthylidène]amino}phénéthyl)-2-méthoxy-10*H-*phénothiazine-1-carboxamide ;
- N'-[4-(2-{[(2-méthoxy-10*H*-phénothiazin-1-yl)méthyl]amino}éthyl)phényl]-2-thiophènecarboximidamide ;
-N'-{4-[(10*H*-phénothiazin-2-yloxy)méthyl]phényl}-2-thiophènecarboximidamide ;
ou leurs sels.

Parmi les composés exemplifiés, on préférera particulièrement les composés suivants :
- {4-{[2-thiényl(imino)méthyl]amino}phénoxy}-N-[4-(phénylamino)phényl]-acétamide ;
-4-{[2-thiényl(imino)méthyl]amino}-N-[2-(phénylamino)phényl]-benzènebutanamide;
- 4-{[2-thiényl(imino)méthyl]amino}-N-[4-(phénylamino)phényl]-benzènebutanamide;
- [4-(phénylamino)phényl]-carbamate de 2-{4-{[2-thiényl(imino)méthyl]amino}phényl}-éthyle ;
-4-{4-{[2-thiényl(imino)méthyl]amino}phényl}-N-[4-(phénylamino)phényl]-1-pipérazine acétamide ;
- 3-{[2-thiényl(imino)méthyl]amino}-N-[4-(phénylamino)phényl]-benzènepropanamide;
- 4-(4-{[amino(2-thiényl)méthylidène]amino}phényl)-N-[2-(4-toluidino)phényl]butanamide;
- N'-{4-[4-(3-anilinophénoxy)butyl]phényl}-2-thiophènecarboximidamide;
- 4-(4-{[amino(2-thiényl)méthylidène]amino}phényl)-N-(9*H*-carbazol-3-yl)butanamide;
- N'-[4-(10*H*-phénothiazin-2-yloxy)phényl]-2-thiophènecarboximidamide;
- 4-(4-{[amino(2-thiényl)méthylidène]amino}phényl)-N-(10*H*-phénothiazin-3-yl)butanamide ;
- N'-(4-{4-[2-(10*H*-phénothiazin-2-yloxy)éthyl]-1-pipérazinyl}phényl)-2-thiophènecarboximidamide ;
- 4-(4-{[amino(2-thiényl)méthylidène]amino}phényl)-N-(4-phénoxyphényl)butanamide ;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidanùde ;
- N-(4-{[amino(2-thiényl)méthylidène]amino}phénéthyl)-2-méthoxy-10*H-*phénothiazine-1-carboxamide ;
ou leurs sels.

On préférera encore plus particulièrement les composés suivants :
-4-{[2-thiényl(imino)méthyl]amino}-N-[2-(phénylamino)phényl]-benzènebutanamide;
- 4-{[2-thiényl(imino)méthyl]amino}-N-[4-(phénylamino)phényl]-benzènebutanamide;
- N'-[4-(10*H*-phénothiazin-2-yloxy)phényl]-2-thiophènecarboximidamide ;
- 4-(4-{[amino(2-thiényl)méthylidène] amino}phényl)-N-(10*H*-phénothiazin-3-yl)butanamide ;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidamide ;
ou leurs sels.

D'une façon générale, seront préférés les composés de formule générale (I) dans lesquels X représente une liaison ou l'un des radicaux -O-, -CH₂-NR₁₆-, -NR₁₆-CO- ou -NR₁₆-CO-O- et Y représente l'un des radicaux -(CH₂)ₘ- ou -(CH₂)ₘ-NR₁₈-(CH₂)ₙ-.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes éniantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention concerne aussi, à titre de produits industriels nouveaux, les intermédiaires de synthèse de formule générale (IS), utiles à la préparation de produits de formule générale (**I**) définie plus haut,

A-X-Y-Φ-T₂ **(IS)**

formule générale (IS) dans laquelle
A représente un radical 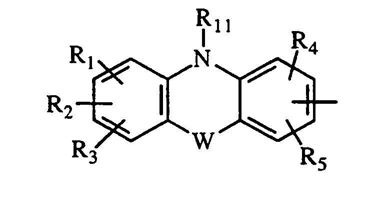 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire
ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 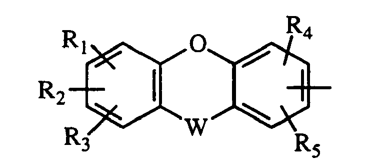 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire
ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone ;
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-,
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine, m et n étant des entiers de 0 à 6 ;
Φ représente un radical phénylène pouvant compter, outre les deux chaînes déjà représentées dans la formule générale **(I)**, jusqu'à deux substituants choisis parmi un atome d'hydrogène, un halogène, un groupe OH, et un radical alkyle ou alkoxy linéaire
ou ramifié ayant de 1 à 6 atomes de carbone ;
T représente NO₂ ou NH₂ ;
R₁₆, R₁₇ et R₁₈ représentent, indépendamment, un atome d'hydrogène ou un radical alkyle linéaire ou ramifié ayant de 1 à 6 atomes de carbone.
L'invention concerne de plus, à titre de produits industriels nouveaux, les intermédiaires de synthèse de formule générale **(IS'),** utiles à la préparation de produits de formule générale **(I)** dans lesquels X représente le radical -NR₁₆-CO- et Y représente le radical - (CH₂)ₘ-NR₁₈-(CH₂)ₙ-, 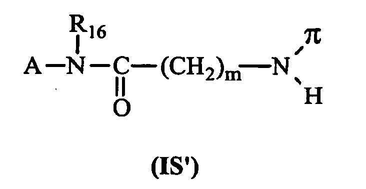 formule générale (**IS'**) dans laquelle
A représente un radical 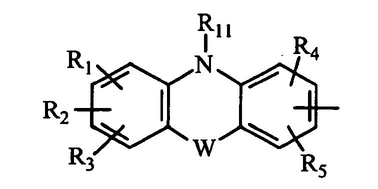 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire
ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 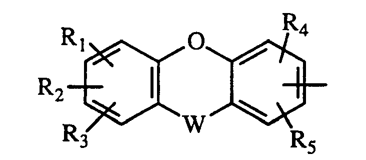 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire
ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone ;
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
π représente un atome d'hydrogène ou un groupe protecteur de type carbamate ;
R₁₆, R₁₇ et R₁₈ représentent, indépendamment, un atome d'hydrogène ou un radical alkyle linéaire ou ramifié ayant de 1 à 6 atomes de carbone ;
et m représente un entier de 0 à 6.

L'invention a également pour objet, à titre de médicaments, les composés de formule générale **(I)** décrits précédemment ou leurs sels pharmaceutiquement acceptables. Elle concerne aussi des compositions pharmaceutiques contenant ces composés ou leurs sels pharmaceutiquement acceptables, et l'utilisation de ces composés ou de leurs sels pharmaceutiquement acceptables pour fabriquer des médicaments destinés à inhiber la NO synthase neuronale ou la NO synthase inductible, à inhiber la péroxidation lipidique ou à assurer la double fonction d'inhibition de la NO synthase et d'inhibition de la péroxidation lipidique.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", J. Pharm. Sci. 66:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par le procédé décrit ci-dessous.

### Préparation des composés de formule générale (I) :

Les composés de formule générale **(I)** peuvent être préparés à partir des intermédiaires de formule générale **(II)** selon le schéma 1 où A, B, X, Y et Φ sont tels que définis ci-dessus et Gp est un groupe protecteur de type carbamate tel que par exemple le groupe t-butoxycarbonyle.

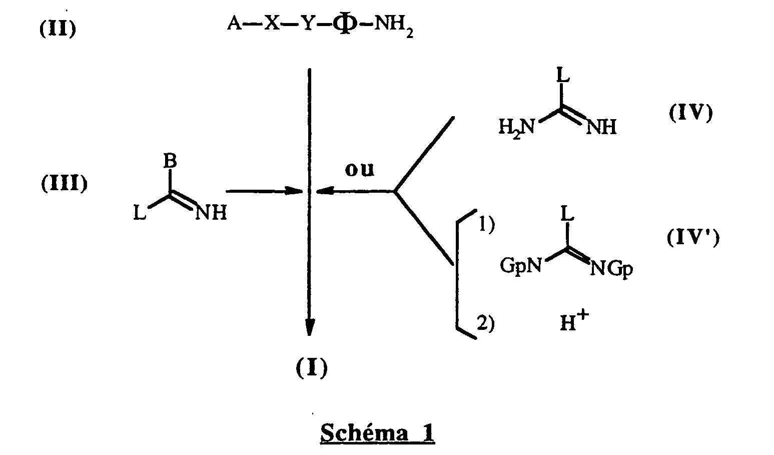

Les dérivés d'aniline de formule générale (**II**), peuvent être condensés sur des composés de formule générale (**III**), dans lesquels L représente un groupe partant (par exemple un radical alkoxy, alkylthio, aralkylthio, acide sulfonique, halogénure, alcool arylique ou tosyle), pour conduire aux composés finaux de formule générale **(I)** de type amidine substituée (cf. schéma 1). Par exemple, pour B = thiophène, on peut condenser les dérivés de formule générale **(II)** sur l'iodhydrate de S-méthylthiophène thiocarboxamide, préparé selon une méthode de la littérature *(*Ann. Chim. (1962), 7, 303-337). La condensation peut s'effectuer par chauffage dans un alcool (par exemple dans du méthanol ou de l'isopropanol), éventuellement en présence de DMF et/ou de pyridine à une température de préférence comprise entre 20 et 100 °C pour une durée généralement comprise entre quelques heures et une nuit.

Dans les cas où B est une amine, les composés finaux de formule générale (**I**) sont des guanidines. Celles-ci peuvent être préparées, par exemple, par la condensation des amines de formule générale **(II)** avec les dérivés de formule générale **(IV)** ou **(IV').** Les réactifs de formule générale **(IV)** dans lesquels L représente, par exemple, un cycle pyrazole sont condensés sur les amines de formule générale (**II**) selon les conditions décrites dans la littérature *(*J. Org. Chem. (1992) 57, 2497-2502) de même pour les réactifs de formule générale (**IV'**) dans lesquels L représente, par exemple, un cycle pyrazole et Gp le groupement tBuOCO *(*Tetrahedron Lett. (1993) 34 (21), 3389-3392) ou bien lorsque L représente le groupement -N-SO₂-CF₃ et Gp le groupement tBuOCO (J. Org. Chem. (1998) 63, 3804-3805). Lors de l'ultime étape de la synthèse, la déprotection de la fonction guanidine est effectuée en présence d'un acide fort tel que par exemple l'acide trifluoroacétique.

L'invention concerne donc également un procédé de préparation d'un produit de formule générale **(I)** tel que défini précédemment, caractérisé en ce que l'on fait réagir l'intermédiaire de formule générale **(II)**

A-X-Y-Φ-NH₂ **(II)**

dans laquelle A, B, X, Y et Φ sont tels que définis ci-dessus,
avec l'intermédiaire de formule générale **(III)** 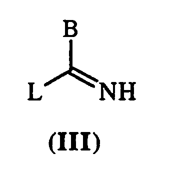 dans laquelle B est tel que défini ci-dessus et L représente un groupe partant, par exemple un radical alkoxy, alkylthio, aralkylthio, acide sulfonique, halogénure, alcool arylique ou tosyle.

L'invention concerne de plus un procédé de préparation d'un produit de formule générale **(I)** dans laquelle B est une amine, caractérisé en ce que l'on fait réagir l'intermédiaire de formule générale (II)

A-X-Y-Φ-NH₂ **(II)**

dans laquelle A, B, X, Y et Φ sont tels que définis ci-dessus,
a) soit avec l'intermédiaire de formule générale (**IV**) 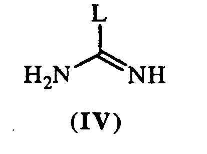 dans laquelle L représente un groupe partant, par exemple un radical alkoxy, alkylthio, aralkylthio, acide sulfonique, halogénure, alcool arylique ou tosyle,
b) soit avec l'intermédiaire de formule générale **(IV')** 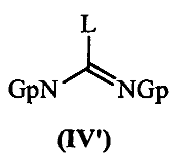
dans laquelle **L** représente un groupe partant, par exemple un radical alkoxy, alkylthio, aralkylthio, acide sulfonique, halogénure, alcool arylique ou tosyle, et Gp un groupe protecteur de type carbamate, par exemple le groupe t-butoxycarbonyle,
cette réaction étant suivie, dans le cas où l'on opte pour la réaction avec le composé de formule générale **(IV'),** par une hydrolyse en présence d'un acide fort, par exemple l'acide trifluoroacétique.

### Préparation des composés de formule générale (II):

Les intermédiaires de formule générale (II), non commerciaux, sont obtenus soit à partir de la coupure d'un groupe protecteur, soit à partir de la réduction d'un précurseur de type azoture ou bien nitro, comme illustrés dans les schémas synthétiques ci-dessous.

### Déprotection du groupe amino :

Les intermédiaires de formule générale **(II),** dans lesquels A, X, Y et Φ sont tels que définis ci-dessus, peuvent être préparés à partir des intermédiaires de formule générale **(V),** schéma 2, qui sont des composés comportant une amine protégée (N=Gp') sous forme, par exemple, de phtalimide ou de 2,5-diméthylpyrrole. Dans le cas des phtalimides, ceux-ci sont déprotégés classiquement à l'aide d'hydrate d'hydrazine au reflux de l'éthanol et dans le cas des pyrroles, la déprotection a lieu par chauffage en présence de chlorhydrate d'hydroxylamine, pour conduire finalement aux amines primaires de formule générale **(II).**

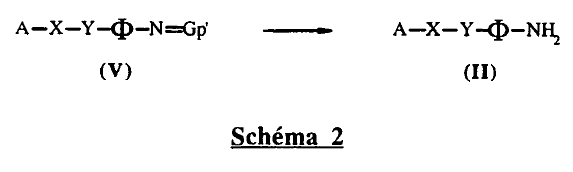

### Réduction des précurseurs de type azido :

Les intermédiaires synthétiques de formule générale **(VI),** schéma 3, dans lesquels A, X, Y et Φ sont tels que définis ci-dessus, sont des dérivés d'azide qui sont transformés en amine primaire de formule générale **(II),** par exemple, à l'aide d'hydrogène en présence de Pd/C dans un solvant approprié tel que l'éthanol.

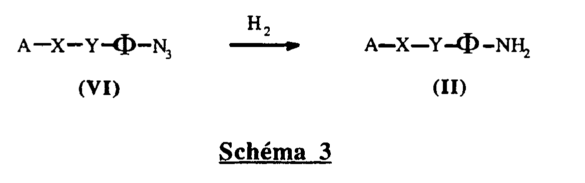

### Réduction des précurseurs de type nitro ;

La réduction de la fonction nitro des intermédiaires de formule générale (**VII)**, schéma 4, dans lesquels A, X, Y et Φ sont tels que définis ci-dessus, est généralement effectuée par hydrogénation catalytique, dans l'éthanol, en présence de Pd/C, sauf dans les cas de molécules sensibles à ces conditions où le groupement nitro est sélectivement réduit, par exemple, en chauffant le produit dans un solvant approprié tel que l'acétate d'éthyle avec un peu d'éthanol en présence de SnCl₂ (J. Heterocyclic Chem. (1987), 24, 927-930 ; Tetrahedron Letters (1984), 25 (8), 839-842), à l'aide également de SnCl₂ en présence de Zn *(*Synthesis (1996), (9), 1076-1078), ou bien à l'aide de NaBH₄-BiCl₃ *(*Synth. Com. (1995) 25 (23), 3799-3803) dans un solvant tel que l'éthanol, ou alors en utilisant du Ni Raney additionné d'hydrate d'hydrazine (Monatshefte für Chemie, (1995), 126, 725-732 ; Pharmazie (1993) 48 (11), 817-820) dans le cas, par exemple, des nitrocarbazoles.

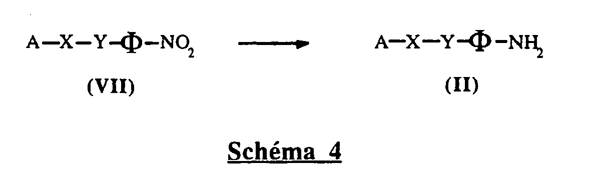

### Préparation des composés de formule générale (V):

Les intermédiaires de formule générale **(V),** schéma 5, comportent une amine protégée sous forme de phtalimide, dans lesquels X = -O-, Y = -(CH₂)ₘ- avec A, R₁, R₂, R₃, R₄, R₅, W, m et Φ tels que définis ci-dessus, peuvent être préparés à partir des cycles aromatiques hydroxylés de formule générale **(VIII).** Dans le cas particulier des hydroxycarbazoles, les composés de formule générale **(VIII)** sont préparés selon un protocole expérimental de la littérature (J. Chem. Soc. (1955), 3475-3477 ; J. Med. Chem. (1964) 7, 158-161) et dans celui des hydroxyphénothiazines le protocole est décrit dans J. Med. Chem. (1992) 35, 716. Les composés de formule générale **(VIII)** sont condensés sur des halogénoalkyl-phtalimides commerciaux en présence d'une base, par exemple NaH, dans un solvant tel que le DMF, pour conduire aux intermédiaires de formule générale **(V).**

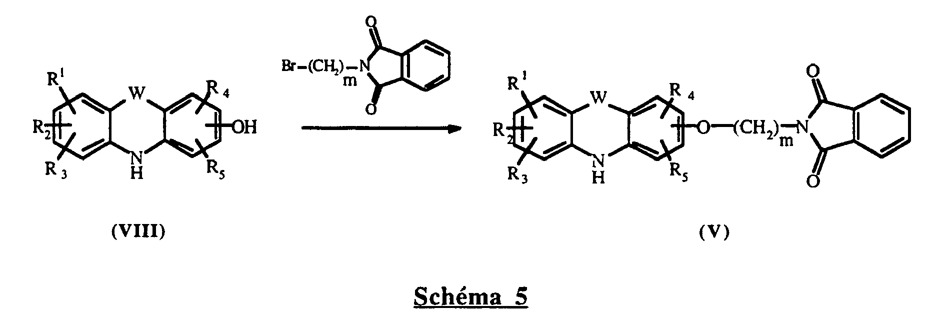

### Préparation des composés de formule générale (VI) :

Les intermédiaires de formule générale **(VI),** schéma 6, dans lesquels A, X, Y, R₁, R₂, R₃, R₄, R₅, W, m et Φ sont tels que définis ci-dessus, sont des dérivés de type azido. Ils sont préparés en deux étapes à partir des intermédiaires de formule générale **(VIII)** (schéma 5). Le radical OH des composés de formule générale **(VIII)** peut être alkylé par des dérivés dihalogénés du type dibromoalkane, en présence d'une base, par exemple NaH ou NaOH, pour conduire aux composés de formule générale **(IX)** qui sont ensuite substitués à l'aide d'azide de sodium dans le DMF pour conduire aux intermédiaires de formule générale (**VI**).

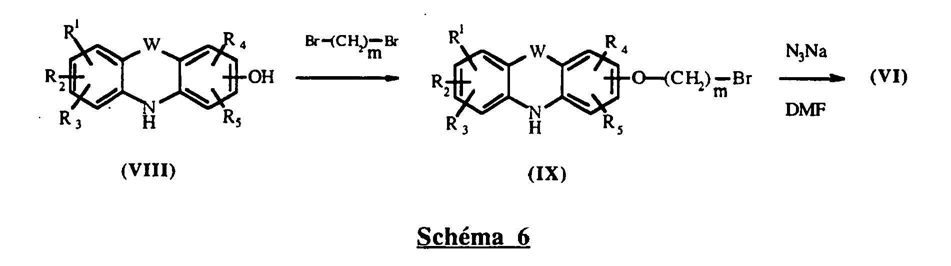

### Préparation des composés de formule générale (VII) :

Les synthèses des composés de formule générale **(VII),** qui portent un groupement nitro terminal, dans lesquels A, X, Y et Φ sont tels que décrits ci-dessus, sont illustrées dans les schémas synthétiques suivants.

### Synthèses des carboxamides de formule générale (VII)

Les carboxamides de formule générale (**VII)**, schéma 7, dans lesquels X représente -NR₁₆-CO- et A, Y, Φ et R₁₆ sont tels que définis ci-dessus, sont préparés par condensation des amines commerciales de formule générale (**X**) avec les acides commerciaux de formule générale (**XI**). Les liaisons carboxamides sont formées dans des conditions classiques de synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) *(*J. Med. Chem. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)). Les synthèses des amines de formule générale **(X)** non commerciales et les synthèses des acides carboxyliques de formule générale **(XI)** non commerciaux sont décrites au chapitre Préparation des Intermédiaires.

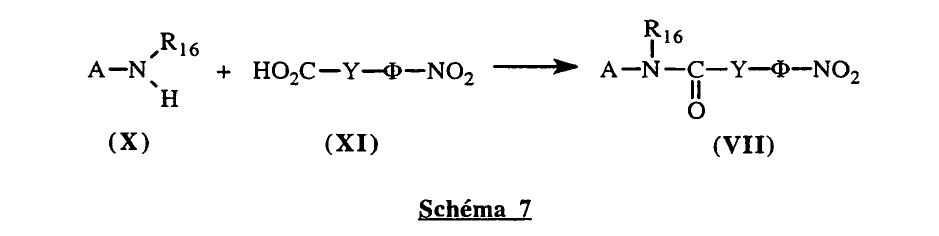

Les carboxamides de formule générale **(VII),** schéma 8, dans lesquels X représente -CO-NR₁₆- et A, Y, Q, Φ et R₁₆ sont tels que définis ci-dessus, sont préparés par condensation des acides de formule générale **(XII)** avec les amines commerciales de formule générale **(XIII)** ou les amines de formule générale **(XIV)** dans les conditions classiques de la synthèse peptidique précédemment décrites. Les synthèses des acides de formule générale **(XII)** et des amines de formule générale **(XIV)** non commerciaux sont décrites au chapitre Préparation des Intermédiaires.

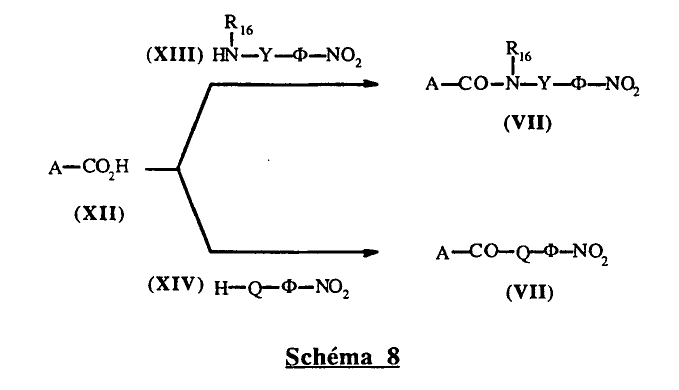

Les carboxamides de formule générale (**VII**), schéma 9, dans lesquels X représente -O-, Y représente -(CH₂)ₘ-NR₁₈-CO-(CH₂)ₙ- avec A, R₁₈, m, n et Φ tels que définis ci-dessus, sont préparés par condensation peptidique classique des acides de formule générale **(XI)** (schéma 7) avec les amines de formule générale **(II)** dont les synthèses ont été décrites dans les schémas 2 et 3.

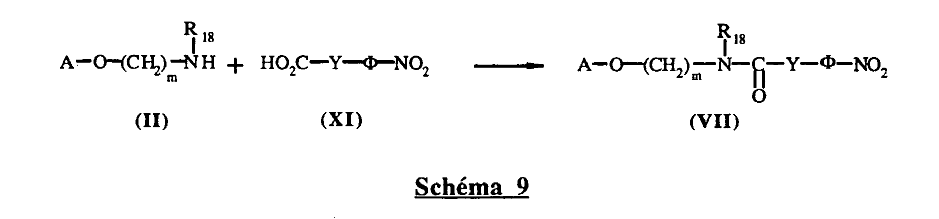

### Synthèse des amines de formule générale (VII) :

Les amines de formule générale **(VII)** dans lesquelles X = -NR₁₆- et Y = -(CH₂)ₘ- avec A, R₁₆, m et Φ tels que définis ci-dessus, sont préparés, schéma 10, à partir des carboxamides de formule générale **(VII).** La réduction de la fonction carboxamide est effectuée en présence d'un excès (5 éq.) de diborane dans le THF, en chauffant le mélange à reflux du solvant pour conduire aux amines de formule générale **(VII).**

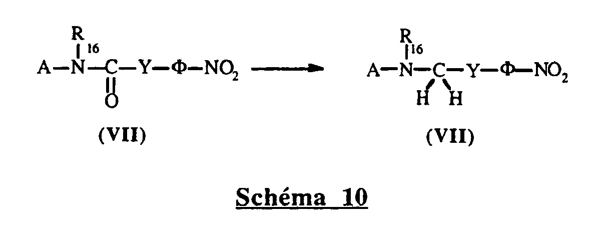

### Synthèse des carbamates de formule générale (VII);

Les dérivés carbamates de formule générale **(VII)** dans lesquels X = -NH-CO-O- et Y = -(CH₂)ₘ- avec A, m et Φ tels que définis ci-dessus, sont préparés, schéma 11, par condensation d'une amine de formule générale **(X)** (schéma 7) avec un alcool commercial de formule générale **(XV)** en présence de triphosgène et d'une base telle que par exemple la N,N-diméthylaniline dans un solvant inerte comme, par exemple, le dichlorométhane, selon un protocole décrit dans Tetrahedron Lett. (1993) 34 (44), 7129-7132.

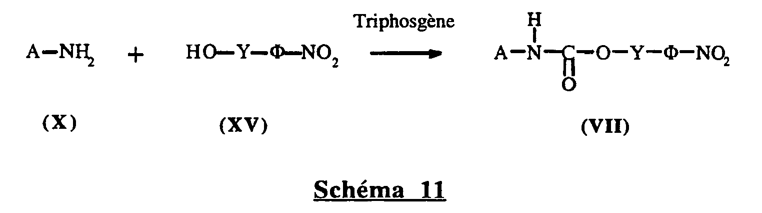

### Synthèse des urées de formule générale (VII) :

Les urées de formule générale **(VII)** dans lesquelles X = -NH-CO-NR₁₇- et Y = -(CH₂)ₘ- ou X-Y = -NH-CO-Q- (dans le cas d'un hétérocycle azoté) avec A, R₁₇, m, Q et Φ tels que définis ci-dessus, sont préparés, schéma 12, à partir des amines primaires de formule générale **(X)** (schéma 7) et des amines de formule générale **(XIII)** ou **(XIV)** (schéma 8) en présence de triphosgène et d'une amine tertiaire, telle que, par exemple, la diisopropyléthylamine, dans un solvant neutre tel que le dichlorométhane (J. Org. Chem. (1994), 59 (7), 1937-1938).

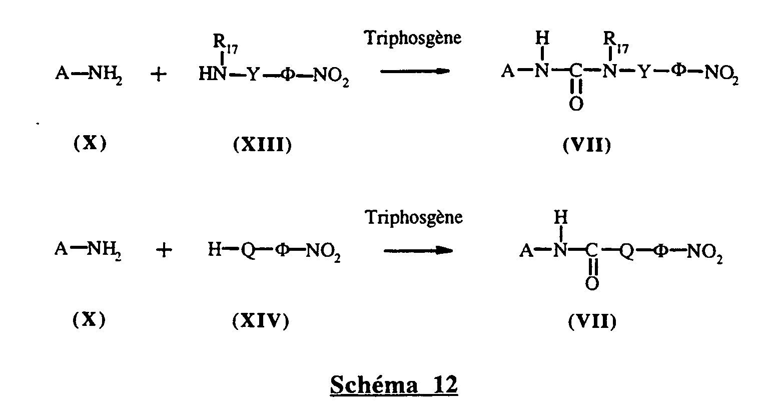

### Synthèse des esters de formule générale (VII) :

Les esters carboxyliques de formule générale (**VII)** dans lesquels X = -O-CO- ou -CO-O- et Y = -(CH₂)ₘ- avec A, m et Φ tels que définis ci-dessus, sont préparés en une seule étape à partir des alcools de formule générale (VIII) (schéma 5) et des acides carboxyliques de formule générale **(XI)** (schéma 7) ou des acides de formule générale **(XII)** (schéma 8) et des alcools de formule générale **(XV)** (schéma 11) en présence d'un agent de couplage tel que, par exemple, le carbonyldiimidazole ou le dicyclohexylcarbodiimide, dans un solvant approprié tel que le dichlorométhane, par exemple.

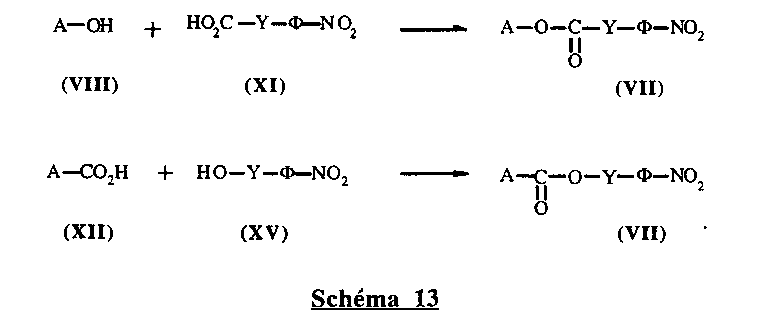

### Synthèse des éthers de formule générale (VII) :

Les éthers de formule générale (**VII**) dans lesquels X = -O- et Y = -(CH₂)ₘ- avec A, m et Φ tels que définis ci-dessus, schéma 14, sont préparés en une seule étape par condensation des alcools aromatiques de formule générale **(VIII)** (schéma 5) et des alcools de formule générale **(XV)** (schéma 11) dans les conditions classiques de Mitsunobu (Synthesis (1981), 1) en présence, par exemple, de diéthylazodicarboxylate et de tributylphosphine, dans un solvant tel que, par exemple, le THF. 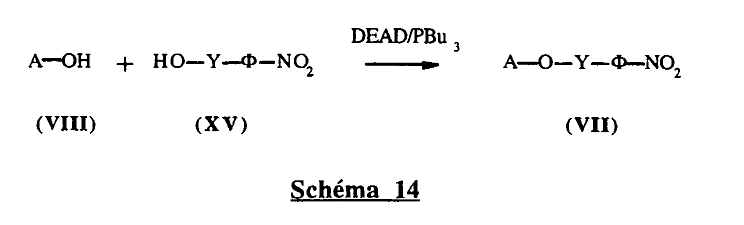 Lorsque X = -O-, Y est une liaison et Φ = phénylène, avec A et n tels que définis ci-dessus, les éthers de formule générale **(VII)**, schéma 15, peuvent également être préparés en une seule étape par condensation des alcools aromatiques de formule générale **(VIII)** (schéma 5) sur des dérivés halogénés de formule générale **(XVI),** dans laquelle Hal représente un atome halogène, en présence d'une base telle que, par exemple, K₂CO₃ ou NaH, dans un solvant polaire tel que, par exemple, le THF ou le DMF, à une température de réaction comprise entre 20 et 140 °C.

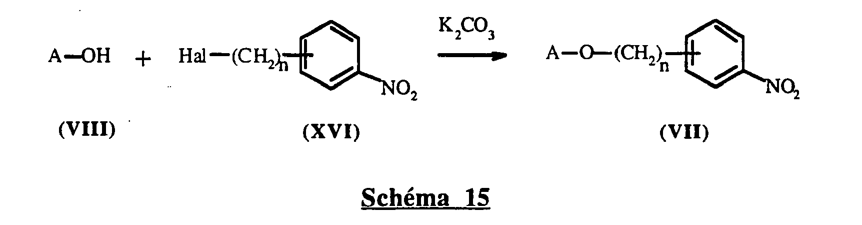

Lorsque X = -O- et Y = -(CH₂)ₘ-Q- avec A, Φ, Q et m tels que définis ci-dessus, les éthers de formule générale **(VII)**, schéma 16, peuvent également être préparés par condensation des alcools aromatiques de formule générale **(VIII)** (schéma 5) sur des dérivés halogénés de formule générale **(XVII),** dans laquelle Hal représente un atome halogène, en présence d'une base telle que, par exemple, K₂CO₃, dans un solvant inerte tel que, par exemple, CH₂Cl₂, à une température comprise entre 40 °C et la température de reflux du mélange réactionnel. La synthèse des composés de formule générale **(XVII)** est décrite au chapitre Préparation des Intermédiaires.

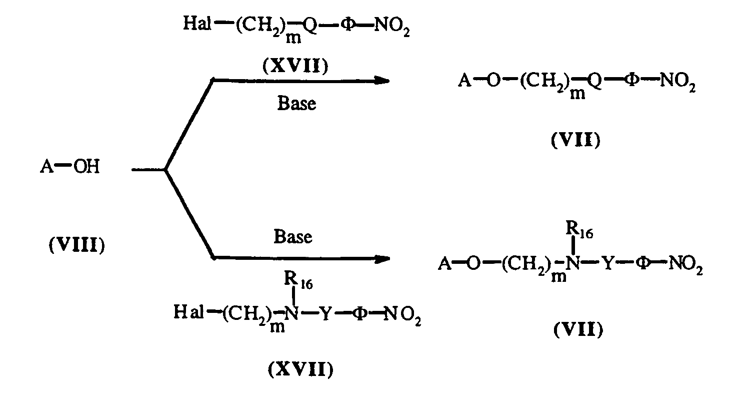

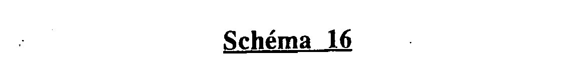

### Synthèse des amines de formule générale (VII) par amination réductrice :

Les amines de formule générale **(VII),** dans lesquelles X = -NR₁₆-CO- et Y = -(CH₂)ₘ-NR₁₈-(CH₂)ₙ- avec A, Φ, R₁₆, R₁₈, m et n tels que définis ci-dessus, sont préparées, schéma 17, par condensation d'un aldéhyde de formule générale **(XIX)** avec une amine de formule générale **(XVIII)** en milieu réducteur. La réaction a lieu dans un solvant alcoolique tel que, par exemple, le méthanol en présence de tamis moléculaire 4 Å pulvérulent, préalablement activé, et d'un agent réducteur tel que par exemple NaBH₄ ou NaBH₃CN. Les synthèses des amines de formule générale **(XVIII),** non commerciales, sont décrites au chapitre Préparation des Intermédiaires.

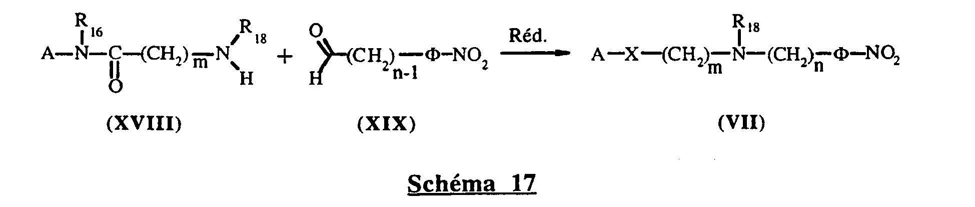

De façon analogue, les amines de formule générale **(VII)**, dans lesquelles X = -CH₂-NR₁₆- , avec A, Y, Φ et R₁₆ tels que définis ci-dessus, sont préparées, schéma 18, par condensation des aldéhydes de formule générale **(XX)** avec les amines de formule générale **(XIII)** (schéma 8) en milieu réducteur dans les conditions précédemment décrites. La préparation des aldéhydes de formule générale **(XX),** non commerciaux, est décrite au chapitre Préparation des Intermédiaires.

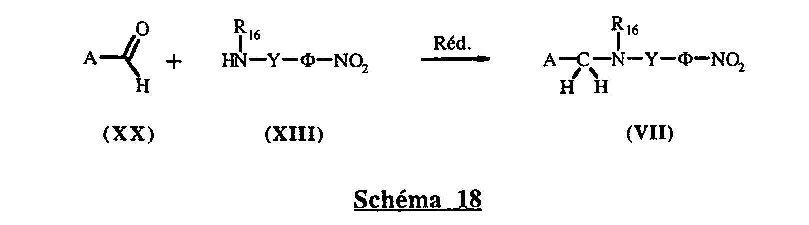

### Modification du radical A dans les composés de formule générale (VII) :

Les intermédiaires de formule générale **(VII),** dans lesquels A, X, Y, Φ, R₁, R₂, R₃, R₄ et R₅ sont tels que décrits ci-dessus, peuvent subir des modifications chimiques au niveau du radical A, schéma 19, en particulier au niveau de l'atome d'azote qui peut être alkylé à l'aide d'un réactif R₁₁-Hal, tel que défini ci-dessus, et en particulier à l'aide de l'iodure de méthyle en présence d'une base telle que, par exemple, NaH, dans un solvant inerte tel que le THF par exemple.

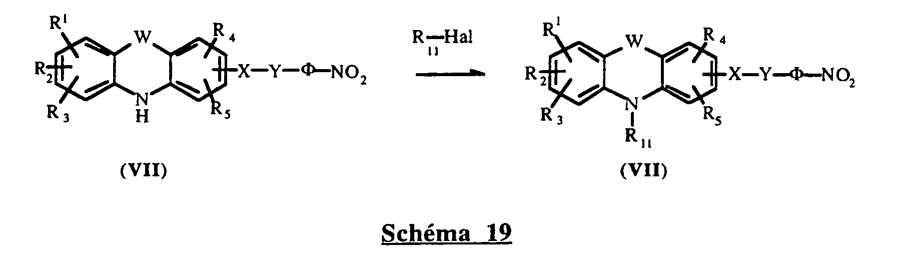

### Préparation des différents intermédiaires de synthèse :

### Synthèse des intermédiaires (X)

Les intermédiaires de formule générale **(X)** dans lesquels A est une diphénylamine (W n'existe pas), sont accessibles à partir des méthodes décrites dans la littérature *(*Synthesis (1990) 430 ; Indian J. Chem. (1981) 20B, 611-613 ; J. Med. Chem. (1975) 18(4), 386-391) qui passent par la réduction d'un intermédiaire nitrodiphénylamine. La réduction de la fonction nitro est effectuée classiquement par hydrogénation en présence d'une quantité catalytique de Pd/C pour accéder aux aminodiphénylamines de formule générale **(X).**

Lorsque A est un dérivé carbazole (W représente alors une liaison directe), les méthodes de préparation des aminocarbazoles de formule générale **(X)** passent par la synthèse d'un intermédiaire nitrocarbazole. Ces méthodes sont décrites dans Pharmazie (1993) 48(11), 817-820 ; Synth. Commun. (1994) 24(1), 1-10 ; J. Org. Chem. (1980) 45, 1493-1496 ; J. Org. Chem. (1964) 29(8), 2474-2476 ; Org. Prep. Proced. Int. (1981) 13(6), 419-421 ou J. Org. Chem. (1963) 28, 884. La réduction de la fonction nitro des intermédiaires nitrocarbazoles est, dans ce cas, effectuée de préférence à l'aide d'hydrate d'hydrazine en présence de Nickel de Raney.

Les intermédiaires de formule générale (**X**) dans lesquels A est un dérivé phénothiazine (W représente un atome de soufre), sont accessibles à partir de méthodes de la littérature qui passent par la synthèse d'un dérivé nitrophénothiazine. En particulier la 3-nitrophénothiazine est décrite dans J. Org. Chem. (1972) 37, 2691. La réduction de la fonction nitro pour accéder aux aminophénothiazines de formule générale **(X)** est effectuée classiquement par hydrogénation en présence d'une quantité catalytique de Pd/C dans un solvant tel que l'éthanol.

### Synthèse des intermédiaires (XI) :

Les synthèses des acides de formule générale (**XI**), non commerciaux, sont décrites dans les schémas 7.1 et 7.2.

Dans le cas particulier où Y = -(CH₂)ₘ-Q-(CH₂)ₙ- et Φ est un radical phénylène, avec Q, m et n tels que décrits ci-dessus, les acides carboxyliques de formule générale (**XI**), schéma 7.1, sont préparés, en 2 étapes, à partir d'une amine hétérocyclique de formule générale **(XIV)** (schéma 8), par exemple la 4-nitrophénylpipérazine, et d'un halogénoester de formule générale **(XI.1)** tel que par exemple le bromoacétate d'éthyle. La condensation s'effectue à 20 °C en présence d'une base telle que, par exemple, la triéthylamine dans un solvant inerte tel que, par exemple, le dichlorométhane pour conduire aux intermédiaires de formule générale **(XI.2).** La saponification par LiOH à 20 °C conduit aux acides carboxyliques de formule générale **(XI).**

Dans les cas où Y = -(CH₂)ₘ-O-(CH₂)ₙ- et Φ est un radical phénylène, avec m et n tels que décrits ci-dessus, la synthèse des acides carboxyliques de formule générale **(XI),** schéma 7.1, passe par la condensation des dérivés halogénés de formule générale **(XI.1)** sur les alcools de formule générale (XI.3) en présence d'une base telle que, par exemple, la triéthylamine ou le carbonate de potassium à reflux d'un solvant polaire tel que, par exemple, le THF ou le DMF. La déprotection de la fonction ester de l'intermédiaire de formule générale **(XI.4)** est ensuite effectuée classiquement en présence d'une base ou bien d'un acide fort dans le cas des esters de *tert*-butyle.

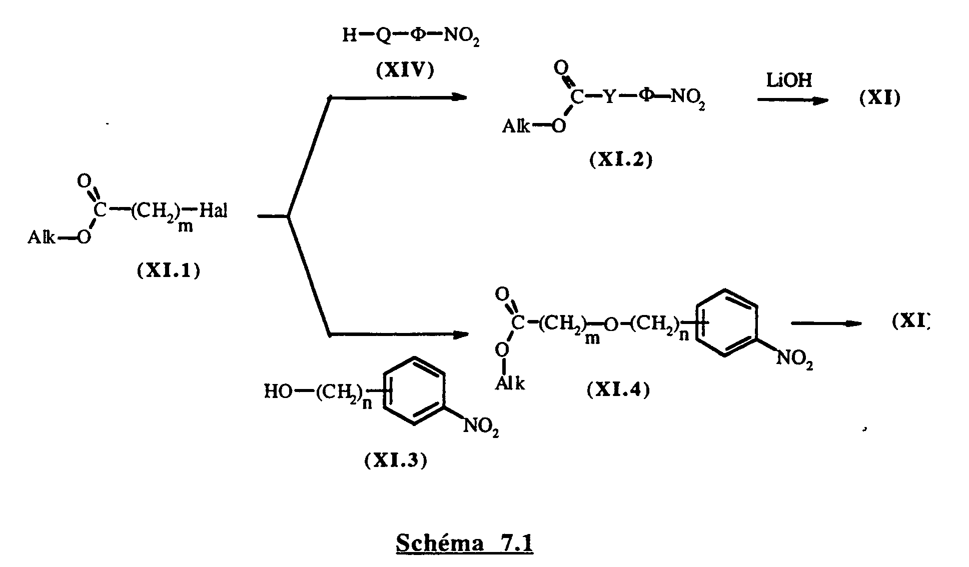

Les acides carboxyliques de formule générale **(XI)** dans lesquels Y = -(CH₂)ₘ- et Φ représente un groupement phénylène substitué, avec m tel que décrit ci-dessus, sont préparés en 3 étapes à partir des alcools commerciaux de formule générale (**XI.3**), schéma 7.2. L'activation de l'alcool est effectué classiquement par du chlorure de méthane sulfonyle (MsCl) en présence d'une base telle que la triéthylamine dans un solvant inerte comme le dichlorométhane pour conduire aux intermédiaires de formule générale (**XI.4**). Le mésylate est ensuite déplacé par du cyanure de sodium dans le DMF pour conduire aux intermédiaires de formule générale (**XI.5**). La fonction nitrile est ensuite hydrolysée par chauffage dans un mélange d'éthanol et de HCl concentré pour donner les acides de formule générale (**XI**).

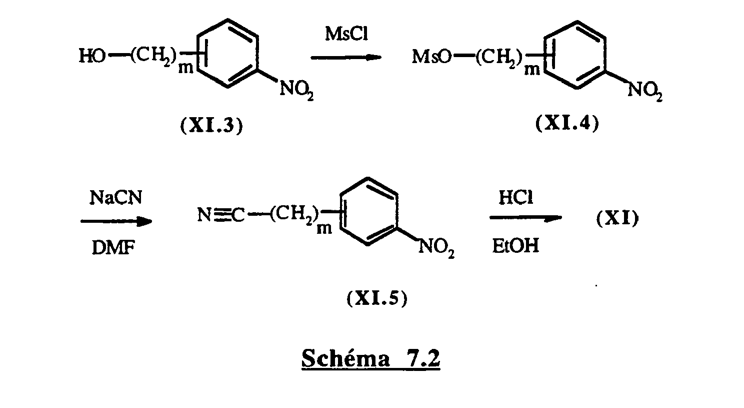

### Synthèse des intermédiaires (XII)

La synthèse des dérivés d'acides carboxyliques des phénothiazines de formule générale (**XII**) est décrite dans la littérature *(*J. Med. Chem. (1992) 35(4), 716-724).

### Synthèse des intermédiaires (XIV) :

Les amines non commerciales de formule générale (**XIV**) définie précédemment dans lesquelles Q représente homopipérazine, 2-méthylpipérazine, 2,5-diméthylpipérazine, 4-aminopipéridine, sont synthétisées en trois étapes à partir des diamines commerciales correspondantes. Les diamines sont sélectivement monoprotégées sous forme de carbamate *(*Synthesis (1984), (12), 1032-1033; Synth. Commun. (1990), 20, (16), 2559-2564) avant de réagir par substitution nucléophile sur un halogénonitrobenzène, en particulier le 4-fluoronitrobenzène. Les amines, précédemment protégées, sont libérées à la dernière étape, selon des méthodes décrites dans la littérature (T.W. Greene et P.G.M. Wuts, Protective Groups in Organic Synthesis, Second edition (Wiley-Interscience, 1991)), pour conduire aux intermédiaires de formule générale (**XIV**).

### Synthése des intermédiaires (XVII)

Les dérivés halogénés de formule générale **(XVII)** définie précédemment, schéma 16.1, sont accessibles en deux étapes à partir des amines de formule générale **(XIII)** ou **(XIV)** (schéma 8) et des dérivés halogénés commerciaux de formule générale **(XVII.1).** La condensation pour conduire aux intermédiaires de formule générale **(XVII.2)** ou **(XVII.3)** se fait classiquement en présence d'une base telle que, par exemple, K₂CO₃ dans un solvant inerte approprié tel que, par exemple, le dichlorométhane. Ensuite la fonction alcool est activée sous forme d'un dérivé halogéné à l'aide, par exemple, du tétrabromure de carbone en présence de triphénylphosphine pour conduire aux intermédiaires de formule générale **(XVII).**

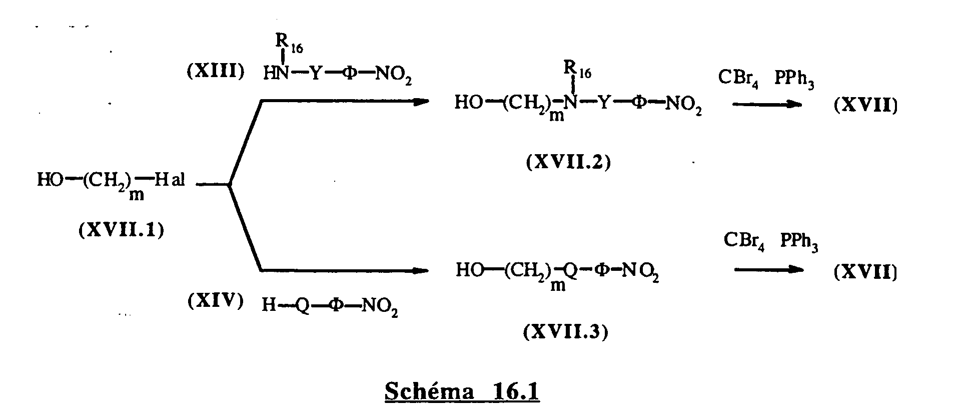

### Synthèse des intermédiaires (XVIII) :

Les amines de formule générale **(XVIII)** définie précédemment, schéma 17.1, dans lesquelles A, R₁₆, R₁₈ et m sont tels que définis ci-dessus, sont préparées par condensation des amines de formule générale **(X)** (schéma 7) sur les aminoacides protégés (Gp : Groupe protecteur) de formule générale **(XVIII.1),** dans les conditions classiques de la synthèse peptidique (voir chapitre synthèse des carboxamides). La déprotection de l'amine des composés de formule générale **(XVIII.2)** est ensuite effectuée classiquement selon les conditions décrites dans la littérature (T.W. Greene et P.G.M. Wuts, Protective Groups in Organic Synthesis, Second edition (Wiley-Interscience, 1991)).

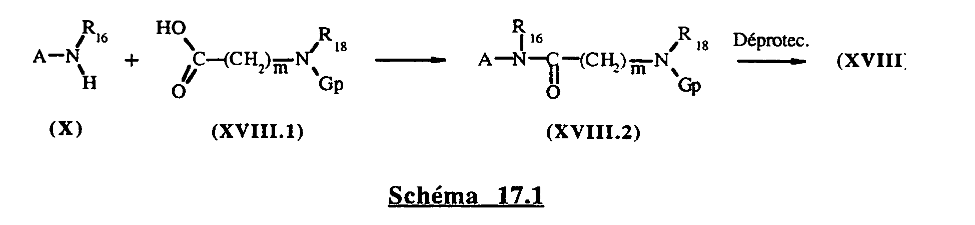

### Synthèse des intermédiaires (XX)

La synthèse des aldéhydes des phénothiazines de formule générale **(XX)** définie précédemment est décrite dans la littérature (J. Chem. Soc. (1951), 1834 ; Bull. Soc. Chim. Fr. (1969), 1769).

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES :

### Exemple 1 : iodhydrate de N-[4-(phénylamino)phényl]-2-thiophène-carboximidamide : 1

Dans un ballon de 50 ml sous atmosphère d'argon, on mélange 0,92 g (5 mmoles) de 4-aminodiphénylamine et 2,85 g (10 mmoles) de iodhydrate de S-méthyl-2-thiophène thiocarboximide dans 15 ml d'isopropanol. Le mélange réactionnel est chauffé à 70 °C pendant 48 heures. Le solvant est partiellement évaporé sous vide et le solide obtenu est filtré et lavé plusieurs fois successivement avec de l'isopropanol et de l'éther éthylique. On obtient une poudre jaune avec un rendement de 98%. Point de fusion : 216,3-216,8 °C.
RMN ¹H (400 MHz, DMSO d6, δ) : 6,90 (m, 1H, arom.) ; 7,10-7,30 (m, 8H, arom.) ; 7,40 (m, 1H, thiophène) ; 8,10-8,20 (m, 2H, thiophène) ; 8,50 (s, 1H, NH) ; 8,75 (s, 1H, NH⁺) ; 9,70 (s, 1H, NH⁺) ; 11,15 (s, 1H, NH⁺).
IR : ν_{C=N} (amidine) : 1590 cm⁻¹.

### Exemple 2 : chlorhydrate de 4-{[2-thiényl(imino)méthyl]amino}-N-[4-(phénylamino)phényl]-benzèneacétamide : 2

### 2.1) 4-nitro-N-[4-(phénylamino)phényl]-benzèneacétamide :

Dans un ballon de 100 ml, on dissout successivement 1,84 g (10 mmoles) de 4-aminodiphénylamine, 1,81 g (10 mmoles) d'acide 4-nitrophénylacétique et 1,48 g (11 mmoles) d'hydroxybenzotriazole dans 40 ml de THF. On ajoute ensuite 2,27 g (11 mmoles) de 1,3-dicyclohexylcarbodiimide (DCC) et on agite le mélange réactionnel pendant 15 heures. Il se forme un précipité de dicyclohexylurée (DCU) qui est filtré et rincé par 100 ml d'acétate d'éthyle. Le filtrat est ensuite lavé successivement par 50 ml d'une solution saturée de Na₂CO₃, 50 ml d'eau, 50 ml d'une solution molaire d'HCl et finalement 2 x 50 ml de saumure. La phase organique est séchée sur sulfate de magnésium, filtrée et concentrée sous vide. Le résidu est purifié rapidement sur une colonne de gel de silice (éluant : heptane/acétate d'éthyle 1/1). Les fractions les plus pures sont collectées et évaporées sous vide pour conduire à une poudre marron. Le produit est investi tel quel dans l'étape suivante.
RMN ¹H (100 MHz, CDCl₃, δ) : 1,61 (s large, 1H, NH) ; 3,82 (s, 2H, CH₂) ; 5,70 (s large, 1H, NH) ; 6,85-7,50 (m, 10H, arom., NH-CO) ; 7,90 (AB, 4H, Ph-NO₂).

### 2.2) 4-amino-N-[4-(phénylamino)phényl]-benzèneacétamide :

Dans un autoclave en acier inox équipé d'un barreau magnétique, on introduit une solution de l'intermédiaire 2.1 (0,54 g, 1,54 mmole) dans 40 ml d'un mélange acétate d'éthyle/éthanol (1/1) ainsi que 0,1 g de Pd/C à 10%. Le mélange réactionnel est agité sous pression d'hydrogène (1,5 bar) pendant 1 heure 30 à une température de 20 °C. Le Pd/C est ensuite éliminé par filtration et le filtrat est concentré sous vide. Le résidu d'évaporation est purifié sur une colonne de gel de silice (éluant : heptane/acétate d'éthyle : 4/6), les fractions pures sont collectées et concentrées sous vide. On obtient une poudre blanche avec un rendement de 90%. Point de fusion : 162-163 °C.
RMN ¹H (100 MHz, CDCl₃, δ) : 1,61 (s large, 1H, NH) ; 3,61 (s, 2H, CH₂) ; 3,70 (s large, 2H, NH₂) ;. 5,62 (s large, 1H, NH-CO) ; 6,68-7,40 (m, 13H, arom.).

### 2.3) chlorhydrate de 4-{[2-thiényl(imino)méthyl]amino}-N-[4-(phénylamino)phényl]-benzèneacétamide : 2

Dans un ballon de 50 ml, on dissout 0,44 g (1,39 mmole) de l'intermédiaire 2.2 et 0,47 g (1,67 mmole) de iodhydrate de S-méthyl-2-thiophène thiocarboximide dans 15 ml d'isopropanol. Le mélange réactionnel est agité pendant 20 heures à une température de 60 °C. Après évaporation du solvant sous vide, le résidu est repris dans 100 ml d'un mélange (1/1) de soude 1N et d'acétate d'éthyle. Après décantation, la phase organique est lavée par 50 ml d'eau suivi de 50 ml de saumure. La solution organique est séchée sur sulfate de magnésium, filtrée, concentrée sous vide et le résidu est purifié sur une colonne de gel de silice (éluant : acétate d'éthyle). Les fractions pures sont collectées et concentrées sous vide. On obtient une poudre blanche avec un rendement de 25%. Le composé est ensuite dissous dans du méthanol et salifié par addition d'une solution d'HCl 1N dans l'éther éthylique (1ml). Après une heure d'agitation à 20 °C, le mélange réactionnel est concentrée sous vide pour conduire à une poudre jaune pâle. Point de fusion : le produit se transforme en mousse.

RMN ¹H (400 MHz, DMSO d6, δ) : 3,71 (s, 2H, CH₂) ; 4,60 (s large, 1H, NH) ; 6,75 (m, 1H, thiophène) ; 7,00 (m, 4H, arom.) ; 7,19 (m, 2H, arom.) ; 7,40 (m, 3H, arom.) ; 7,55 (m, 4H, arom.) ; 8,14 (m, 2H, thiophène) ; 8,95 (s large, 1H, NH⁺); 9,86 (s large, 1H, NH⁺) ; 10,41 (s, 1H, NH-CO) ; 11,60 (s large, 1H, NH⁺).
IR : ν_{C=O} (amide) : 1649 cm⁻¹ ; ν_{C=N} (amidine) : 1597 cm⁻¹.

### Exemple 3 : iodhydrate de {4-{[2-thiényl(imino)méthyl]amino}phénoxy}-N-[4-(phénylamino)phényl]-acétamide : 3

### 3.1) 4-nitrophénoxyacétate de tertiobutyle :

Sous atmosphère d'azote, dans un ballon de 250 ml contenant 100 ml de THF, on introduit 3 g (21,6 mmoles) de paranitrophénol, 8,94 g (64,8 mmoles) de carbonate de potassium et 8,42 g (43,2 mmoles) de bromoacétate de tertiobutyle. Le mélange réactionnel est agité à reflux pendant 2 heures. Le solide est filtré et le filtrat concentré sous pression réduite. Le résidu est repris par 50 ml d'acétate d'éthyle et lavé successivement par 50 ml d'eau et 50 ml de saumure. La phase organique est séchée sur sulfate de sodium, filtrée et évaporée sous vide. Après purification sur colonne de gel de silice (éluant : acétate d'éthyle/ heptane 1:8) et concentration sous vide des fractions pures, on obtient une poudre blanche avec 50% de rendement. Point de fusion : 81-83 °C.
RMN ¹H (100 MHz, CDCl₃, δ) : 1,50 (s, 9H, 3 x CH₃) ; 4,60 (s, 2H, CH₂) ; 7,57 (AB, 4H, Ph-NO₂).

### 3.2) Acide 4-nitrophénoxyacétique :

Dans un ballon de 100 ml, sous atmosphère d'azote, on dissout 2,58 g (10,2 mmoles) de l'intermédiaire 3.1 dans 45 ml de dichlorométhane. L'ensemble est refroidi à 0 °C et on ajoute goutte à goutte 7,85 ml (102 mmoles) d'acide trifluoroacétique. Le mélange réactionnel est agité pendant 3 heures et demi à température ambiante. La solution est ensuite concentrée sous pression réduite. Le résidu d'évaporation est repris par 30 ml d'acétate d'éthyle et lavé par 20 ml d'eau. La phase organique est séchée sur sulfate de sodium, filtrée et concentrée sous vide. On obtient un solide jaune avec un rendement de 89%. Le produit obtenu est suffisamment pur pour être utilisé directement dans l'étape suivante. Point de fusion : 190-192 °C.
RMN ¹H (100 MHz, CDCl₃, δ) : 2,00 (s large, 1H, COOH) ; 4,80 (s, 2H, CH₂) ; 7,60 (AB, 4H, Ph-NO₂).

### 3.3) (4-nitrophénoxy)-N-[(4-phénylamino)phényl]acétamide :

Dans un ballon de 100 ml, sous atmosphère d'azote, on dissout 1,65 g (8,98 mmoles) de 4-aminodiphénylamine, 1,77 g (8,98 mmoles) de l'intermédiaire 3.2 et 1,27 g (9,42 mmoles) d' hydroxybenzotriazole dans 40 ml de THF. Quand tout est dissous, on ajoute 1,94 g (9,42 mmoles) de 1,3-dicyclohexylcarbodiimide et on laisse agiter pendant 15 heures. Le précipité de dicyclohexylurée formé est filtré et rincé par de l'acétate d'éthyle. Le filtrat est évaporé sous vide et le résidu d'évaporation est repris par de l'acétate d'éthyle, il se forme alors un précipité qui est filtré et rincé à l'aide du même solvant. On obtient un solide verdâtre avec un rendement de 65%. Le produit obtenu est suffisamment pur pour être utilisé directement dans l'étape suivante. Point de fusion : 192-195 °C.
RMN ¹H (100 MHz, CDCl₃, δ) : 4,75 (s, 2H, CH₂-O) ; 5,70 (s large, 1H, NH) ; 7,10 (m, 9H, arom.) ; 7,85 (AB, 4H, Ph-NO₂); 8,05 (s large, 1H, NH-CO).

### 3.4) (4-aminophénoxy)-N-[(4-phénylamino)phényl]acétamide :

Dans un autoclave de 300 ml, on introduit 1 g (2,75 mmoles) de l'intermédiaire 3.3 dissous dans 200 ml d'un mélange de solvants (éthanol/dichlorométhane/THF 1:1:1) et 0,1 g de Palladium sur charbon à 10%. L'ensemble est placé sous une pression d'hydrogène de 1,5 bar et agité à température ambiante pendant 15 minutes. Le catalyseur est filtré et les solvants concentrés sous pression réduite pour donner un solide beige rosé avec un rendement de 71%. Point de fusion : 146-148 °C.
RMN ¹H (100 MHz, CDCl₃, δ) : 3,50 (s large, 2H, NH₂) ; 4,50 (s, 2H, CH₂-O) ; 5,70 (s large, 1H, NH) ; 6,70 (m, 4H, arom.) ; 7,10 (m, 4H, arom.) ; 7,25 (m, 5H, arom.) ; 8,20 (s large, 1H, NH-CO).

### 3.5) Iodhydrate de [4-{[imino(2-thiényl)méthyl]amino}phénoxy]-N-[(4-phénylamino)phényl]acétamide : 3

On chauffe à 50 °C, pendant 15 heures, un mélange de 0,3 g (0,9 mmole) de l'intermédiaire 3.4 en présence de 0,25 g (0,9 mmole) d'iodhydrate de S-méthyl-2-thiophène thiocarboximide en solution dans 20 ml d'isopropanol. Le mélange réactionnel est filtré et le solide obtenu est rincé par de l'éther éthylique. Une poudre jaune est obtenue avec un rendement de 78%. Point de fusion : 163-166 °C. RMN ¹H (400 MHz, DMSO d6, δ) : 4,75 (s, 2H, CH₂O) ; 6,77 (m, 1H, thiophène) ; 7,04 (m, 4H, arom.) ; 7,19 (m, 4H, arom.) ; 7,40 (m, 3H, arom.) ; 7,50 (m, 2H, arom.) ; 8,12 (m, 2H, thiophène) ; 8,81 (s large, 1H, NH⁺) ; 9,70 (s large, 1H, NH⁺) ; 10,01 (s, 1H, CO-NH) ; 11,20 (s large, 1H, NH⁺).
IR : ν_{C=O} (amide) : 1647 cm⁻¹ ; ν_{C=N} (amidine) : 1598 cm⁻¹.

### Exemple 4 : 4-{[2-thiényl(imino)méthyl]amino}-N-[2-(phénylamino)phényl]-benzènebutanamide : 4

Le protocole expérimental utilisé est le même que celui décrit pour l'exemple 2. Le produit est obtenu sous forme de base libre (solide blanc). Point de fusion : 164-167°C. RMN ¹H (400 MHz, DMSO d6, δ) : 1,86 (m, 2H, CH₂) ; 2,35 (m, 2H, CH₂) ; 2,55 (m, 2H, CH₂) ; 6,37 (s large, 2H, NH₂) ; 6,76 (m, 3H, arom.) ; 6,87 (m, 2H, arom.) ; 6,96 (m, 1H, thiophène) ; 7,10 (m, 3H, thiophène) ; 7,18 (m, 2H, arom.) ; 7,25 (m, 1H, arom.) ; 7,33 (s, 1H, NH) ; 7,52 (m, 1H, thiophène) ; 7,73 (m, 1H, thiophène) ; 9,36 (s, 1H, NH-CO).
IR : ν_{C=O} (amide) : 1627cm⁻¹ ; ν_{C=N} (amidine) : 1591 cm⁻¹.

### Exemple 5 : chlorhydrate de 4-{[2-thiényl(imino)méthyl]amino}-N-[4-(phénylamino)phényl]-benzènebutanamide : 5

Le protocole expérimental utilisé est le même que celui décrit pour l'exemple 2. On obtient le chlorhydrate sous forme d'une poudre saumonée. Point de fusion : 167-170 °C.
RMN ¹H (400 MHz, DMSO d6, δ) : 1,90 (m, 2H, CH₂) ; 2,35 (m, 2H, CH₂) ; 2,70 (m, 2H, CH₂) ; 6,70 (m, 1H, thiophène) ; 7,00 (m, 4H, arom.) ; 7,20 (m, 2H, arom.) ; 7,40 (m, 5H, arom.) ; 7,50 (m, 2H, arom.) ; 8,20 (m, 2H, thiophène) ; 8,90 (s, 1H, NH⁺) ; 9,85 (s, 1H, NH⁺) ; 9,90 (s, 1H, NHCO) ; 11,55 (s, 1H, NH⁺). IR : ν_{C=O} (amide) : 1654 cm⁻¹ ; ν_{C=N} (amidine) : 1597 cm⁻¹.

### Exemple 6 : chlorhydrate de 4-{[2-thiényl(imino)méthyl]amino}-N-[4-(4-méthoxyphénylamino)phényl]-benzènebutanamide : 6

Le protocole expérimental utilisé est le même que celui décrit pour l'intermédiaire 2.3, l'intermédiaire 6.2 remplaçant le 4-amino-N-[4-(phénylamino)phényl]-benzèneacétamide. On obtient une poudre beige avec un rendement de 65%. Point de fusion : 200-202°C. RMN ¹H (400 MHz, DMSO d6, δ) : 1,91 (m, 2H, CH₂) ; 2,33 (m, 2H, CH₂) ; 2,67 (m, 2H, CH₂) ; 3,69 (s, 3H, O-CH₃) ; 4,71 (s large, 1H, NH) ; 6,81-7,00 (m, 6H, arom.) ; 7,37-7,45 (m, 7H, arom.) ; 8,20 (m, 2H, thiophène) ; 8,90 (s large, 1H, NH⁺) ; 9,87 (s large, 1H, NH⁺) ; 9,92 (s, 1H, NH-CO) ; 11,67 (s large, 1H, NH⁺). IR :ν_{C=O} (amide) : 1664 cm⁻¹ ; ν_{C=N} (amidine) : 1603 cm⁻¹.

### Exemple 7 : chlorhydrate de [4-(phénylamino)phényl]-carbamate de 2-{4-{[2-thiényl(imino)méthyl]amino}phényl}-éthyle : 7

### 7.1) [4-(phénylamino)phényl]-carbamate de 2-(4-nitrophényl)-éthyle :

Dans un ballon de 250ml, sous argon, on dissout 1,18 g (3,9 mmoles) de triphosgène dans 15 ml de dichlorométhane. Au moyen d'une seringue motorisée, on additionne en 1 heure une solution de 2 g (12 mmoles) de 4-nitrophényléthanol et de 1,7 ml (13 mmoles) de N,N-diméthylaniline dans 40 ml de dichlorométhane. Le mélange réactionnel est agité quelques minutes à 20 °C avant l'addition en une seule fois d'une solution de 2,2 g (12 mmoles) de 4-aminodiphénylamine et de 1,7 ml (13 mmoles) de N,N-diméthylaniline dans 40 ml de dichlorométhane. Après une heure d'agitation à 20 °C, le contenu du ballon est versé dans 100ml d'eau. Le mélange est dilué par 100 ml de dichlorométhane et agité. La phase organique est décantée, séchée sur sulfate de magnésium, filtrée et évaporée sous vide. Le solide obtenu est repris par de l'éther éthylique, trituré et filtré. Après séchage, on obtient une poudre verdâtre avec un rendement de 22%. Point de fusion : 146,4-148°C.
RMN ¹H (400 MHz, CDCl₃, δ) : 3,10 (m, 2H, CH₂) ; 4,40 (m, 2H, CH₂) ; 5,65 (s, 1H, NH) ; 6,50 (s, 1H, NH) ; 6,80-7,60 (m, 11H, arom.) ; 8,20 (m, 2H, arom.).

### 7.2) [4-(phénylamino)phényl]-carbamate de 2-(4-aminophényl)-éthyle :

Le protocole expérimental utilisé est le même que celui décrit pour l'intermédiaire 2.2, l'intermédiaire 7.1 remplaçant le 4-nitro-N-[4-(phénylamino)phényl]-benzèneacétamide. On obtient un solide blanc avec un rendement de 48%. Point de fusion : 140-140,5 °C. RMN ¹H (400 MHz, DMSO d6, δ) : 2,75 (m, 2H, CH₂) ; 4,15 (m, 2H, CH₂) ; 5,20 (s, 2H, NH₂) ; 6,50 (m, 2H, arom.) ; 6,70 (m, 1H, arom.) ; 7,00 (m, 6H, arom.) ; 7,15 (m, 2H, arom.) ; 7,30 (m, 2H, arom.) ; 8,00 (s, 1H, NH) ; 9,40 (s, 1H, NH).

### 7.3) chlorhydrate de [4-(phénylamino)phényl]-carbamate de 2-{4-{[2-thiényl(imino)méthyl]amino}phényl}-éthyle : 7

Le protocole expérimental utilisé est le même que celui décrit pour l'intermédiaire 2.3, l'intermédiaire 7.2 remplaçant le 4-amino-N-[4-(phénylamino)phényl]-benzèneacétamide. On obtient un solide blanc avec un rendement de 34%. Point de fusion : 153-159 °C. RMN ¹H (400 MHz, DMSO d6, δ) : 3,00 (m, 2H, CH₂) ; 4,30 (m, 2H, CH₂) ; 6,60-7,70 (m, 14H, arom.) ; 8,20 (m, 2H, thiophène) ; 8,90 (s, 1H, NH⁺) ; 9,50 (s, 1H, NH-CO) ; 9,90 (s, 1H, NH⁺) ; 11,70 (s, 1H, NH⁺).
IR : ν_{C=O} (carbamate) : 1719 cm⁻¹ ; ν_{C=N} (amidine) : 1598 cm⁻¹.

### Exemple 8 : chlorhydrate de N-{2-{4-{[2-thiényl(imino)méthyl]amino}phényl}éthyl}-N'-[4-(phénylamino)phényl]-urée : 8

### 8.1) N-[2-(4-nitrophényl)-éthyl]- N'-[4-(phénylamino)phényl]-urée :

Dans un ballon de 100 ml, sous argon, on dissout 0,5 g (1,7 mmole) de triphosgène dans 8 ml de dichlorométhane. Au moyen d'une seringue motorisée, on additionne en 1 heure une solution de 0,92 g (5 mmoles) de 4-aminodiphénylamine et de 1,44 ml (8,2 mmoles) de diisopropyléthylamine dans 15 ml de dichlorométhane. Cinq minutes après la fin de l'addition, on ajoute en une seule portion 1,01 g (5 mmoles) de chlorhydrate de 4-nitrophénétylamine suivie d'une solution de 1,44 ml (8,2 mmoles) de diisopropyléthylamine dans 10 ml de dichlorométhane. Après deux heures d'agitation à 20 °C, le mélange réactionnel est dilué par 50 ml de dichlorométhane et 20 ml d'eau. La phase organique est décantée et relavée par 20 ml d'eau. Après séchage sur MgSO₄ et filtration, la solution organique est concentrée partiellement sous vide. Le précipité formé est recueilli par filtration et rincé par du dichlorométhane. On obtient un solide jaune avec un rendement de 40%. Point de fusion : 204-205 °C.
RMN ¹H (100 MHz, DMSO d6, δ) : 2,96 (m, 2H, CH₂) ; 3,50 (m, 2H, CH₂-NH) ; 5,78 (m, 1H, HN-CH₂) ; 6,45 (s large, 1H, Ph-NH-CO) ; 6,72-7,49 (m, 11H, arom.) ; 7,81 (s large, 1H, NH) ; 8,15 (m, 2H, arom.).

### 8.2) N-[2-(4-aminophényl)-éthyl]- N'-[4-(phénylamino)phényl]-urée :

Dans un autoclave en acier inox équipé d'un barreau magnétique, on introduit une solution de l'intermédiaire 8.1 (0,68 g, 1,81 mmole) dans 40 ml d'un mélange THF/éthanol (3/1) ainsi que 0,1 g de Pd/C à 10%. Le mélange réactionnel est agité sous pression d'hydrogène (1,5 bar) pendant 1 heure à une température de 20 °C. Le Pd/C est ensuite éliminé par filtration et le filtrat est concentré sous vide. Le solide obtenu est lavé successivement par de l'acétate d'éthyle et du dichlorométhane. On obtient une poudre beige avec un rendement de 61%. Point de fusion > 260°C.
RMN ¹H (100 MHz, DMSO d6, δ) : 2,70 (m, 2H, CH₂) ; 3,40 (m, 2H, CH₂-NH) ; 5,18 (s large, 2H, NH₂) ; 6,07 (m, 1H, HN-CH₂) ; 6,60-7,45 (m, 13H, arom.) ; 8,00 (s large, 1H, NH) ; 8,41 (s large, 1H, Ph-NH-CO).

### 8.3) chlorhydrate de N-{2-{4-{[2-thiényl(imino)méthyl]amino}phényl}-éthyl}-N'-[4-(phénylamino)phényl]-urée : 8

Dans un ballon de 50 ml, on dissout 0,38 g (1,10 mmole) de l'intermédiaire 8.2 et 0,34 g (1,21 mmole) d'iodhydrate de S-méthyl-2-thiophène thiocarboximide dans 20 ml d'isopropanol. Le mélange réactionnel est agité pendant 20 heures à une température de 60 °C. Après évaporation du solvant sous vide, le résidu est repris dans 50ml d'un mélange 1/1 d'une solution saturée de Na₂CO₃ et d'acétate d'éthyle. Le milieu réactionnel est agité fortement et au bout de quelques instants un précipité apparaît. Celui-ci est recueilli, filtré et rincé successivement par de l'acétate d'éthyle et de l'eau. Après séchage, le précipité est purifié sur une colonne de gel de silice (éluant THF). Les fractions pures sont collectées et concentrées sous vide. Le solide obtenu (300 mg) est redissous dans 80 ml de THF auxquels on ajoute 2 ml d'une solution de HCl 1N dans l'éther éthylique. Le chlorhydrate formé précipite, il est filtré et rincé à l'aide de THF suivi d'éther éthylique pour conduire à une poudre gris clair. Point de fusion : le produit se transforme en mousse.
RMN ¹H (400 MHz, DMSO d6, δ) : 2,80 (m, 2H, CH₂) ; 3,37 (m, 2H, CH₂) ; 4,46 (s large, 1H, NH) ; 6,40 (s large, 1H, NH-CH₂) ; 6,70 (m, 1H, thiophène) ; 6,94 (m, 4H, arom.) ; 7,15 (m, 2H, arom.) ; 7,28 (m, 2H, arom.) ; 7,40 (m, 5H, arom.) ; 8,17 (m, 2H, thiophène) ; 8,78 (s large, 1H, Ph-NH-CO) ; 8,93 (s large, 1H, NH⁺) ; 9,84 (s large, 1H, NH⁺) ; 11,52 (s large, 1H, NH⁺).
IR : ν_{C=O} (urée) : 1654 cm⁻¹ ; ν_{C=N} (amidine) : 1598 cm⁻¹.

### Exemple 9: chlorhydrate de 4-{4-{[2-thiényl(imino)méthyl]amino}phényl}-N-[4-(phénylamino)phényl]-1-pipérazine acétamide : 9

### 9.1) 4-(nitrophényl)-1-pipérazine acétate d'éthyle :

Dans un ballon de 100 ml, on dissout 3 g (14,5 mmoles) de 1-(4-nitrophénylpipérazine) et 1,8 ml (15,9 mmoles) de bromoacétate d'éthyle dans 60 ml de dichlorométhane. Après addition de 2,42 ml (17,4 mmoles) de triéthylamine, le mélange réactionnel est agité, à 20 °C, pendant une heure. La solution est ensuite versée dans 100 ml d'eau et extraite à l'aide de 100 ml de dichlorométhane. Après décantation, la phase organique est séchée sur sulfate de magnésium, filtrée et concentrée sous vide. Le solide obtenu est repris par de l'éther éthylique, trituré et filtré. On obtient une poudre jaune avec un rendement de 89%. Point de fusion : 122,1-122,5 °C.
RMN ¹H (400 MHz, CDCl₃, δ) : 1,3 (t, 3H, CH₃, J = 7 Hz) ; 2,75 (m, 4H, pipérazine) ; 3,30 (s, 2H, CO-CH₂) ; 3,50 (m, 4H, pipérazine) ; 4,20 (q, 2H, CH₂-CH₃, J = 7 Hz) ; 7,45 (AB, 4H, Ph-NO₂).

### 9.2) Acide-4-(nitrophényl)-1-pipérazine acétique :

Dans un ballon contenant une solution de 3,8 g (13 mmoles) de l'intermédiaire 9.1 en solution dans 80 ml de THF, on ajoute goutte à goutte 32,4 ml d'une solution aqueuse 1M de LiOH, à 20 °C. Après une heure d'agitation, le mélange réactionnel est acidifié à pH = 5 par une solution 2N d'acide chlorhydrique. Le précipité obtenu est filtré et rincé par le minimum de THF et d'eau. Le produit est utilisé tel quel dans l'étape suivante.
RMN ¹H (100 MHz, D₂O, δ) : 3,30 (m, 4H, pipérazine) ; 3,60 (m, 6H, pipérazine + CO-CH₂) ; 7,45 (AB, 4H, Ph-NO₂).

### 9.3) 4-(4-nitrophényl)-N-[4-(phénylamino)phényl]-1-pipérazine acétamide :

Le protocole utilisé est le même que celui décrit pour l'intermédiaire 2.1, l'intermédiaire 9.2 remplaçant l'acide 4-nitrophénylacétique. On obtient un solide jaune avec un rendement de 84%. Point de fusion : 212-213 °C.
RMN ¹H (400 MHz, CDCl₃, δ) : 2,80 (m, 4H, pipérazine) ; 3,25 (s, 2H, CO-CH₂) ; 3,50 (m, 4H, pipérazine) ; 5,70 (s, 1H, NH) ; 6,90 (m, 3H, arom.) ; 7,10 (m, 4H, arom.) ; 7,30 (m, 2H, arom.) ; 7,85 (AB, 4H, Ph-NO₂) ; 8,90 (s, 1H, NHCO).

### 9.4) 4-(4-aminophényl)-N-[4-(phénylamino)phényl]-1-pipérazine acétamide :

Le protocole utilisé est le même que celui décrit pour l'intermédiaire 2.2, l'intermédiaire 9.3 remplaçant le 4-nitro-N-[4-(phénylamino)phényl]-benzèneacétamide. On obtient une huile marron avec un rendement de 71%.
RMN ¹H (400 MHz, CDCl₃, δ) : 2,80 (m, 4H, pipérazine) ; 3,15 (m, 4H, pipérazine) ; 3,20 (s, 2H, CO-CH₂) ; 5,70 (s, 1H, NH) ; 6,70 (m, 2H, arom.) ; 6,90 (m, 3H, arom.) ; 7,10 (m, 4H, arom.) ; 7,30 (m, 2H, arom.) ; 7,50 (m, 2H, arom.) ; 9,10 (s, 1H, NHCO).

### 9.5) chlorhydrate de 4-{4-{[2-thiényl(imino)méthyl]amino}phényl}-N-[4-(phénylamino)phényl]-1-pipérazine acétamide : 9

Le protocole utilisé est le même que celui décrit pour l'intermédiaire 2.3, l'intermédiaire 9.4 remplaçant le 4-amino-N-[4-(phénylamino)phényl]-benzèneacétamide. On obtient un solide jaune avec un rendement de 30%. Point de fusion : 230-240 °C.
RMN ¹H (400 MHz, DMSO d6, δ) : 3,10-3,50 (m, 4H, pipérazine) ; 3,65 (m, 2H, pipérazine) ; 3,90 (m, 2H, pipérazine) ; 4,30 (s, 2H, CO-CH₂) ; 6,80 (m, 1H, thiophène) ; 6,90-7,40 (m, 11H, arom.) ; 7,50 (m, 2H, arom.) ; 8,15 (m, 2H, thiophène) ; 8,75 (s, 1H, NH⁺) ; 9,80 (s, 1H, NH⁺) ; 10,9 (m, 2H, NHCO + NH⁺) ; 11,40 (s, 1H, NH+).
IR : ν_{C=O} (amide) : 1680 cm⁻¹ ; ν_{C=N} (amidine) : 1512 cm⁻¹.

### Exemple 10 : chlorhydrate de 1-{[(4-phénylamino)phénylamino]carbonyl}-4-{4-{[2-thiényl(imino)méthyl]amino}phényl}-pipérazine : 10

Le protocole expérimental utilisé est le même que celui décrit pour l'exemple 8. Le produit est salifié dans des conditions identiques au composé **2** sauf que le THF remplace le méthanol. On obtient une poudre jaune. Point de fusion : 239-240 °C.
RMN ¹H (400 MHz, DMSO d6, δ) : 3,30 (s large, 4H, pipérazine) ; 3,70 (s large, 4H, pipérazine) ; 5,80 (s large, 1H, NH) ; 6,73 (m, 1H, thiophène) ; 6,98 (m, 4H, arom.) ; 7,17 (m, 2H, arom.) ; 7,28-7,37 (m, 7H, arom.) ; 8,16 (m, 2H, thiophène) ; 8,65 (s large, 1H, Ph-NH-CO) ; 8,80 (s large, 1H, NH⁺) ; 9,80 (s large, 1H, NH⁺) ; 11,52 (s large, 1H, NH⁺).
IR : ν_{C=O} (urée) : 1654 cm⁻¹ ; ν_{C=N} (amidine) : 1597 cm⁻¹.

### Exempte 11 : chlorhydrate de 4-{[2-thiényl(imino)méthyl]amino}-N-[4-(phénylamino)phényl]-benzènebutanamine : 11

### 11.1) 4-Nitro-N-[4-(phénylamino)phényl]-benzènebutanamine :

Dans un tricol de 250ml sous atmosphère d'argon, on dissout 1,12 g (3 mmoles) de 4-nitro-N-[4-(phénylamino)phényl]-benzènebutanamide (obtenu dans les mêmes conditions que l'intermédiaire 2.1) dans 50 ml de THF anhydre. La solution est refroidie à l'aide d'un bain de glace, avant l'addition goutte à goutte de 15 ml (15 mmoles) d'une solution de diborane/THF. Le mélange réactionnel est chauffé à reflux pendant 5 heures. Après retour à 20 °C, 25 ml d'une solution d'HCl (6N) sont ajoutés lentement goutte à goutte et l'ensemble est porté à reflux pendant 2 heures. La solution est ensuite refroidie à l'aide d'un bain de glace avant l'addition d'une solution de soude à 20% jusqu'à pH basique. Le produit est extrait à l'aide d'éther éthylique (2 x 50 ml), la solution organique est lavée à la saumure (2 x 50 ml) et séchée sur sulfate de magnésium. Après filtration et concentration sous vide, le résidu est purifié sur une colonne de gel de silice (éluant : heptane/AcOEt 1/1). Les fractions pures sont collectées et évaporées sous vide pour conduire à une huile brune avec un rendement de 28%.
RMN ¹H (400 MHz, DMSO d6, δ) : 1,55 (m, 2H, CH₂) ; 1,71 (m, 2H, CH₂) ; 2,75 (m, 2H, CH₂-Arom) ; 2,98 (m, 2H, HN-CH₂); 5,29 (m, 1H, NH) ; 6,51-7,51 (m, 12H, Arom. + NH) ; 8,15 ( m, 2H, Ph-NO₂).

### 11.2) 4-Amino-N-[4-(phénylamino)phényl]-benzènebutanamine :

Le protocole expérimental utilisé est le même que celui décrit pour l'intermédiaire 2.2, l'intermédiaire 11.1 remplaçant le 4-nitro-N-[4-(phénylamino)phényl]-benzèneacétamide. On obtient une huile brune avec un rendement de 36%.
RMN ¹H (400 MHz, DMSO d6, δ) : 1,55 (m, 4H, 2 x CH₂) ; 2,44 (m, 2H, CH₂) ; 2,97 (m, 2H, CH₂) ; 4,81 (s, 2H, NH₂) ; 5,27 (m, 1H, NH) ; 6,47-7,10 (m, 13H, arom.) ; 7,49 (s, 1H, NH).

### 11.3) chlorhydrate de 4-{[2-thiényl(imino)méthyl]amino}-N-[4-(phénylamino)phényl]-benzènebutanamine : 11

Dans un ballon de 50 ml, on dissout 0,10 g (0,3 mmole) de l'intermédiaire 11.2 et 0,11 g (0,37 mmole) d'iodhydrate de S-méthyl-2-thiophène thiocarboximide dans 5 ml d'isopropanol additionné de 0,05 ml (0,6 mmole) de pyridine. Le mélange réactionnel est agité pendant 20 heures à une température de 23 °C. Après évaporation du solvant sous vide, le résidu est repris dans 25 ml d'un mélange (1/1) d'une solution saturée de NaHCO₃ et de dichlorométhane. Après décantation, la phase organique est lavée par 2 x 25 ml de saumure. La solution organique est séchée sur sulfate de magnésium, filtrée, concentrée sous vide et le résidu est purifié sur une colonne de gel de silice (éluant : dichlorométhane + 5% d'éthanol). Les fractions pures sont collectées et concentrées sous vide. On obtient une poudre rosée qui est salifiée par addition d'une solution d'HCl 1N dans l'éther éthylique (1 ml) à la solution de la base dans l'acétone. Après une heure d'agitation à 20 °C, le mélange réactionnel est filtré et la poudre est lavée successivement par 20 ml d'acétone et 20 ml d'éther éthylique. Point de fusion : 165-166 °C.
RMN ¹H (400 MHz, DMSO d6, δ) : 1,71 (m, 4H, 2 x CH₂) ; 2,66 (m, 2H, CH₂) ; 3,20 (m, 2H, CH₂) ; 6,85-7,41 (m, 14H, arom.) ; 8,16 (m, 2H, thiophène) ; 8,53 (s large, 1H, NH) ; 8,87 (s large, 1H, NH⁺) ; 9,83 (s large, 1H, NH+) ; 11,19 (s large, 2H, 2 x NH⁺) ; 11,56 (s large, 1H, NH⁺).
IR : ν_{C=N} (amidine) : 1595 cm⁻¹.

### Exemple 12: chlorhydrate de 3-{[2-thiényl(imino)méthyl]amino}-N-[4-(phénylamino)phényl]-benzènepropanamide : 12

### 12.1) mésylate de 3-nitrophényléthanol :

A une solution de 10 g (59,8 mmoles) de 3-nitrophényléthanol et de 8,31 ml (59,8 mmoles) de triéthylamine dans 120 ml de dichlorométhane, refroidie par un bain de glace, on ajoute goutte à goutte une solution de 4,63 ml (59,8 mmoles) de chlorure de méthane sulfonyle dilué par 20ml de dichlorométhane. L'agitation est maintenue 1 heure à 0 °C et 2 heures à 20 °C. Le mélange réactionnel est ensuite concentré sous vide et le résidu est repris par 125 ml d'acétate d'éthyle et 100 ml d'eau. Après agitation et décantation, la phase organique est lavée successivement par 100 ml d'eau et par 100 ml de saumure. La solution organique est séchée sur sulfate de magnésium, filtrée et concentrée sous vide. Le résidu d'évaporation est purifié sur une colonne de silice (éluant heptane/acétate d'éthyle 6/4) et les fractions pures sont collectées et évaporées pour conduire à une huile jaune avec un rendement de 71%.
RMN ¹H (100 MHz, CDCl₃, δ) : 3,00 (s, 3H, CH₃) ; 3,20 (t, 2H, CH₂, J = 5,8 Hz) ; 4,50 (t, 2H, CH₂) ; 7,60 (m, 2H, arom.) ; 8,20 (m, 2H, arom.).

### 12.2) 3-nitrobenzène-propanenitrile :

Dans un ballon de 100 ml, sous atmosphère d'argon, contenant une solution de 1,22 g (5 mmoles) de l'intermédiaire 12.1 dans 20 ml DMF sec, on introduit en une portion 0,49 g (10 mmoles) de NaCN. Le mélange réactionnel est chauffé à 60 °C pendant 3 heures et, après retour à 20 °C, versé dans 100 ml d'eau. La solution est extraite par 5x 50 ml d'acétate d'éthyle, les phases organiques sont rassemblées et lavées successivement par 100 ml d'eau et 100 ml de saumure. Après séchage sur sulfate de magnésium, la solution organique est concentrée sous vide et le résidu est purifié sur colonne de silice (éluant : heptane/acétate d'éthyle : 7/3). Les fractions pures sont collectées et concentrées sous vide pour conduire à une poudre jaune claire avec un rendement de 78%. Point de fusion : 86-88 °C.
RMN ¹H (100 MHz, CDCl₃, δ) : 2,70 (t, 2H, CH₂, J = 5,8 Hz) ; 3,10 (t, 2H, CH₂) ; 7,60 (m, 2H, arom.) ; 8,20 (m, 2H, arom.).

### 12.3) acide 3-nitrobenzène propanoïque :

Une solution de 2,33 g (19,2 mmoles) de l'intermédiaire 12.2 dans 100 ml d'une solution aqueuse à 10% d'HCl et 100 ml d'éthanol est portée à reflux pendant 72 heures. Après retour à 20 °C, le mélange réactionnel est concentré à sec sous vide. Le résidu est repris par 100 ml d'acétate d'éthyle et lavé par 3 x 100 ml d'eau et par 50 ml de saumure. Après séchage sur sulfate de sodium, la solution organique est filtrée et concentrée sous vide. Le résidu d'évaporation est purifié sur une colonne de silice (éluant : heptane/acétate d'éthyle 95/5 jusqu'à 80/20). On obtient une poudre jaune pâle avec un rendement de 21%. Point de fusion : 107-109 °C.
RMN ¹H (100 MHz, CDCl₃, δ) : 2,70 (m, 2H, CH₂) ; 3,10 (m, 2H, CH₂) ; 5,40 (s large, 1H, 7,50 (m, 2H, arom.) ; 8,10 (m, 2H, arom.).

### 12.4) 3-nitro-N-[4-(phénylamino)phényl]-benzènepropanamide :

Le protocole expérimental utilisé est le même que celui décrit pour l'intermédiaire 2.1, l'intermédiaire 12.3 remplaçant l'acide 4-nitrophénylacétique. On obtient une poudre brune avec un rendement de 70%. Point de fusion : 130-132 °C.
RMN ¹H (CDCl₃, 100 MHz, δ) : 2,70 (t, 2H, CH₂, J = 5,8 Hz) ; 3,20 (t, 2H, CH₂) ; 5,70 (s large, 1H, NH) ; 6,90-7,60 (m, 13H, arom.).

### 12.5) 3-amino-N-[4-(phénylamino)phényl]-benzènepropanamide :

Le protocole expérimental utilisé est le même que celui décrit pour l'intermédiaire 2.2, l'intermédiaire 12.4 remplaçant le 4-nitro-N-[4-(phénylamino)phényl]-benzèneacétamide. On obtient une poudre blanche avec un rendement de 64%. Point de fusion : 164-166 °C.
RMN ¹H (CDCl₃, 100 MHz, δ) : 2,80 (m, 2H, CH₂) ; 3,50 (m, 2H, CH₂) ; 5,10 (s large, 2H, NH₂); 6,50 (m, 3H, arom.) ; 6,80-7,45 (m, 8H, arom.) ; 7,60 (m, 2H, arom.) ; 8,15 (s, 1H, NH) ; 9,88 (s, 1H, NH-CO).

### 12.6) chlorhydrate de 3-{[2-thiényl(imino)méthyl]amino}-N-[4-(phénylamino)phényl]-benzènepropanamide : 12

Le protocole expérimental utilisé est le même que celui décrit pour l'intermédiaire 2.3, l'intermédiaire 12.5 remplaçant le 4-amino-N-[4-(phénylamino)phényl]-benzèneacétamide. On obtient, après salification, une poudre beige claire avec un rendement de 78%. Point de fusion : 228-230 °C.
RMN ¹H (400 MHz, DMSO d6, δ) : 2,70 (m, 2H, CH₂) ; 2,96 (m, 2H, CH₂) ; 5,20 (s large, 1H, NH) ; 6,74 (m, 1H, thiophène) ; 7,00 (m, 4H, arom.) ; 7,19 (m, 2H, arom.) ; 7,29 (m, 1H, arom.) ; 7,39 (m, 3H, arom.) ; 7,47 (m, 3H, arom.) ; 8,18 (m, 2H, thiophène) ; 8,96 (s large, 1H, NH⁺) ; 9,90 (s large, 1H, NH⁺) ; 10,07 (s, 1H, NH-CO) ; 11,60 (s large, 1H, NH⁺).
IR : ν_{C=O} (amide) : 1649 cm⁻¹ ; ν_{C=N} (amidine) : 1596 cm⁻¹.

### Exemple 13: 4-(4-{[amino(2-thiényl)méthylidène]amino}phényl)-N-[2-(4-toluidino)phényl]butanamide : 13

### 13.1) N'-(4-méthylphényl)-1,2-benzènediamine :

La réduction de la fonction nitro de la N-(4-méthylphényl)-2-nitroaniline (*Synthesis* (1990) 430) est effectuée en présence de Pd/C dans de l'éthanol, dans les conditions décrites précédemment pour l'intermédiaire 2.2. On obtient un produit violet sous forme mi-huile mi-cristaux avec un rendement de 90%.

### 13.2) 4-(4-{[amino(2-thiényl)méthylidène]amino}phényl)-N-[2-(4-toluidino)phényl]butanamide :

Le protocole expérimental utilisé est le même que celui décrit pour l'exemple 2, à partir de l'acide 4-nitrophénylbutyrique et de l'intermédiaire 13.1. Le produit est isolé sous forme de base libre. Solide blanc. Point de fusion : 66-68 °C.

### Exemple 14 : 4-anilinophényl-4-(4-{[amino(2-thiényl)méthylidène] amino}-phényl)butanoate : 14

### 14.1) 4-anilinophényl 4-(4-nitrophényl)butanoate :

A une solution de 1,25 g (5,96 mmoles) d'acide 4-nitrophénylbutyrique dans 25 ml de CH₂Cl₂, on ajoute lentement 0,98 g (6,02 mmoles) de 1,1'-carbonyldiimidazole à 20 °C. Le mélange réactionnel est agité 30 minutes avant addition de 1 g (5,42 mmoles) de 4-hydroxy-diphénylamine. Après 3 heures d'agitation, la réaction est arrêtée par addition de 3 ml de MeOH et le solvant est évaporé sous vide. Le résidu d'évaporation est purifié sur une colonne de silice (éluant : Heptane/AcOEt : 100/0 à 80/20). On obtient un solide jaune avec un rendement de 89%.

### 14.2) 4-anilinophényl 4-(4-{[amino(2-thiényl)méthylidène]amino}phényl) butanoate :

Le protocole expérimental utilisé est le même que celui décrit pour les intermédiaires 2.2 et 2.3 à partir de l'intermédiaire 14.1. Le produit est isolé sous forme de base libre. Solide jaune pâle. Point de fusion : 147-148 °C.

### Exemple 15 : 4-(4-{[amino(2-thiényl)méthylidène]amino}phényl)-N-[2-(4-toluidino)phényl]butanamide : 15

### 15.1) N-{4-[4-(4-nitrophényl)butoxy]phényl}-N-phénylamine .

Dans un ballon contenant 10 ml de CH₂Cl₂, on introduit successivement 2,0 g (10,88 mmoles) de 4-hydroxy-diphénylamine, 2,0 ml (12 mmoles) de 4-(4-nitrophényl)-1-butanol et 1,66 ml (12 mmoles) de tributylphosphine. On ajoute ensuite, goutte-à-goutte, 1,90 ml (12 mmoles) de diéthylazodicarboxylate et l'ensemble est agité à 20 °C pendant 16 heures. Le solvant est évaporé sous vide et le résidu est purifié sur une colonne de silice (éluant : Heptane/AcOEt: 100/0 à 80/20). Le produit attendu est obtenu sous forme d'une huile rouge foncée avec un rendement de 35%.

### 15.2) N'-{4-[4-(4-anilinophénoxy)butyl]phényl}-2-thiophènecarboximidamide :

Le protocole expérimental utilisé est le même que celui décrit pour les intermédiaires 2.2 et 2.3 à partir de l'intermédiaire 14.1. Le produit est isolé sous forme de base libre. Solide blanc. Point de fusion : 120-121 °C.

### Exemple 16 : N'-{4-[4-(3-anilinophénoxy)butyl]phényl}-2-thiophènecarboximidamide : 16

Le protocole expérimental utilisé est le même que celui décrit pour l'exemple 15, à partir de la 3-hydroxy-diphénylamine. Le produit est isolé sous forme de base libre. Solide blanc. Point de fusion : 73-74 °C.

### Exemple 17 : N'-(9H-carbazol-3-yl)-2-thiophènecarboximidamide : 17

Le protocole expérimental utilisé est le même que celui décrit pour l'exemple 1, à partir du 3-aminocarbazole *(Pharmazie* (1993) **48(11)**, 817-820). Le produit est isolé sous forme de base libre. Solide beige clair. Point de fusion : 243-244 °C.

### Exemple 18 : chlorhydrate de 4-(4-{[amino(2-thiényl)méthylidène]amino}phényl)-N-(9H-carbazol-3-yl)butanamide : 18

Le protocole expérimental utilisé est le même que celui décrit pour l'exemple 2, à partir du 3-aminocarbazole *(*Pharmazie (1993) 48(11), 817-820) et de l'acide 4-nitrophénylbutyrique. Solide beige clair. Point de fusion > 250 °C.
MS : MH⁺ : 452,2.

### Exemple 19 : iodhydrate de N'-[4-(10H-phénothiazin-2-yloxy)phényl]-2-thiophènecarboximidamide : 19

### 19.1) 2-(4-nitrophénoxy)-10H-phénothiazine :

Dans un ballon, sous atmosphère d'argon, on mélange 1,1 g (5,11 mmoles) de 2-hydroxy-10*H*-phénothiazine *(*J. Med. Chem. (1992) 35, 716), 1,34 g (9,71 mmoles) de K₂CO₃ et 0,94 g (6,64 mmoles) de 4-fluoro-1-nitrobenzène dans 25 ml de DMF anhydre. Le mélange réactionnel est chauffé à 70 °C pendant 18 heures. Le solvant est ensuite évaporé sous vide et le résidu est repris par 50 ml d'AcOEt et 50 ml d'eau. Après agitation et décantation, la phase organique est lavée par 50 ml de saumure. La solution organique est séchée sur MgSO₄, filtrée et concentrée sous vide. Le résidu est cristallisé dans l'éther diisopropylique. Après séchage, on obtient un solide jaune avec un rendement de 83%. Point de fusion : 210-211 °C.

### 19.2) iodhydrate de N'-[4-(10H-phénothiazin-2-yloxy)phényl]-2-thiophènecarboximidamide :

Le protocole expérimental utilisé est le même que celui décrit pour les intermédiaires 2.2 et 2.3, à partir de l'intermédiaire 19.1. Le produit final attendu précipite directement dans le mélange réactionnel, il est isolé par filtration et lavé à l'aide d'iPrOH. Solide jaune. Point de fusion : 175-180 °C.

### Exempte 20 : chlorhydrate de N'-{4-[(10-méthyl-10H-phénothiazin-2-yl)oxy]phényl}-2-thiophènecarboximidamide : 20

### 20.1) 10-méthyl-10H-phénothiazin-2-yl 4-nitrophényléther :

Dans un ballon, sous atmosphère d'argon, contenant une solution de 0,1 g (0,29 mmole) de l'intermédiaire 19.1 dans 10 ml de DMF anhydre, on ajoute 0,014 g (0,58 mmole) de NaH (60%). L'agitation est maintenue, à 20 °C, pendant 16 heures. On additionne ensuite 0,04 ml (0,58 mmole) de Mel au mélange réactionnel, sous agitation à 20 °C. A la fin de la réaction, l'ensemble est versé sur 50 ml d'eau glacée et le produit est extrait à l'aide de 50 ml d'AcOEt. La phase organique est décantée, lavée avec 50 ml de saumure, séchée sur MgSO₄, filtrée et concentrée sous vide. Le résidu est purifié sur une colonne de silice (éluant : Heptane/AcOEt : 80/20). On obtient une huile orange avec un rendement de 50%.

### 20.2) chlorhydrate de N'-{4-[(10-méthyl-10H-phénothiazin-2-yl)oxy]phényl}-2-thiophènecarboximidamide :

Le protocole expérimental utilisé est le même que celui décrit pour les intermédiaires 2.2 et 2.3, à partir de l'intermédiaire 20.1. On obtient un solide blanc. Point de fusion : 256-257 °C.

### Exemple 21 : chlorhydrate de 4-(4-{[amino(2-thiényl)méthylidène] amino}phényl)-N-(10H-phénothiazin-3-yl)butanamide : 21

### 21.1) 3-amino-10H-phénothiazine :

La réduction de la fonction nitro de la 3-nitro-10*H*-phénothiazine *(*J. Org. Chem. (1972) 37, 2691) est effectuée en présence de Pd/C dans un mélange EtOH/THF dans les conditions décrites pour l'intermédiaire 2.2. On obtient un solide gris avec un rendement de 97%. Point de fusion : 150-156°C.

### 21.2) chlorhydrate de 4-(4-{[amino(2-thiényl)méthylidène]amino} phényl)-N-(10H-phénothiazin-3-yl)butanamide :

Le protocole expérimental utilisé est le même que celui décrit pour l'exemple 2, à partir de l'acide 4-nitrophénylbutyrique et de l'intermédiaire 21.1. Solide vert clair. Point de fusion : 170-176 °C.

### Exemple 22 : dichlorhydrate de N'-(4-{4-[2-(10H-phénothiazin-2-yloxy)éthyl]-1-pipérazinyl}phényl)-2-thiophènecarboximidamide : 22

### 22.1) 2-[4-(4-nitrophényl)-1-pipérazinyl]-1-éthanol :

A un mélange de 10,35 g (50 mmoles) de 1-(4-nitrophényl)pipérazine, de 7,6 g (55 mmoles) de K₂CO₃ et de 9 ml (65 mmoles) de Et₃N dans 200 ml de CH₂Cl₂, sous atmosphère d'argon, on ajoute 7,5 g (60 mmoles) de 2-bromoéthanol. L'ensemble est alors chauffé à 45 °C pendant 18 heures. Le mélange réactionnel est finalement dilué par 50 ml d'eau, agité et décanté. La phase organique est lavée par 50 ml de saumure, séchée sur MgSO₄, filtrée et concentrée sous vide. Le résidu est cristallisé dans de l'éther diisopropylique. On obtient un solide jaune avec un rendement de 89%. Point de fusion : 98-99 °C.

### 22.2) 1-(2-bromoéthyl)-4-(4-nitrophényl)pipérazine :

A une solution de 5 g (20 mmoles) de l'intermédiaire 22.1 dans 75 ml de CH₂Cl₂, on ajoute 8,6 g (26 mmoles) de CBr₄. L'ensemble est refroidi à l'aide d'un bain de glace avant l'addition, par portions, de 6,3 g (24 mmoles) de triphénylphosphine. L'agitation est maintenue 2 heures à 20 °C. Après addition de 50 ml d'eau, agitation et décantation, la phase organique est lavée par 50 ml de saumure, séchée sur MgSO₄, filtrée et concentrée sous vide. Le résidu d'évaporation est purifié sur une colonne de silice (éluant CH₂Cl₂/EtOH : 95/5) et finalement cristallisé dans de l'éther éthylique. On obtient un solide jaune-orange avec un rendement de 40%. Point de fusion : 134-135 °C.

### 22.3) 2-{2-[4-(4-nitrophényl)-1-pipérazinyl]éthoxy}-10H-phénothiazine :

Le protocole expérimental utilisé est le même que celui décrit pour l'intermédiaire 19.1, à partir de l'intermédiaire 22.2. On obtient un solide jaune avec un rendement de 43%. Point de fusion : 224-225 °C.

### 22.4) dichlorhydrate de N'-(4-{4-[2-(10H-phénothiazin-2-yloxy)éthyl]-1-pipérazinyl}-phényl)-2-thiophènecarboximidamide :

Le protocole expérimental utilisé est le même que celui décrit pour les intermédiaires 2.2 et 2.3 à partir de l'intermédiaire 22.3. Solide beige clair. Point de fusion : 198-200 °C.

### Exemple 23: chlorhydrate de 4-(4-{[amino(2-thiényl)méthylidène]amino} phényl)-N-[4-(4-toluidino)phényl]butanamide : 23

### 23.1) N'-(4-méthylphényl)-1,4-benzènediamine :

La réduction de la fonction nitro de la N-(4-méthylphényl)-4-nitroaniline *(*Indian J. Chem. (1981) 20B, 611-613) est effectuée en présence de Pd/C dans de l'éthanol, dans les conditions décrites pour l'intermédiaire 2.2. On obtient un solide gris avec un rendement de 85%.

### 23.2) chlorhydrate de 4-(4-{[amino(2-thiényl)méthylidène]amino} phényl)-N-[4-(4-toluidino)phényl]butanamide :

Le protocole expérimental utilisé est le même que celui décrit pour l'exemple 2, à partir de l'intermédiaire 22.3 et de l'acide 4-nitrophénylbutyrique. Solide jaune. Point de fusion : 142-145 °C.

### Exemple 24 : 3-anilinophényl 4-(4-{[amino(2-thiényl)méthylidène]amino}-phényl)butanoate : 24

Le protocole expérimental utilisé est le même que celui décrit pour l'exemple 14, à partir de la 3-hydroxy-diphénylamine et de l'acide 4-nitrophénylbutyrique. Solide blanc. Point de fusion : 110-112 °C.

### Exemple 25 : chlorhydrate de 2-(4-{[amino(2-thiényl)méthylidène]amino} phényl)-N-[2-(9H-carbazol-4-yloxy)éthyl]acétamide : 25

### 25.1) 3-(2-bromoéthoxy)-9H-carbazole :

On chauffe pendant 5 heures, à reflux, un mélange de 1,83 g (10 mmoles) de 4-hydroxycarbazole (J. Chem. Soc. (1955), 3475-3477 ; J. Med. Chem. (1964) 7, 158-161), 1,08 ml (12,5 mmoles) de 1,2-dibromoéthane et de 2,6 ml (10,5 mmoles) d'une solution aqueuse 4 M de NaOH dans 2 ml d'eau. Après retour à 20 °C, le produit est extrait à l'aide de 2 fois 30 ml de CH₂Cl₂. Les solutions organiques collectées sont ensuite successivement lavées par 20 ml d'eau et 20 ml de saumure. Après séchage sur MgSO₄, filtration et concentration sous vide, le résidu est purifié sur une colonne de silice (éluant : Heptane/AcOEt : 80/20). On obtient une poudre beige avec un rendement de 32%. Point de fusion : 135-136 °C.

### 25.2) 3-(2-azidoéthoxy)-9H-carbazole :

Un mélange de 0,9 g (3,1 mmoles) de l'intermédiaire 25.1 et de 0,20 g (3,1 mmoles) de NaN₃ dans 10 ml de DMF est chauffé à 70 °C pendant 1 heure. L'ensemble est ensuite versé dans 30 ml d'un mélange eau et glace. Après addition de 50 ml d'AcOEt et agitation, la phase organique est décantée et lavée successivement par 20 ml d'eau et 20 ml de saumure. La solution organique est ensuite séchée sur Na₂SO₄, filtrée et concentrée sous vide. Après séchage, on obtient une poudre beige (rendement quantitatif) qui est utilisée telle quelle dans l'étape suivante.

### 25.3) 2-(9H-carbazol-3-yloxy)éthylamine :

Dans un autoclave en acier inox équipé d'un barreau magnétique, on introduit une solution de l'intermédiaire 25.2 dans 50 ml d'EtOH ainsi que 0,3 g de Pd/C (10%). Le mélange réactionnel est agité pendant 2 heures sous 1,5 bar d'H₂ à la température de 25 °C. Le Pd/C est ensuite éliminé par filtration et le filtrat est concentré à sec sous vide. Le résidu est repris dans de l'éther éthylique et les cristaux formés sont filtrés et rincés abondamment par de l'éther éthylique. Après séchage, on obtient une poudre blanche avec un rendement de 82%. Point de fusion : 145-146 °C.

### 25.4) chlorhydrate de 2-(4-{[amino(2-thiényl)méthylidène]amino} phényl)-N-[2-(9H-carbazol-4-yloxy)éthyl]acétamide :

Le protocole expérimental utilisé est le même que celui décrit pour l'exemple 2, à partir de l'intermédiaire 25.3 et de l'acide 4-nitrophénylbutyrique. Solide beige clair. Point de fusion : 233-234 °C.

### Exemple 26 : chlorhydrate de N-(4-{[amino(2-thiényl)méthylidène] amino}phenéthyl)-2-anilinobenzamide : 26

Le protocole expérimental utilisé est le même que celui décrit pour l'exemple 2, à partir de l'acide N-phénylanthranilique et de la 4-nitrophénétylamine. Solide jaune pâle. Point de fusion : 163-165 °C.

### Exemple 27 : chlorhydrate de N-(4-{[amino(2-thiényl)méthylidène] amino}phénéthyl)-2-(2,3-diméthylanilino)benzamide : 27

Le protocole expérimental utilisé est le même que celui décrit pour l'exemple 2, à partir de l'acide méfénamique et de la 4-nitrophénétylamine. Solide jaune pâle. Point de fusion: 168-170°C.

### Exemple 28 : dichlorhydrate de N'-{4-[4-(2-anilinobenzoyl)-1-pipérazinyl]phényl}-2-thiophènecarboximidamide : 28

Le protocole expérimental utilisé est le même que celui décrit pour l'exemple 2, à partir de l'acide N-phénylanthranilique et de la 4-nitrophénylpipérazine. Solide jaune pâle. Point de fusion : 168-170 °C.

### Exemple 29 : dichlorhydrate de N'-(4-{4-[2-(2,3-diméthylanilino) benzoyl]-1-pipérazinyl}phényl)-2-thiophènecarboximidamide : 29

Le protocole expérimental utilisé est le même que celui décrit pour l'exemple 2, à partir de l'acide méfénamique et de la 4-nitrophénylpipérazine. Solide jaune pâle. Point de fusion : 166-168 °C.

### Exemple 30: chlorhydrate de 4-(4-{[amino(2-thiényl)méthylidène]amino} phényl)-N-(4-phénoxyphényl)butanamide : 30

Le protocole expérimental utilisé est le même que celui décrit pour l'exemple 14, à partir de l'acide 4-nitrophénylbutyrique et du 4-phénoxyphénol. Solide jaune pâle. Point de fusion : 119-123 °C.

### Exemple 31 : chlorhydrate de N-(4-{[amino(2-thiényl)méthylidène] amino}phénéthyl)-4-(4-hydroxyphénoxy)benzamide : 31

Le protocole expérimental utilisé est le même que celui décrit pour l'exemple 2, à partir de l'acide 4-(4-hydroxyphénoxy)benzoïque et de la 4-nitrophénétylamine. Solide jaune pâle. Point de fusion : 155-157 °C.

### Exemple 32 : N-[2-(9H-carbazol-4-yloxy)éthyl]-2-thiophènecarboximidamide : 32

Le protocole expérimental utilisé est le même que celui décrit pour l'intermédiaire 2.3, à partir de l'intermédiaire 25.3. Le produit attendu est isolé sous forme de base libre. Solide blanc. Point de fusion : 180-181 °C.

### Exempte 33 : N-[3-(9H-carbazol-4-yloxy)propyl]-2-thiophènecarboximidamide : 33

### 33.1) 2-[3-(9H-carbazol-4-yloxy)propyl]-1H-isoindole-1,3(2H)dione :

A une suspension, sous argon, de 0,23 g (5,73 mmoles) de NaH (60%) dans 20 ml de DMF anhydre, on ajoute 1 g (5,46 mmoles) de 4-hydroxycarbazole (J. Chem. Soc. (1955), 3475-3477 ; J. Med. Chem. (1964) 7, 158-161). Après une demi-heure d'agitation à 20 °C, 1,46 g (5,46 mmoles) de 3-bromopropylphtalimide en solution dans 10 ml de DMF anhydre sont additionnés goutte-à-goutte au mélange réactionnel. L'ensemble est chauffé à 80 °C pendant 16 heures. Après retour à 20 °C, on ajoute 5 ml d'eau et on concentre le mélange sous vide. Le résidu est repris par 300 ml de CH₂Cl₂ et la solution organique est lavée successivement par 50 ml de NaOH 1 M, 100 ml d'eau et 100 ml de saumure. Après séchage sur MgSO₄, filtration et concentration sous vide, on obtient un résidu huileux qui cristallise lentement. Les cristaux sont lavés à l'aide d'éther éthylique. On obtient un solide beige avec un rendement de 40%. Point de fusion 171-172°C.

### 33.2) 3-(9H-carbazol-4-yloxy)propylamine :

A une solution de 0,8 g (2,16 mmoles) de l'intermédiaire 33.1 dans 30 ml d'éthanol, chauffée à reflux, on ajoute goutte-à-goutte une solution de 0,13 ml (3,24 mmoles) d'hydrate d'hydrazine dans 5 ml d'éthanol. Le mélange réactionnel est agité et chauffé à reflux pendant 4 heures. Après retour à 20 °C, le produit est partitionné entre 100 ml d'AcOEt et 50 ml de NaOH 1 M. Après décantation, la phase organique est lavée successivement par 50 ml d'eau et 50 ml de saumure. La solution organique est séchée sur Na₂SO₄, filtrée et concentrée sous vide. On obtient une poudre beige avec un rendement de 41%. Point de fusion : 146-147 °C.

### 33.3) N-[3-(9H-carbazol-4-yloxy)propyl]-2-thiophènecarboximidamide :

Le protocole expérimental utilisé est le même que celui décrit pour l'intermédiaire 2.3, à partir de l'intermédiaire 33.2. Le produit attendu est isolé sous forme de base libre. Solide beige pâle. Point de fusion : 189-190 °C.

### Exemple 34 : iodhydrate de N-{4-[4-(10H-phénothiazin-2-yloxy)butyl]phényl}-2-thiophènecarboximidamide : 34

Le protocole expérimental utilisé est le même que celui décrit pour l'exemple 15, à partir de la 4-(4-nitrophényl)-1-butanol et de 2-hydroxy-10*H*-phénothiazine *(*J. Med. Chem. (1992) 35, 716). Le produit final attendu précipite directement dans le mélange réactionnel, il est isolé par filtration et lavé à l'aide d'iPrOH. Solide jaune. Point de fusion : 262-270°C.

### Exemple 35 : trichlorhydrate de 3-[(3-{[amino(2-thiényl) méthylidène]amino}-benzyl)amino]-N-(4-anilinophényl)propanamide : 35

### 35.1) tert-butyl 3-(4-anilinoanilino)-3-oxopropyl carbamate :

Le protocole expérimental utilisé est le même que celui décrit pour l'intermédiaire 2.1, à partir de la Boc-β-Alanine et de la 4-aminodiphénylamine. Après une filtration rapide sur colonne de silice (éluant : Heptane/AcOEt : 1/1), on obtient le produit attendu avec un rendement quantitatif.

### 35.2) 3-amino-N-(4-anilinophényl)propanamide :

On dissout 15 g (42,2 mmoles) de l'intermédiaire 35.1 dans 300 ml d'AcOEt et on ajoute 120 ml d'une solution aqueuse d'HCl 6 N. Le mélange réactionnel est agité fortement, à 20 °C, pendant 1 heure. Après décantation, la phase aqueuse est recueillie et rendue basique (pH > 11) par addition d'une solution aqueuse de NaOH 2 M. Le produit est alors extrait à l'aide de 2 fois 50 ml de CH₂Cl₂ et la phase organique est lavée par 50 ml de saumure. Après séchage sur MgSO₄, filtration et concentration sous vide, le résidu est purifié sur une colonne de silice (éluant : CH₂Cl₂/EtOH/NH₄OH (20%) : 20/5/0,5). On obtient une poudre violette avec un rendement de 73%. Point de fusion : 108-110 °C.

### 35.3) N-(4-anilinophényl)-3-[(3-nitrobenzyl)amino]propanamide :

Dans un ballon contenant 100 ml de MeOH anhydre, sous atmosphère inerte, on ajoute successivement 1,40 g (5,5 mmoles) de l'intermédiaire 35.2, 0,92 g (6 mmoles) de 3-nitrobenzaldéhyde et 3 g de tamis moléculaire 4 Å pulvérulent préalablement activé. Le mélange réactionnel est agité vigoureusement pendant 15 heures avant l'addition, par portions, de 0,24 g (6 mmoles) de NaBH₄. L'agitation est maintenue 4 heures supplémentaires avant addition de 10 ml d'eau. Après un quart d'heure, le tamis est filtré et le mélange réactionnel est extrait par 2 fois 100 ml de CH₂Cl₂. La phase organique est lavée successivement par 50 ml d'eau, 50 ml de saumure, séchée sur sulfate de sodium, filtrée et concentrée sous vide. Le résidu est purifié sur une colonne de silice (éluant : CH₂Cl₂/EtOH : 20/1). On obtient une huile orange avec un rendement de 94%.

### 35.4) 3-[(3-aminobenzyl)amino]-N-(4-anilinophényl)propanamide :

La réduction de la fonction nitro de l'intermédiaire 35.3 est effectuée en présence de Pd/C dans de l'éthanol, dans les conditions décrites pour l'intermédiaire 2.2. Après filtration du Pd/C et concentration sous vide, le produit est utilisé directement dans l'étape suivante.

### 35.5) trichlorhydrate de 3-[(3-{[amino(2-thiényl)méthylidène]amino}-benzyl)amino]-N-(4-anilinophényl)propanamide :

Dans un ballon de 50 ml, on dissout 0,50 g (1,40 mmole) de l'intermédiaire 35.4 et 0,50 g (1,75 mmole) de iodhydrate de S-méthyl-2-thiophène thiocarboximide dans 15 ml d'isopropanol et 15 ml de DMF, en présence de 0,11 ml (1,40 mmole) de pyridine. Le mélange réactionnel est agité pendant 20 heures à 23 °C. Après évaporation du solvant sous vide, le résidu est repris dans 100 ml d'un mélange (1/1) de soude 1N et d'acétate d'éthyle. Après décantation, la phase organique est lavée par 50 ml d'eau suivi de 50 ml de saumure. La solution organique est séchée sur sulfate de magnésium, filtrée, concentrée sous vide et le résidu est purifié sur une colonne de gel de silice (éluant : CH₂Cl₂/EtOH/NH₄OH (20%) : 20/5/0,5). Les fractions pures sont collectées et concentrées sous vide. Le composé est ensuite dissous dans du méthanol et salifié par addition d'une solution d'HCl 1N dans l'éther éthylique (10 ml). Après une heure d'agitation à 20 °C, le mélange réactionnel est concentrée sous vide pour conduire à une poudre jaune pâle. Point de fusion : 184-186°C.

### Exemple 36 : N'-(4-{2-[(10H-phénothiazin-3-ylméthyl)amino]éthyl} phényl)-2-thiophènecarboximidamide : 36

Le protocole expérimental utilisé est le même que celui décrit pour les intermédiaires 35.3, 35.4 et 35.5, à partir de la 10*H-*phénothiazine-3-carbaldéhyde (J. Chem. Soc. (1951), 1834 ; Bull. Soc. Chim. Fr. (1969), 1769) et de la 4-nitrophénétylamine. Mousse beige. MS : MH+: 457,1.

### Exemple 37 : chlorhydrate de N-(4-{[amino(2-thiényl)méthylidène]amino}phénéthyl)-2-méthoxy-10H-phénothiazine-1-carboxamide : 37

Le protocole expérimental utilisé est le même que celui décrit pour l'exemple 2, à partir de l'acide 2-méthoxy-10*H*-phénothiazine-1-carboxylique (J. Med. Chem. (1992) 35(4), 716-724) et de la 4-nitrophénétylamine. Solide jaune pâle. Point de fusion > 200 °C (décomposition).

### Exemple 38 : N'-[4-(2-{[(2-méthoxy-10H-phénothiazin-1-yl)méthyl] amino}éthyl)phényl]-2-thiophènecarboximidamide : 38

### 38.1) 2-méthoxy-10H-phénothiazine-1-carbaldéhyde :

Dans un tricol, sous atmosphère d'argon, contenant 140 ml d'éther éthylique anhydre, on dissout 4,6 g (20 mmoles) de 2-méthoxy-10*H*-phénothiazine. On ajoute ensuite, goutte-à-goutte, 20 ml (50 mmoles) d'une solution de nBuLi (2,5 M) dans l'hexane, à 20 °C. Le mélange réactionnel est agité 3 heures à 20°C avant l'addition, goutte-à-goutte, de 6,2 ml (80 mmoles) de DMF anhydre. L'agitation est maintenue à 20 °C pendant 15 heures supplémentaires. L'ensemble est ensuite versé sur 150 ml d'eau glacé et le produit est extrait à l'aide de 2 fois 200 ml d'acétate d'éthyle. La solution organique est lavée par 100 ml de saumure, séchée sur MgSO₄, filtrée et concentrée sous vide. Le résidu d'évaporation est repris par de l'éther diisopropylique, filtré et séché pour conduire à un solide rouge avec un rendement de 30%. Point de fusion : 155-160°C.

### 38.2) N'-[4-(2-{[(2-méthoxy-10H-phénothiazin-1-yl)méthyl]amino} éthyl)phényl]-2-thiophènecarboximidamide :

Le protocole expérimental utilisé est le même que celui décrit pour l'exemple 36, à partir de l'intermédiaire 38.1 et de la 4-nitrophénétylamine. Solide gris. MS : MH+: 487,2.

### Exemple 39 : N'-{4-[(10H-phénothiazin-2-yloxy)méthyl]phényl}-2-thiophènecarboximidamide : 39

### 39.1) 2-[(4-nitrobenzyl)oxy]-10H-phénothiazine :

Dans un ballon, sous atmosphère d'argon, on dissout 1,08 g (5 mmoles) de 2-hydroxy-10*H*-phénothiazine *(*J. Med. Chem. (1992) 35, 716) dans 20 ml de THF anhydre. La solution est refroidie à 0°C et on ajoute, par portions, 0,22 g (5,5 mmoles) de NaH (60%). Après 15 minutes d'agitation, on additionne par portions 1,2 g (5,5 mmoles) de bromure de 4-nitrobenzyle et le mélange réactionnel est agité 15 heures, à 20 °C, avant d'être versé dans 50 ml d'eau glacée. Le produit est extrait par 2 fois 25 ml de CH₂Cl₂ et la solution organique est successivement lavée par 25 ml d'eau et 25 ml de saumure. Après séchage sur MgSO₄, filtration et concentration sous vide, le résidu d'évaporation est purifié sur une colonne de silice (éluant : CH₂Cl₂/EtOH : 99/1 à 98/2). Après concentration des fractions les plus pures, on obtient un solide marron qui est recristallisé à l'aide d'acétate d'isopropyle. On obtient finalement un solide marron avec un rendement de 37%.

### 39.2) 4-[(10H-phénothiazin-2-yloxy)méthyl]aniline :

A une solution de 0,65 g (1,86 mmole) de l'intermédiaire 39.1 dans un mélange de 9,3 ml d'acide acétique glacial et 1,2 ml de HCl (12 N), on ajoute successivement 1,02 g (4,52 mmoles) de SnCl₂, 2H₂O et 0,29 g (4,52 mmoles) de Zn. L'ensemble est agité 18 heures à 20 °C. Le mélange réactionnel est ensuite rendu basique par addition d'une solution aqueuse de NaOH à 30%. Le produit est alors extrait à l'aide de 2 fois 50 ml de CH₂Cl₂. La solution organique est lavée par 50 ml de saumure, séchée sur MgSO₄, filtrée et concentrée sous vide. Le résidu est purifié sur une colonne de silice (éluant : Heptane/AcOEt : 1/1). On obtient un solide jaune pâle avec un rendement de 20%. Point de fusion : > 175 °C (décomposition).

### 39.3) N'-{4-[(10H-phénothiazin-2-yloxy)méthyl]phényl}-2-thiophènecarboximidamide :

Le protocole expérimental utilisé est le même que celui décrit pour l'intermédiaire 2.3, à partir de l'intermédiaire 39.2. Solide rose saumon. Point de fusion : 105-116 °C.

### Etude pharmacologique des produits de l'invention

### Etude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (Proc. Natl. Acad. Sci. USA, (1990) 87: 682-685). Des cervelets de rats Sprague-Dawley (300g - Charles River) sont rapidement prélevés, disséqués à 4 °C et homogénéisés dans un volume de tampon d'extraction (HEPES 50 mM, EDTA 1 mM, pH 7,4, pepstatine A 10 mg/ml, leupeptine 10 mg/ml). Les homogénats sont ensuite centrifugés à 21000 g pendant 15 min à 4 °C. Le dosage se fait dans des tubes à essai en verre dans lesquels sont distribués 100 µl de tampon d'incubation contenant 100 mM d'HEPES (pH 7,4), 2 mM d'EDTA, 2,5 mM de CaCl₂, 2 mM de dithiotréitol, 2 mM de NADPH réduit et 10 µg/ml de calmoduline. On ajoute 25 µl d'une solution contenant 100 nM de [³H]L-arginine (Activité spécifique : 56,4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide. Les composés des exemples 3 à 5, 7, 9 à 12, 15, 16, 18, 19, 21, 22, 26, 27, 30, 31 et 35 à 37 décrits ci-dessus présentent une CI₅₀ inférieure à 3,5 µM.

### Etude des effets sur la péroxidation lipidique du cortex cérébral de rat

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, Meth. Enzymol. (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g/ 15 ml et centrifugé à 515 g pendant 10 minutes à 4 °C. Le surnageant est utilisé immédiatement pour la détermination de la péroxidation lipidique. L'homogénat de cortex cérébral de rat (500 µl) est incubé à 37 °C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. European J. Pharmacol. (1995) 285, 203-206). Les composés des exemples 1 à 9, 12 à 19, 21 à 23, 30 et 35 à 37 décrits ci-dessus présentent une CI₅₀ inférieure à 30 µM.

## Revendications

1. Produit de formule générale **(I)** 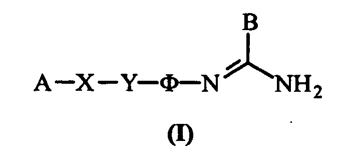 dans laquelle
Φ représente un radical phénylène pouvant compter, outre les deux chaînes déjà représentées dans la formule générale (**I**), jusqu'à deux substituants choisis parmi un atome d'hydrogène, un halogène, un groupe OH, et un radical alkyle ou alkoxy linéaire ou ramifié ayant de 1 à 6 atomes de carbone ;
A représente un radical 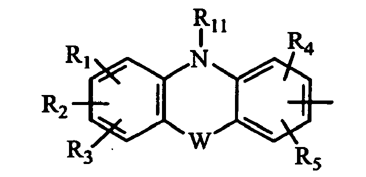 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 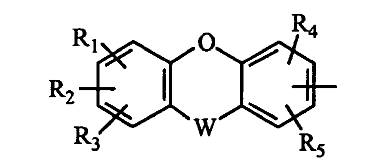 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone ;
B représente -CH₂-NO₂, un radical alkyle linéaire ou ramifié ayant de 1 à 6 atomes de carbone, aryle carbocyclique ou hétérocyclique à 5 ou 6 chaînons contenant de 1 à 4 hétéroatomes choisis parmi O S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone,
ou B représente un radical NR₁₃R₁₄, dans lequel R₁₃ et R₁₄ représentant, indépendamment, un atome d'hydrogène ou un radical alkyle linéaire ou ramifié ayant de 1 à 6 atomes de carbone ou un radical cyano ou nitro, ou R₁₃ et R₁₄ forment avec l'atome d'azote un hétérocycle non aromatique de cinq à six chaînons, les éléments de la chaîne étant choisis parmi un groupe composé de -CH₂-, -NH-, -O- ou -S- ;
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-,
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine, m et n étant des entiers compris de 0 à 6 ;
R₁₆, R₁₇ et R₁₈ représentent, indépendamment, un atome d'hydrogène ou un radical alkyle linéaire ou ramifié ayant de 1 à 6 atomes de carboné ;
ou sel dudit produit.

2. Produit selon la revendication 1, **caractérisé en ce que** :
A représente un radical 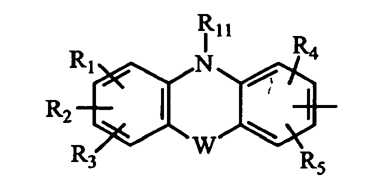 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone,
R₁₁ représentant un atome d'hydrogène ou un radical alkyle linéaire ou ramifié ayant de 1 à 6 atomes de carbone,
ou un radical 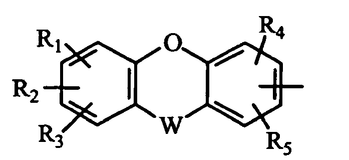 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone ;
B représente un radical aryle carbocyclique ou hétérocyclique à 5 ou 6 chaînons contenant de 1 à 4 hétéroatomes choisis parmi O, S, N et notamment les radicaux thiophène, furanne, pyrrole ou thiazole, le radical aryle étant éventuellement substitué par un ou plusieurs groupes choisis parmi les radicaux alkyle, alkényle ou alkoxy linéaires ou ramifiés ayant de 1 à 6 atomes de carbone ;
W n'existe pas, ou représente une liaison, S ou NR₁₅, dans lequel R₁₅ représente un atome d'hydrogène ou un radical alkyle linéaire ou ramifié ayant de 1 à 6 atomes de carbone ;
X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-,
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine,
m et n étant des entiers compris de 0 à 6.

3. Produit selon la revendication 1 ou 2, **caractérisé en ce que** :
A représente un radical 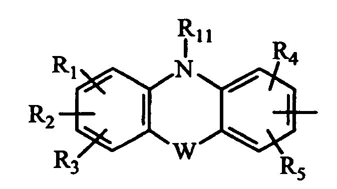 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone,
R₁₁ représentant un atome d'hydrogène ou un radical méthyle,
ou un radical 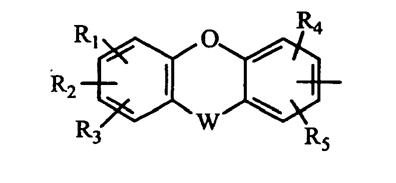 dans lequel R₁, R₂, R₃, R₄, R₅ représentent, indépendamment, un atome d'hydrogène, le groupe OH ou un radical alkyle ou alkoxy linéaire ou ramifié ayant de 1 à 6 atomes de carbone ;
B représente l'un des radicaux phényle, thiophène, furanne, pyrrole ou thiazole éventuellement substitués par un ou plusieurs groupes choisis parmi les radicaux alkyle, alkényle ou alkoxy linéaires ou ramifiés ayant de 1 à 6 atomes de carbone ;
W n'existe pas, ou représente une liaison, S ou NR₁₅, dans lequel R₁₅ représente un atome d'hydrogène ou un radical alkyle linéaire ou ramifié ayant de 1 à 6 atomes de carbone ;
X représente une liaison ou un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇₋
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine, m et n étant des entiers compris de 0 à 6.

4. Produit selon l'un des revendications 1 à 3, **caractérisé en ce que** :
A représente un radical 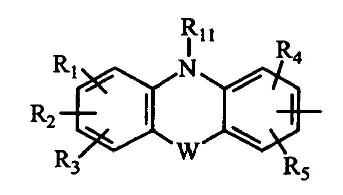 dans lequel R₁, R₂, R₃, R₄, R₅ représentent, indépendamment, un atome d'hydrogène ou un radical méthyle,
R₁₁ représentant un atome d'hydrogène ou un radical méthyle ;
B représente le radical thiophène ;
W n'existe pas, représente une simple liaison ou S ;
X n'existe pas ou représente un radical -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-, k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine,
m et n étant des entiers compris de 0 à 6 ;
R₁₆, R₁₇ et R₁₈ représentent un atome d'hydrogène.

5. Produit selon la revendication 1, **caractérisé en ce qu'**il s'agit d'un des composés suivants :
- 4-{[2-thiényl(imino)méthyl]amino}-N-[4-(phénylamino)phényl]-benzèneacétamide ;
- {4-{[2-thiényl(imino)méthyl]amino}phénoxy}-N-[4-(phénylamino)phényl]-acétamide ;
- 4-{[2-thiényl(imino)méthyl]amino}-N-[2-(phénylamino)phényl]-benzènebutanamide ;
- 4-{[2-thiényl(imino)méthyl]amino}-N-[4-(phénylamino)phényl]-benzènebutanamide ;
- 4-{[2-thiényl(imino)méthyl]amino}-N-[4-(4-méthoxyphénylamino)phényl]-benzènebutanamide ;
- [4-(phénylamino)phényl]-carbamate de 2-{4-{[2-thiényl(imino)méthyl]amino}phényl}-éthyle ;
- N- {2-{4-{[2-thiényl(imino)méthyl]amino}phényl)éthyl}-N'-[4-(phénylamino)phényl]-urée ;
- 4-{4-{[2-thiényl(imino)méthyl]amino}phényl}-N-[4-(phénylamino)phényl]-1-pipérazine acétamide ;
- 1-{[(4-phénylamino)phénylamino]carbonyl}-4-{4-{[2-thiényl(imino)méthyl] amino}phényl}-pipérazine ;
- 4-{[2-thiényl(imino)méthyl]amino}-N-[4-(phénylamino)phényl]-benzènebutanamine ;
- 3-{[2-thiényl(imino)méthyl]amino}-N-[4-(phénylamino)phényl]-benzènepropanamide ;
- 4-(4-{[amino(2-thiényl)méthylidène]amino}phényl)-N-[2-(4-toluidino)phényl]butanamide ;
- 4-anilinophényl-4-(4-{[amino(2-thiényl)méthylidène]amino}-phényl)butanoate ;
- 4-(4-([amino(2-thiényl)méthylidène]amino)phényl)-N-[2-(4-toluidino)phényl]butanamide ;
- N'-{4-[4-(3-anilinophénoxy)butyl]phényl}-2-thiophènecarboximidamide ;
- 4-(4-{[amino(2-thiényl)méthylidène]amino}phényl)-N-(9*H*-carbazol-3-yl)butanamide ;
- N'-[4-(10*H*-phénothiazin-2-yloxy)phényl]-2-thiophènecarboximidamide ;
- N'-{4-[(10-méthyl-10*H*-phénothiazin-2-yl)oxy]phényl}-2-thiophènecarboximidamide ;
- 4-(4-{[amino(2-thiényl)méthylidène]amino}phényl)-N-(10*H*-phénothiazin-3-yl)butanamide ;
- N'-(4-{4-[2-(10*H*-phénothiazin-2-yloxy)éthyl]-1-pipérazinyl}phényl)-2-thiophènecarboximidamide ;
- 4-(4-{[amino(2-thiényl)méthylidène]amino} phényl)-N-[4-(4-toluidino)phényl]butanamide ;
- 3-anilinophényl 4-(4-{[amino(2-thiényl)méthylidène]amino}-phényl)butanoate ;
- 2-(4- {[amino(2-thiényl)méthylidène]amino}phényl)-N-[2-(9H-carbazol-4-yloxy)éthyl]acétamide ;
- N-(4-{[amino(2-thiényl)méthylidène]amino}phenéthyl)-2-anilinobenzamide;
- N-(4- {[amino(2-thiényl)méthylidène]amino}phénéthyl)-2-(2,3-diméthylanilino)benzamide ;
- N'-{4-[4-(2-anilinobenzoyl)-1-pipérazinyl]phényl}-2-thiophènecarboximidamide;
- N'-(4-{4-[2-(2,3-diméthylanilino)benzoyl]-1-pipérazinyl}phényl)-2-thiophènecarboximidamide;
- 4-(4- {[amino(2-thiényl)méthylidène]amino}phényl)-N-(4-phénoxyphényl)butanamide ;
- N-(4-{[amino(2-thiényl)méthylidène]amino}phénéthyl)-4-(4-hydroxyphénoxy)benzamide ;
- N-{4-[4-(10*H*-phénothiazin-2-yloxy)butyl]phényl}-2-thiophènecarboximidamide;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidamide ;
- N-(4- {[amino(2-thiényl)méthylidène]amino}phénéthyl)-2-méthoxy-10*H-*phénothiazine-1-carboxamide ;
- N'-[4-(2-{[(2-méthoxy-10*H*-phénothiazin-1-yl)méthyl]amino}éthyl)phényl]-2-thiophènecarboximidamide ;
- N'-{4-[(10*H*-phénothiazin-2-yloxy)méthyl]phényl}-2-thiophènecarboximidamide ;
ou d'un de leurs sels.

6. Produit selon la revendication 5, **caractérisé en ce qu'**il s'agit d'un des composés suivants :
- {4-{[2-thiényl(imino)méthyl]amino}phénoxy}-N-[4-(phénylamino)phényl]-acétamide ;
- 4-{[2-thiényl(imino)méthyl]amino}-N-[2-(phénylamino)phényl]-benzènebutanamide ;
- 4-{[2-thiényl(imino)méthyl]amino}-N-[4-(phénylamino)phényl]-benzènebutanamide ;
- [4-(phénylamino)phényl]-carbamate de 2-{4-{[2-thiényl(imino)méthyl]amino}phényl}-éthyle ;
- 4-{4-{[2-thiényl(imino)méthyl]amino}phényl}-N-[4-(phénylamino)phényl]-1-pipérazine acétamide ;
- 3-{[2-thiényl(imino)méthyl]amino}-N-[4-(phénylamino)phényl]-benzènepropanamide ;
- 4-(4-{[amino(2-thiényl)méthylidène]amino}phényl)-N-[2-(4-toluidino)phényl]butanamide ;
- N'-{4-[4-(3-anilinophénoxy)butyl]phényl}-2-thiophènecarboximidamide ;
- 4-(4-{[amino(2-thiényl)méthylidène]amino}phényl)-N-(9*H*-carbazol-3-yl)butanamide ;
- N'-[4-(10*H*-phénothiazin-2-yloxy)phényl]-2-thiophènecarboximidamide ;
- 4-(4-{[amino(2-thiényl)méthylidène]amino}phényl)-N-(10*H*-phénothiazin-3-yl)butanamide ;
- N'-(4-{4-[2-(10*H*-phénothiazin-2-yloxy)éthyl]-1-pipérazinyl}phényl)-2-thiophènecarboximidamide ;
- 4-(4-{[amino(2-thiényl)méthylidène]amino}phényl)-N-(4-phénoxyphényl)butanamide ;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidamide ;
- N-(4-{[amino(2-thiényl)méthylidène]amino}phénéthyl)-2-méthoxy-10*H-*phénothiazine-1-carboxamide ;
ou d'un de leurs sels.

7. Produit selon la revendication 6, **caractérisé en ce qu'**il s'agit d'un des composés suivants :
- 4-{[2-thiényl(imino)méthyl]amino}-N-[2-(phénylamino)phényl]-benzènebutanamide ;
- 4-{[2-thiényl(imino)méthyl]amino}-N-[4-(phénylamino)phényl]-benzènebutanamide ;
- N'-[4-(10*H*-phénothiazin-2-yloxy)phényl]-2-thiophènecarboximidamide ;
- 4-(4- {[amino(2-thiényl)méthylidène] amino}phényl)-N-(10*H*-phénothiazin-3-yl)butanamide ;
- 3-[(3-{[amino(2-thiényl)méthylidène]amino}-benzyl)amino]-N-(4-anilinophényl)propanamide ;
- N'-(4-{2-[(10*H*-phénothiazin-3-ylméthyl)amino]éthyl}phényl)-2-thiophènecarboximidamide ;
ou d'un de leurs sels.

8. A titre de produits industriels nouveaux, les composés de formule générale (IS)
A-X-Y-Φ-T **(IS)**
dans laquelle
A représente un radical 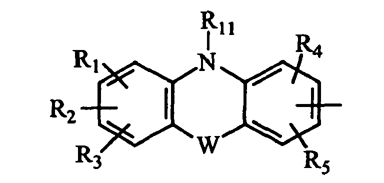 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 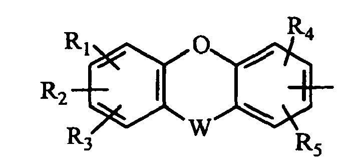 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
X représente une liaison ou un radical (CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇-,
k représentant 0 ou 1 ;
Y représente une liaison ou un radical choisi parmi les radicaux -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
Q représentant pipérazine, homopipérazine, 2-méthylpipérazine, 2,5-diméthylpipérazine, 4-oxypipéridine ou 4-aminopipéridine,
m et n étant des entiers de 0 à 6 ;
Φ représente un radical phénylène pouvant compter, outre les deux chaînes déjà représentées dans la formule générale **(I),** jusqu'à deux substituants choisis parmi un atome d'hydrogène, un halogène, un groupe OH, et un radical alkyle ou alkoxy linéaire
ou ramifié ayant de 1 à 6 atomes de carbone ;
T représente NO₂ ou NH₂;
R₁₆, R₁₇ et R₁₈ représentent, indépendamment, un atome d'hydrogène ou un radical alkyle linéaire ou ramifié ayant de 1 à 6 atomes de carbone.

9. A titre de produits industriels nouveaux, les composés de formule générale (IS') 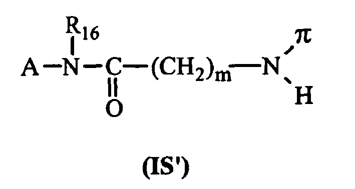 dans laquelle
A représente un radical 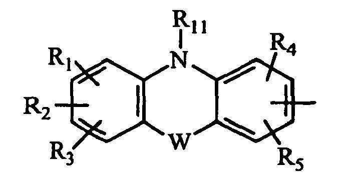 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
R₁₁ représentant un atome d'hydrogène, le groupe OH, un radical alkyle ou alkoxy linéaire ou ramifié ayant de 1 à 6 atomes de carbone, ou un radical -COR₁₂,
et R₁₂ représentant un atome d'hydrogène, le groupe OH, un radical alkyle linéaire ou ramifié ayant de 1 à 6 atomes de carbone,
ou un radical 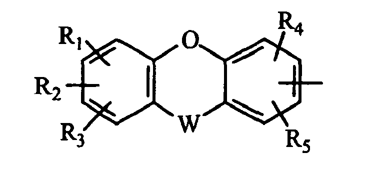 dans lequel R₁, R₂, R₃, R₄, R₅ représentent, indépendamment, un atome d'hydrogène, un halogène, le groupe OH, un radical alkyle ou alkoxy linéaire ou ramifié ayant de 1 à 6 atomes de carbone, ou un radical cyano, nitro ou NR₆R₇,
R₆ et R₇ représentant, indépendamment, un atome d'hydrogène, le groupe OH, un radical alkyle ou alkoxy linéaire ou ramifié ayant de 1 à 6 atomes de carbone, ou encore un groupe -COR₈,
R₈ représentant un atome d'hydrogène, le groupe OH, un radical alkyle ou alkoxy linéaire ou ramifié ayant de 1 à 6 atomes de carbone, ou NR₉R₁₀,
R₉ et R₁₀ représentant, indépendamment, un atome d'hydrogène, le groupe OH ou un radical alkyle linéaire ou ramifié ayant de 1 à 6 atomes de carbone,
W n'existe pas, ou représente une liaison, ou O, S ou NR₁₅, dans lequel R₁₅ représente un atome d'hydrogène ou un radical alkyle linéaire ou ramifié ayant de 1 à 6 atomes de carbone ;
π représente un atome d'hydrogène ou un groupe protecteur de type carbamate ;
R₁₆, R₁₇ et R₁₈ représentent, indépendamment, un atome d'hydrogène ou un radical alkyle linéaire ou ramifié ayant de 1 à 6 atomes de carbone ;
et m représente un entier de 0 à 6.

10. A titre de médicament, un produit de formule générale (**I**) selon l'une des revendications 1 à 7, ou un sel pharmaceutiquement acceptable dudit produit.

11. Composition pharmaceutique contenant à titre de principe actif au moins un produit selon l'une des revendications 1 à 7, ou un sel pharmaceutiquement acceptable dudit produit.

12. Utilisation d'un produit de formule générale **(I)** selon l'une quelconque des revendications 1 à 7, ou d'un sel pharmaceutiquement acceptable dudit produit, pour fabriquer un médicament destiné à inhiber la NO synthase.

13. Utilisation d'un produit de formule générale **(I)** selon l'une quelconque des revendications 1 à 7, ou d'un sel pharmaceutiquement acceptable dudit produit, pour fabriquer un médicament destiné à inhiber la péroxidation lipidique.

14. Utilisation d'un produit de formule générale **(I)** selon l'une quelconque des revendications 1 à 7, ou d'un sel pharmaceutiquement acceptable dudit produit, pour fabriquer un médicament ayant à la fois une activité d'inhibition de la NO synthase et d'inhibition de la péroxidation lipidique.

## Claims

1. Product of general formula (I) 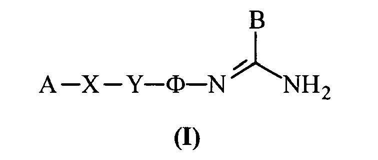 in which:
Φ represents a phenylene radical which can comprise, in addition to the two chains already represented in general formula (I), up to two substituents chosen from a hydrogen atom, a halogen, an OH group, and a linear or branched alkyl or alkoxy radical having 1 to 6 carbon atoms;
A represents a 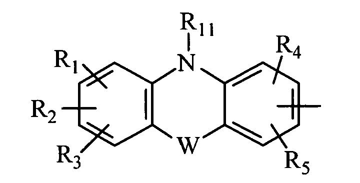 radical in which R₁, R₂, R₃, R₄, R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ and R₁₀ representing, independently, a hydrogen atom, the OH group or a linear or branched alkyl radical having 1 to 6 carbon atoms,
R₁₁ representing a hydrogen atom, the OH group, a linear or branched alkyl or alkoxy radical having 1 to 6 carbon atoms, or a -COR₁₂ radical,
and R₁₂ representing a hydrogen atom, the OH group, a linear or branched alkyl radical having 1 to 6 carbon atoms,
or a 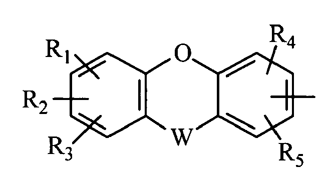 radical in which R₁, R₂, R₃, R₄, R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ and R₁₀ representing, independently, a hydrogen atom, the OH group or a linear or branched alkyl radical having 1 to 6 carbon atoms,
B represents -CH₂-NO₂, a linear or branched alkyl radical having 1 to 6 carbon atoms, carbocyclic or heterocyclic aryl with 5 or 6 members containing 1 to 4 heteroatoms chosen from O, S, N and in particular the thiophene, furan, pyrrole or thiazole radical, the aryl radical being optionally substituted by one or more groups chosen from linear or branched alkyl, alkenyl or alkoxy radicals having 1 to 6 carbon atoms,
or B represents an NR₁₃R₁₄ radical, in which R₁₃ and R₁₄ representing, independently, a hydrogen atom or a linear or branched alkyl radical having 1 to 6 carbon atoms or a cyano or nitro radical, or R₁₃ and R₁₄ form with the nitrogen atom a non aromatic heterocycle with five to six members, the elements of the chain being chosen from a group composed of -CH₂-, -NH-, -O- or -S-;
W does not exist, or represents a bond, or O, S or NR₁₅, in which R₁₅ represents a hydrogen atom or a linear or branched alkyl radical having 1 to 6 carbon atoms;
X represents a bond or a (CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- radical,
k representing 0 or 1;
Y represents a bond or a radical chosen from the -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ- radicals,
Q representing piperazine, homopiperazine, 2-methylpiperazine, 2,5-dimethylpiperazine, 4-oxypiperidine or 4-aminopiperidine radicals, m and n being integers from 0 to 6;
R₁₆, R₁₇ and R₁₈ represent, independently, a hydrogen atom or a linear or branched alkyl radical having 1 to 6 carbon atoms;
or a salt of said product.

2. Product according to claim 1, **characterized in that**:
A represents a 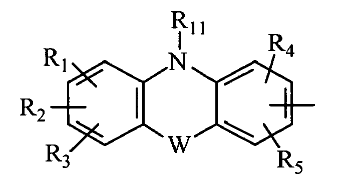 radical in which R₁, R₂, R₃, R₄, R₅ represent, independently, a hydrogen atom, the OH group or a linear or branched alkyl or alkoxy radical having 1 to 6 carbon atoms,
R₁₁ representing a hydrogen atom or a linear or branched alkyl radical having 1 to 6 carbon atoms,
or a 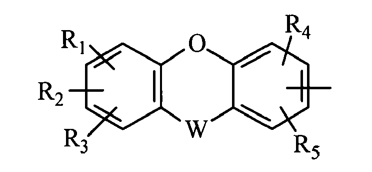 radical in which R₁, R₂, R₃, R₄, R₅ represent, independently, a hydrogen atom, the OH group or a linear or branched alkyl or alkoxy radical having 1 to 6 carbon atoms;
B represents a carbocyclic or heterocyclic aryl radical with 5 or 6 members containing 1 to 4 heteroatoms chosen from O, S, N and in particular the thiophene, furan, pyrrole or thiazole radicals, the aryl radical being optionally substituted by one or more groups chosen from linear or branched alkyl, alkenyl or alkoxy radicals having 1 to 6 carbon atoms;
W does not exist, or represents a bond, S or NR₁₅, in which R₁₅ represents a hydrogen atom or a linear or branched alkyl radical having 1 to 6 carbon atoms;
X represents a bond or an -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- radical
k representing 0 or 1;
Y represents a bond or a radical chosen from the -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ- radicals,
Q representing piperazine, homopiperazine, 2-methylpiperazine, 2,5-dimethylpiperazine, 4-oxypiperidine or 4-aminopiperidine,
m and n being integers from 0 to 6.

3. Product according to claim 1 or 2, **characterized in that**:
A represents a 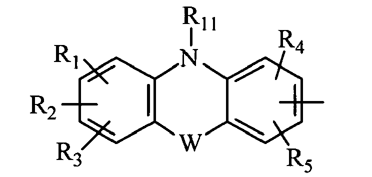 radical in which R₁, R₂, R₃, R₄ and R₅ represent, independently, a hydrogen atom, the OH group or a linear or branched alkyl or alkoxy radical having 1 to 6 carbon atoms,
R₁₁ representing a hydrogen atom or a methyl radical,
or a 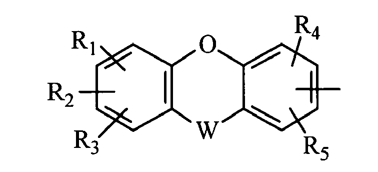 radical in which R₁, R₂, R₃, R₄, R₅ represent, independently, a hydrogen atom, the OH group or a linear or branched alkyl or alkoxy radical having 1 to 6 carbon atoms;
B represents one of the phenyl, thiophene, furan, pyrrole or thiazole radicals optionally substituted by one or more groups chosen from linear or branched alkyl, alkenyl or alkoxy radicals having 1 to 6 carbon atoms;
W does not exist, or represents a bond, S or NR₁₅, in which R₁₅ represents a hydrogen atom or a linear or branched alkyl radical having 1 to 6 carbon atoms;
X represents a bond or an -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- radical, k representing 0 or 1;
Y represents a bond or a radical chosen from the -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ- radicals,
Q representing piperazine, homopiperazine, 2-methylpiperazine, 2,5-dimethylpiperazine, 4-oxypiperidine or 4-aminopiperidine,
m and n being integers comprised from 0 to 6.

4. Product according to one of claims 1 to 3, **characterized in that**:
A represents a 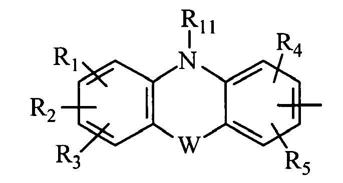 radical in which R₁, R₂, R₃, R₄, R₅ represent, independently, a hydrogen atom or a methyl radical,
R₁₁ representing a hydrogen atom or a methyl radical;
B represents the thiophene radical;
W does not exist, represents a single bond or S;
X does not exist or represents a -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- radical;
k representing 0 or 1;
Y represents a bond or a radical chosen from the -(CH₂)ₘ-, -(CH₂)ₘ-O-(CH₂)ₙ-, -(CH₂)m-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ- radicals,
Q representing piperazine,
m and n being integers comprised from 0 and 6;
R₁₆, R₁₇ and R₁₈ represent a hydrogen atom.

5. Product according to claim 1, **characterized in that** it is one of the following compounds:
- 4- {[2-thienyl(imino)methyl]amino}-N-[4-(phenylamino)phenyl]-benzeneacetamide;
- {4-{[2-thienyl(imino)methyl]amino}phenoxy}-N-[4-(phenylamino)phenyl]-acetamide;
- 4-{[2-thienyl(imino)methyl]amino}-N-[2-(phenylamino)phenyl]-benzenebutanamide;
- 4-{[2-thienyl(imino)methyl]amino}-N-[4-(phenylamino)phenyl]-benzenebutanamide;
- 4-{[2-thienyl(imino)methyl]amino}-N-[4-(4-methoxyphenylamino)phenyl]-benzenebutanamide;
- 2-{4-{[2-thienyl(imino)methyl]amino}phenyl}-ethyl [4-(phenylamino)phenyl]-carbamate;
- N-{2-{4-{[2-thienyl(imino)methyl]amino}phenyl}ethyl}-N'-[4-(phenylamino)phenyl]-urea;
- 4-{4-{[2-thienyl(imino)methyl]amino}phenyl}-N-[4-(phenylamino)phenyl]-1-piperazine-acetamide;
- 1-{[(4-phenylamino)phenylamino]carbonyl}-4-{4-{[2-thienyl(imino)methyl] amino}phenyl}-piperazine;
- 4-{[2-thienyl(imino)methyl]amino}-N-[4-(phenylamino)phenyl]-benzenebutanamine;
- 3-{[2-thienyl(imino)methyl]amino}-N-[4-(phenylamino)phenyl]-benzenepropanamide;
- 4-(4-{[amino(2-thienyl)methylidene]amino}phenyl)-N-[2-(4-toluidino)phenyl]butanamide;
- 4-anilinophenyl-4-(4- {[amino(2-thienyl)methylidene]amino}-phenyl)butanoate;
- 4-(4-{[amino(2-thienyl)methylidene]amino}phenyl)-N-[2-(4-toluidino)phenyl]butanamide;
- N'- {4-[4-(3 -anilinophenoxy)butyl]phenyl}-2-thiophenecarboximidamide;
- 4-(4-{[amino(2-thienyl)methylidene]amino}phenyl)-N-(9*H*-carbazol-3-yl)butanamide;
- N'-[4-(10*H*-phenothiazin-2-yloxy)phenyl]-2-thiophenecarboximidamide;
- N'-{4-[(10-methyl-10*H*-phenothiazin-2-yl)oxy]phenyl}-2-thiophenecarboximidamide;
- 4-(4-{[amino(2-thienyl)methylidene]amino}phenyl)-N-(10*H*-phenothiazin-3-yl)butanamide;
- N'-(4-{4-[2-(10*H*-phenothiazin-2-yloxy)ethyl]-1-piperazinyl)phenyl)-2-thiophenecarboximidamide;
- 4-(4-{[amino(2-thienyl)methylidene]amino} phenyl)-N-[4-(4-toluidino)phenyl]butanamide;
- 3-anilinophenyl 4-(4- {[amino(2-thienyl)methylidene]amino}-phenyl)butanoate;
- 2-(4-{[amino(2-thienyl)methylidene]amino}phenyl)-N-[2-(9*H*-carbazol-4-yloxy)ethyl]acetamide;
- N-(4-{[amino(2-thienyl)methylidene]amino}phenethyl)-2-anilinobenzamide;
- N-(4-{[amino(2-thienyl)methylidene]amino}phenethyl)-2-(2,3-dimethylanilino)benzamide;
- N'-{4-[4-(2-anilinobenzoyl)-1-piperazinyl]phenyl}-2-thiophenecarboximidamide;
- N'-(4-{4-[2-(2,3-dimethylanilino)benzoyl]-1-piperazinyl}phenyl)-2-thiophenecarboximidamide;
- 4-(4- {[amino(2-thienyl)methylidene]amino}phenyl)-N-(4-phenoxyphenyl)butanamide;
- N-(4-{[amino(2-thienyl)methylidene]amino}phenethyl)-4-(4-hydroxyphenoxy)benzamide;
- N-{4-[4-(10*H*-phenothiazin-2-yloxy)butyl]phenyl}-2-thiophenecarboximidamide;
- 3-[(3-{[amino(2-thienyl)methylidene]amino}-benzyl)amino]-N-(4-anilinophenyl)propanamide;
- N'-(4-{2-[(10*H*-phenothiazin-3-ylmethyl)amino]ethyl}phenyl)-2-thiophenecarboximidamide;
- N-(4-{[amino(2-thienyl)methylidene]amino}phenethyl)-2-methoxy-10*H-*phenothiazine-1-carboxamide;
- N'-[4-(2-{[(2-methoxy-10*H*-phenothiazin-1-yl)methyl]amino}ethyl)phenyl]-2-thiophenecarboximidamide;
- N'-{4-[(10*H*-phenothiazin-2-yloxy)methyl]phenyl}-2-thiophenecarboximidamide; or one of their salts.

6. Product according to claim 5, **characterized in that** it is one of the following compounds:
- {4-{[2-thienyl(imino)methyl]amino}phenoxy}-N-[4-(phenylamino)phenyl]-acetamide;
- 4-{[2-thienyl(imino)methyl]amino}-N-[2-(phenylamino)phenyl]-benzenebutanamide;
- 4-{[2-thienyl(imino)methyl]amino}-N-[4-(phenylamino)phenyl]-benzenebutanamide;
- 2-{4-{[2-thienyl(imino)methyl]amino}phenyl}-ethyl [4-(phenylamino)phenyl]-carbamate ;
- 4-{4-{[2-thienyl(imino)methyl]amino}phenyl}-N-[4-(phenylamino)phenyl]-1-piperazine-acetamide;
- 3- {[2-thienyl(imino)methyl]amino}-N-[4-(phenylamino)phenyl]-benzenepropanamide;
- 4-(4-{[amino(2-thienyl)methylidene]amino}phenyl)-N-[2-(4-toluidino)phenyl]butanamide;
- N'-{4-[4-(3-anilinophenoxy)butyl]phenyl}-2-thiophenecarboximidamide ;
- 4-(4-{[amino(2-thienyl)methylidene]amino}phenyl)-N-(9*H*-carbazol-3-yl)butanamide;
- N'-[4-(10*H*-phenothiazin-2-yloxy)phenyl]-2-thiophenecarboximidamide;
-4-(4-{[amino(2-thienyl)methylidene]amino}phenyl)-N-(10*H*-phenothiazin-3-yl)butanamide;
- N'-(4-{4-[2-(10*H*-phenothiazin-2-yloxy)ethyl]-1-piperazinyl}phenyl)-2-thiophenecarboximidamide;
- 4-(4-{[amino(2-thienyl)methylidene]amino}phenyl)-N-(4-phenoxyphenyl)butanamide;
- 3-[(3-{[amino(2-thienyl)methylidene]amino}-benzyl)amino]-N-(4-anilinophenyl)propanamide;
- N'-(4-{2-[(10*H*-phenothiazin-3-ylmethyl)amino]ethyl}phenyl)-2-thiophenecarboximidamide;
- N-(4-{([amino(2-thienyl)methylidene]amino}phenethyl)-2-methoxy-10*H-*phenothiazine-1-carboxamide;
or one of their salts.

7. Product according to claim 6, **characterized in that** it is one of the following compounds:
- 4-{[2-thienyl(imino)methyl]amino}-N-[2-(phenylamino)phenyl]-benzenebutanamide;
- 4- {[2-thienyl(imino)methyl]amino}-N-[4-(phenylamino)phenyl]-benzenebutanamide;
- N'-[4-(10*H*-phenothiazin-2-yloxy)phenyl]-2-thiophenecarboximidamide;
- 4-(4- {[amino(2-thienyl)methylidene] amino}phenyl)-N-(10*H*-phenothiazin-3-yl)butanamide;
- 3-[(3-{[amino(2-thienyl)methylidene]amino}-benzyl)amino]-N-(4-anilinophenyl)propanamide;
- N'-(4-{2-[(10*H*-phenothiazin-3-ylmethyl)amino]ethyl}phenyl)-2-thiophenecarboximidamide;
or one of their salts.

8. As new industrial products, the compounds of general formula (IS)
A-X-Y-Φ-T **(IS)**
in which
A represents a 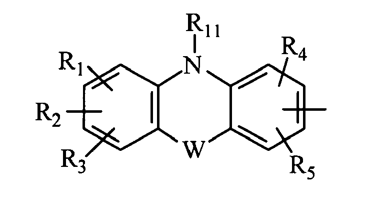 radical in which R₁, R₂, R₃, R₄, R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ and R₁₀ representing, independently, a hydrogen atom, the OH group or a linear or branched alkyl radical having 1 to 6 carbon atoms,
R₁₁ representing a hydrogen atom, the OH group, a linear or branched alkyl or alkoxy radical having 1 to 6 carbon atoms, or a -COR₁₂ radical,
and R₁₂ representing a hydrogen atom, the OH group, a linear or branched alkyl radical having 1 to 6 carbon atoms,
or a 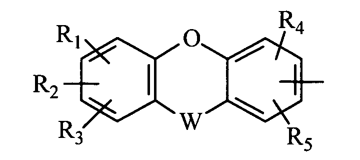 radical in which R₁, R₂, R₃, R₄, R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ and R₁₀ representing, independently, a hydrogen atom, the OH group or a linear or branched alkyl radical having 1 to 6 carbon atoms;
W does not exist, or represents a bond, or O, S or NR₁₅, in which R₁₅ represents a hydrogen atom or a linear or branched alkyl radical having 1 to 6 carbon atoms;
X represents a bond or a -(CH₂)ₖ-NR₁₆-, -O-, -S-, -CO-, -NR₁₆-CO-, -CO-NR₁₆-, -O-CO-, -CO-O-, -NR₁₆-CO-O- or -NR₁₆-CO-NR₁₇- radical, k representing 0 or 1;
Y represents a bond or a radical chosen from the -(CH₂)ₘ-, -(CH₂)ₘ-O-(CH₂)ₙ-, - (CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ- and -(CH₂)ₘ-Q-(CH₂)n- radicals,
Q representing piperazine, homopiperazine, 2-methylpiperazine, 2,5-dimethylpiperazine, 4-oxypiperidine or 4-aminopiperidine,
m and n being integers from 0 to 6;
Φ represents a phenylene radical which may comprise, in addition to the two chains already represented in general formula **(I),** up to two substituents chosen from a hydrogen atom, a halogen, an OH group, and a linear or branched alkyl or alkoxy radical having 1 to 6 carbon atoms;
T represents NO₂ or NH₂;
R₁₆, R₁₇ and R₁₈ represent, independently, a hydrogen atom or a linear or branched alkyl radical having 1 to 6 carbon atoms.

9. As new industrial products, the compounds of general formula (**IS'**) 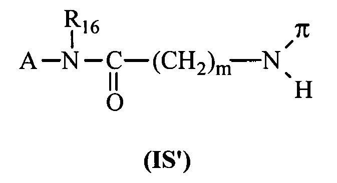 in which
A represents a 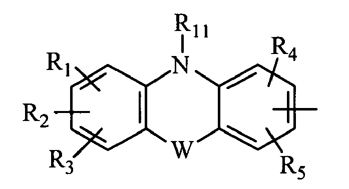 radical in which R₁, R₂, R₃, R₄ and R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ and R₁₀ representing, independently, a hydrogen atom, the OH group, a linear or branched alkyl radical having 1 to 6 carbon atoms,
R₁₁ representing a hydrogen atom, the OH group, a linear or branched alkyl or alkoxy radical having 1 to 6 carbon atoms, or a -COR₁₂ radical,
and R₁₂ representing a hydrogen atom, the OH group, a linear or branched alkyl radical having 1 to 6 carbon atoms,
or a 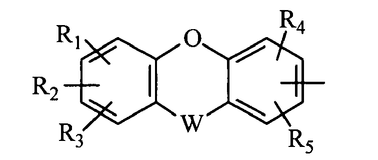 radical in which R₁, R₂, R₃, R₄, R₅ represent, independently, a hydrogen atom, a halogen, the OH group, a linear or branched alkyl or alkoxy radical having 1 to 6 carbon atoms, or a cyano, nitro or NR₆R₇ radical,
R₆ and R₇ representing, independently, a hydrogen atom, the OH group, a linear or branched alkyl or alkoxy radical having 1 to 6 carbon atoms, or also a -COR₈ group,
R₈ representing a hydrogen atom, the OH group, a linear or branched alkyl or alkoxy radical having 1 to 6 carbon atoms, or NR₉R₁₀,
R₉ et R₁₀ representing, independently, a hydrogen atom, the OH group, a linear or branched alkyl radical having 1 to 6 carbon atoms;
W does not exist, or represents a bond, or O, S or NR₁₅, in which R₁₅ represents a hydrogen atom or a linear or branched alkyl radical having 1 to 6 carbon atoms;
π represents a hydrogen atom or a protective group of the carbamate type;
R₁₆, R₁₇ and R₁₈ represent, independently, a hydrogen atom or a linear or branched alkyl radical having 1 to 6 carbon atoms; and m represents an integer from 0 to 6.

10. As a medicament, a product of general formula (I) according to one of claims 1 to 7, or a pharmaceutically acceptable salt of said product.

11. Pharmaceutical composition containing as active ingredient at least one product according to one of claims 1 to 7, or a pharmaceutically acceptable salt of said product.

12. Use of a product of general formula **(I)** according to any one of claims 1 to 7, or a pharmaceutically acceptable salt of said product, for the production of a medicament intended to inhibit NO synthase.

13. Use of a product of general formula **(I)** according to any one of claims 1 to 7, or a pharmaceutically acceptable salt of said product, for the production of a medicament intended to inhibit lipidic peroxidation.

14. Use of a product of general formula **(I)** according to any one of claims 1 to 7, or a pharmaceutically acceptable salt of said product, for the production of a medicament having both an NO synthase inhibition activity and a lipidic peroxidation inhibition activity.

## Patentansprüche

1. Produkt der allgemeinen Formel (I) 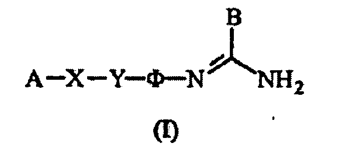 in der
Φ einen Phenylenrest darstellt, der neben den beiden bereits in der allgemeinen Formel (I) dargestellten Ketten bis zu zwei Substituenten aufweisen kann, die aus einem Wasserstoffatom, einem Halogen, einer OH-Gruppe und einem linearen oder verzweigten Alkyl- oder Alkoxyrest mit 1 bis 6 Kohlenstoffatomen ausgewählt sind:
A einen Rest darstellt 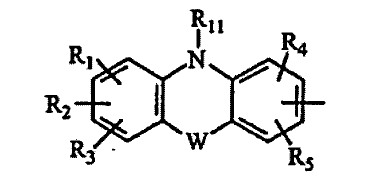 in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen oder einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
R₁₁ ein Wasserstoffatom, die OH-Gruppe, einen linearen oder verzweigten Alkyl- oder Alkoxyrest mit 1 bis 6 Kohlenstoffatomen oder einen Rest -COR₁₂ darstellt,
und R₁₂ ein Wasserstoffatom, die OH-Gruppe, einen linearen oder verzweigten Alkylrest mit 1 bis 6 Kohlenstoffatomen darstellt,
oder einen Rest 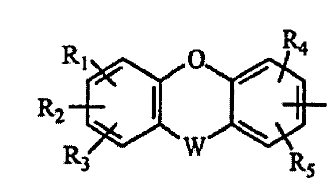 in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen oder einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
B -CH₂-NO₂, einen linearen oder verzweigten Alkylrest mit 1 bis 6 Kohlenstoffatomen, carbocyclischen oder heterocyclischen Arylrest mit 5 oder 6 Gliedern, enthaltend 1 bis 4 Heteroatome, die aus O, S, N ausgewählt sind, und insbesondere die Reste Thiophen, Furan, Pyrrol oder Thiazol darstellt, wobei der Arylrest gegebenenfalls mit einer oder mehreren Gruppen substituiert ist, ausgewählt aus den Resten lineares oder verzweigtes Alkyl, Alkenyl oder Alkoxy mit 1 bis 6 Kohlenstoffatomen,
oder B einen Rest NR₁₃R₁₄ darstellt, in dem R₁₃ und R₁₄ unabhängig voneinander ein Wasserstoffatom oder einen linearen oder verzweigten Alkylrest mit 1 bis 6 Kohlenstoffatomen oder einen Rest Cyano oder Nitro darstellen, oder R₁₃ und R₁₄ mit dem Stickstoffatom einen nicht-aromatischen Heterocyclus von 5 bis 6 Gliedern bilden, wobei die Elemente der Kette aus einer Gruppe ausgewählt sind, die aus -CH₂-, -NH-, -O- oder -S- besteht;
W nicht existiert oder eine Bindung oder O, S oder NR₁₅ darstellt, worin R₁₅ ein Wasserstoffatom oder einen linearen oder verzweigten Alkylrest mit 1 bis 6 Kohlenstoffatomen darstellt;
X eine Bindung oder einen Rest -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- darstellt,
wobei k 0 oder 1 darstellt;
Y eine Bindung oder einen Rest darstellt, der ausgewählt ist aus den Resten -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
wobei Q Piperazin, Homopiperazin, 2-Methylpiperazin, 2,5-Dimethylpiperazin, 4-Oxypiperidin oder 4-Aminopiperidin darstellt,
wobei m und n ganze Zahlen von 0 bis 6 sind;
R₁₆, R₁₇ und R₁₈ unabhängig voneinander ein Wasserstoffatom oder einen linearen oder verzweigten Alkylrest mit 1 bis 6 Kohlenstoffatomen darstellen;
oder ein Salz dieses Produkts.

2. Produkt nach Anspruch 1, **dadurch gekennzeichnet, dass**
A einen Rest 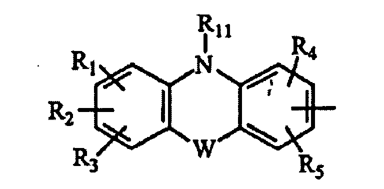 darstellt, in dem R₁, R₂, R₃, R₄, R₅ unabhängig voneinander ein Wasserstoffatom, die OH-Gruppe oder einen linearen oder verzweigten Alkyl- oder Alkoxyrest mit 1 bis 6 Kohlenstoffatomen darstellen,
wobei R₁₁ ein Wasserstoffatom oder einen linearen oder verzweigten Alkylrest mit 1 bis 6 Kohlenstoffatomen darstellt,
oder einen Rest 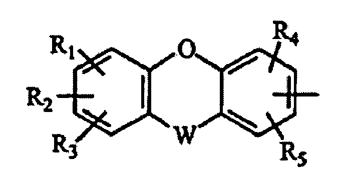 in dem R₁, R₂, R₃, R₄, R₅ unabhängig voneinander ein Wasserstoffatom, die OH-Gruppe oder einen linearen oder verzweigten Alkyl- oder Alkoxyrest mit 1 bis 6 Kohlenstoffatomen darstellen;
B einen carbocyclischen oder heterocyclischen Arylrest mit 5 oder 6 Gliedern darstellt, enthaltend 1 bis 4 Heteratome, die aus O, S, N ausgewählt sind, und insbesondere die Reste Thiophen, Furan, Pyrrol oder Thiazol, wobei der Arylrest gegebenenfalls mit einer oder mehreren Gruppen substituiert ist, die aus den Resten lineares oder verzweigtes Alkyl, Alkenyl oder Alkoxy mit 1 bis 6 Kohlenstoffatomen ausgewählt sind;
W nicht existiert oder eine Bindung, S oder NR₁₅ darstellt, worin R₁₅ ein Wasserstoffatom oder einen linearen oder verzweigten Alkylrest mit 1 bis 6 Kohlenstoffatomen darstellt;
X eine Bindung oder einen Rest -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- darstellt,
wobei k 0 oder 1 darstellt;
Y eine Bindung oder einen Rest darstellt, der aus den Resten -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈(CH₂)ₙ-, -(CH₂)ₘ-Q-(CH₂)ₙ-ausgewählt ist,
wobei Q Piperazin, Homopiperazin, 2-Methylpiperazin, 2,5-Dimethylpiperazin, 4-Oxypiperidin oder 4-Aminopiperidin darstellt,
wobei m und n ganze Zahlen von 0 bis 6 sind.

3. Produkt nach Anspruch 1 oder 2, **dadurch gekennzeichnet, dass**
A einen Rest 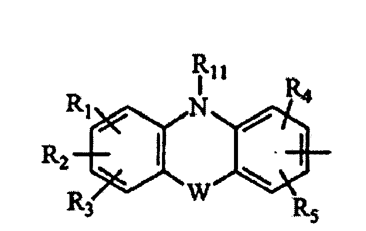 darstellt, in dem R₁, R₂, R₃, R₄, R₅ unabhängig voneinander ein Wasserstoffatom, die OH-Gruppe oder einen linearen oder verzweigten Alkyl- oder Alkoxyrest mit 1 bis 6 Kohlenstoffatomen darstellen,
wobei R₁₁ ein Wasserstoffatom oder einen Methylrest darstellt,
oder einen Rest 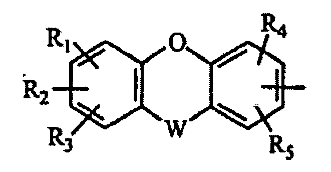 in dem R₁, R₂, R₃, R₄, R₅ unabhängig voneinander ein Wasserstoffatom, die OH-Gruppe oder einen linearen oder verzweigten Alkyl- oder Alkoxyrest mit 1 bis 6 Kohlenstoffatomen darstellen;
B einen der Reste Phenyl, Thiophen, Furan, Pyrrol oder Thiazol darstellt, die gegebenenfalls mit einer oder mehreren Gruppen substituiert sind, die aus den Resten lineares oder verzweigtes Alkyl, Alkenyl oder Alkoxy mit 1 bis 6 Kohlenstoffatomen ausgewählt sind;
W nicht existiert oder eine Bindung, S oder NR₁₅ darstellt, worin R₁₅ ein Wasserstoffatom oder einen linearen oder verzweigten Alkylrest mit 1 bis 6 Kohlenstoffatomen darstellt;
X eine Bindung oder einen Rest -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- darstellt,
wobei k 0 oder 1 darstellt;
Y eine Bindung oder einen Rest darstellt, der aus den Resten -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-ausgewählt ist,
wobei Q Piperazin, Homopiperazin, 2-Methylpiperazin, 2,5-Dimethylpiperazin, 4-Oxypiperidin oder 4-Aminopiperidin darstellt,
wobei m und n ganze Zahlen von 0 bis 6 sind.

4. Produkt nach einem der Ansprüche 1 bis 3, **dadurch gekennzeichnet, dass**:
A einen Rest 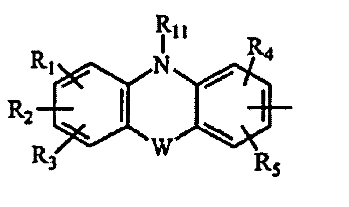 darstellt, in dem R₁, R₂, R₃, R₄, R₅ unabhängig voneinander ein Wasserstoffatom oder einen Methylrest darstellen,
wobei R₁₁ ein Wasserstoffatom oder einen Methylrest darstellt;
B den Thiophenrest darstellt;
W nicht existiert, eine einfache Bindung oder S darstellt;
X nicht existiert oder einen Rest -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- darstellt,
wobei k 0 oder 1 darstellt;
Y eine Bindung oder einen Rest darstellt, der aus den Resten -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-ausgewählt ist,
wobei Q Piperazin darstellt,
wobei m und n ganze Zahlen von 0 bis 6 sind;
R₁, R₁₇ und R₁₈ ein Wasserstoffatom darstellen.

5. Produkt nach Anspruch 1, **dadurch gekennzeichnet, dass** es sich um eine der folgenden Verbindungen handelt:
- 4-{[2-Thienyl(imino)methyl]amino}-N-[4-(phenylamino)phenyl]-benzenacetamid;
- {4-{[2-Thienyl(imino)methyl]amino}phenoxy}-N-[4-(phenylamino)phenyl]-acetamid;
- 4-{[2-Thienyl(imino)methyl]amino}-N-[2-(phenylamino)phenyl]-benzenbutanamid;
- {4-{[2-Thienyl(imino)methyl]amino}-N-[4-(phenylamino)phenyl]-benzenbutanamid;
- {4-{[2-Thienyl(imino)methyl]amino}-N-[4-(4-methoxyphenylamino)phenyl]-benzenbutanamid;
- 2-{4-{[2-Thienyl(imino)methyl]amino}phenyl}-ethyl-[4-(phenylamino)phenyl]-carbamat;
- N-{2-{4-{[2-Thienyl(imino)methyl]amino}phenyl}ethyl}-N'-[4-(phenylamino)phenyl]-harnstoff;
- 4-{4-{[2-Thienyl(imino)methyl]amino}phenyl}-N-[4-(phenylamino)phenyl]-1-piperazinacetamid;
- 1-{[(4-Phenylamino)phenylamino]carbonyl}-4-{4-{[2-thienyl(imino)methyl]amino}phenyl-piperazin;
- 4-{[2-Thienyl(imino(methyl]amino}-N-[4-(phenylamino)phenyl]-benzenbutanamin;
- 3-{[2-Thienyl(imino)methyl]amino}-N-[4-(phenylamino)phenyl]-benzenpropanamid;
- 4-(4-{[Amino(2-thienyl)methyliden]amino}phenyl)-N-[2-(4-toluidino)phenyl]butanamid;
- 4-Anilinophenyl-4-(4-{[amino(2-thienyl)methyliden]amino}-phenyl)butanoat;
- 4-(4-{[Amino(2-thienyl)methyliden]amino}phenyl)-N-[2-(4-toluidino)phenyl]butanamid;
- N'-{4-[4-(3-Anilinophenoxy)butyl]phenyl}-2-thiophencarboximidamid;
- 4-(4-{[Amino(2-thienyl)methyliden]amino}phenyl)-N-(9*H*-carbazol-3-yl)butanamid;
- N'-[4-(10*H*-Phenothiazin-2-yloxy)phenyl]-2-thiophencarboximidamid;
- N'-{4-[(10-Methyl-10*H*-phenothiazin-2-yl)oxy]phenyl}-2-thiophencarboximidamid;
- 4-(4-{[Amino(2-thienyl)methyliden]amino}phenyl)-N-(10*H*-phenothiazin-3-yl)butanamid;
- N'-(4-{4-[2-(10*H*-phenothiazin-2-yloxy)ethyl]-1-piperazinyl}phenyl)-2-thiophencarboximidamid;
- 4-(4-{[Amino(2-thienyl)methyliden]amino}phenyl)-N-[4-(4-toluidino)phenyl]butanamid;
- 3-Anilinophenyl-4-(4-{[amino(2-thienyl)methyliden]amino}-phenyl)butanoat;
- 2-(4-{[Amino(2-thienyl)methyliden]amino}phenyl)-N-[2-(9H-carbazol-4-yloxy)ethyl]acetamid;
- N-(4-{[Amino(2-thienyl)methyliden]amino}phenethyl)-2-anilinobenzamid;
- N-(4-{[Amino(2-thienyl)methyliden]amino}phenethyl)-2-(2,3-dimethylanilino)benzamid;
- N'-{4-[4-(2-Anilinobenzoyl)-1-piperazinyl]phenyl}-2-thiophencarboximidamid;
- N'-(4-{4-[2-(2,3-Dimethylanilino)benzoyl]-1-piperazinyl}phenyl)-2-thiophencarboximidamid;
- 4-(4-{[Amino(2-thienyl)methyliden]amino}phenyl)-N-(4-phenoxyphenyl)butanamid;
- N-(4-{[Amino(2-thienyl)methyliden]amino}phenethyl)-4-(4-hydroxyphenoxy)benzamid;
- N-{4-[4-(10*H*-Phenothiazin-2-yloxy)butyl]phenyl}-2-thiophencarboximidamid;
- 3-[(3-{[Amino(2-thienyl)methyliden]amino}-benzyl)amino]-N-(4-anilinophenyl)propanamid;
- N'-(4-(2-[(10*H*-Phenothiazin-3-ylmethyl)amino]ethyl)phenyl)-2-thiophencarboximidamid;
- N-(4-{[Amino(2-thienyl)methyliden]amino}phenethyl)-2-methoxy-10*H*-phenothiazin-1-carboxamid;
- N'-[4-(2-{[(2-Methoxy-10*H*-phenothiazin-1-yl)methyl]amino}ethyl)phenyl]-2-thiophencarboximidamid;
- N'-{4-[(10*H*-Phenothiazin-2-yloxy)methyl]phenyl}-2-thiophencarboximidamid; oder eines ihrer Salze.

6. Produkt nach Anspruch 5, **dadurch gekennzeichnet, dass** es sich um eine der folgenden Verbindungen handelt:
- {4-{[2-Thienyl(imino)methyl]amino}phenoxy}-N-[4-(phenylamino)phenyl]-acetamid;
- 4-{[2-Thienyl(imino)methyl]amino}-N-[2-(phenylamio)phenyl]-benzenbutanamid;
- 4-{[2-Thienyl(imino)methyl]amino}-N-[4-(phenylamio)phenyl]-benzenbutanamid;
- 2-{4-{[2-Thienyl(imino)methyl]amino}phenyl}-ethyl-[4-(phenylamino)phenyl]carbamat;
- 4-{4-{[2-Thienyl(imino)methyl]amino}phenyl}-N-[4-(phenylamino)phenyl]-1-piperazinacetamid;
- 3-{[2-Thienyl(imino)methyl]amino}-N-[4-(phenylamino)phenyl]-benzenpropanamid;
- 4-(4-{[Amino(2-thienyl)methyliden]amino}phenyl)-N-[2-(4-toluidino)phenyl]butanamid;
- N'-{4-[4-(3-Anilinophenoxy)butyl]phenyl}-2-thiophencarboximidamid;
- 4-(4-{[Amino(2-thienyl)methyliden]amino}phenyl)-N-(9*H*-carbazol-3-yl)butanamid;
- N'-[4-(10*H*-Phenothiazin-2-yloxy)phenyl]-2-thiophencarboximidamid;
- 4-(4-{[Amino(2-thienyl)methyliden]amino}phenyl)-N-(10*H*-phenothiazin-3-yl)butanamid;
- N'-(4-{4-[2-(10*H*-Phenothiazin-2-yloxy)ethyl]-1-piperazinyl}phenyl)-2-thiophencarboximidamid;
- 4-(4-{[Amino(2-thienyl)methyliden]amino}phenyl)-N-(4-phenoxyphenyl)butanamid;
- 3-[(3-{[Amino(2-thienyl)methyliden]amino}-benzyl)amino]-N-(4-anilinophenyl)propanamid;
- N'-(4-{2-[(10*H*-Phenothiazin-3-ylmethyl)amino]ethyl}phenyl)-2-thiophencarboximidamid;
- N-(4-{[Amino(2-thienyl)methyliden]amino}phenethyl)-2-methoxy-10*H*-phenothiazin-1-carboxamid; oder eines ihrer Salze.

7. Produkt nach Anspruch 6, **dadurch gekennzeichnet, dass** es sich um eine der folgenden Verbindungen handelt:
- 4-{[2-Thienyl(imino)methyl]amino}-N-[2-(phenylamino)phenyl]-benzenbutanamid;
- 4-{[2-Thienyl(imino)methyl]amino}-N-[4-(phenylamino)phenyl]-benzenbutanamid;
- N'-[4-(10H-Phenothiazin-2-yloxy)phenyl]-2-thiophencarboximidamid;
- 4-(4-{[Amino(2-thienyl)methyliden]amino}phenyl)-N-(10H-phenothiazin-3-yl)butanamid;
- 3-[(3-{[Amino(2-thienyl)methyliden]amino}-benzyl)amino]-N-(4-anilinophenyl)propanamid;
- N'-(4-{2-[(10H-Phenothiazin-3-ylmethyl)amino]ethyl}phenyl)-2-thiophencarboximidamid;
oder eines ihrer Salze.

8. Die Verbindungen der allgemeinen Formel (IS)
A-X-Y-Φ-T **(IS)**
als neue Industrieprodukte, in der
A einen Rest 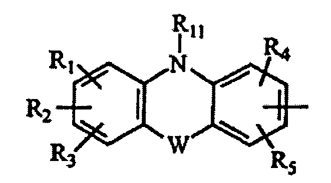 darstellt, in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen, einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
R₁₁ ein Wasserstoffatom, die OH-Gruppe, einen linearen oder verzweigten Alkyl- oder Alkoxyrest mit 1 bis 6 Kohlenstoffatomen oder einen Rest -COR₁₂ darstellt,
und R₁₂ ein Wasserstoffatom, die OH-Gruppe, einen linearen oder verzweigten Alkylrest mit 1 bis 6 Kohlenstoffatomen darstellt,
oder einen Rest 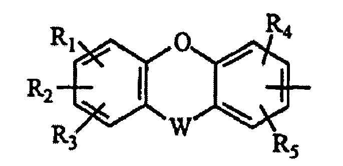 in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen oder einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
W nicht existiert oder eine Bindung oder O, S oder NR₁₅ darstellt, worin R₁₅ ein Wasserstoffatom oder einen linearen oder verzweigten Alkylrest mit 1 bis 6 Kohlenstoffatomen darstellt;
X eine Bindung oder einen Rest -(CH₂)ₖ-NR₁₆-, -O-, -S-, -CO-, -NR₁₆-CO-, -CO-NR₁₆-, -O-CO-, -CO-O-, -NR₁₆-CO-O-, -NR₁₆-CO-NR₁₇- darstellt,
wobei k 0 oder 1 darstellt;
Y eine Bindung oder einen Rest darstellt, der ausgewählt ist aus den Resten -(CH₂)ₘ-, -(CH₂)ₘ-O-(CH₂)ₙ-, -(CH₂)ₘ-S-(CH₂)ₙ-, -(CH₂)ₘ-NR₁₈-(CH₂)ₙ-, -(CH₂)ₘ-NR₁₈-CO-(CH₂)ₙ-, -(CH₂)ₘ-CO-NR₁₈-(CH₂)ₙ-, -(CH₂)ₘ-Q-(CH₂)ₙ-,
wobei Q Piperazin, Homopiperazin, 2-Methylpiperazin, 2,5-Dimethylpiperazin, 4-Oxypiperidin oder 4-Aminopiperidin darstellt,
wobei m und n ganze Zahlen von 0 bis 6 sind;
Φ einen Phenylenrest darstellt, der neben den beiden bereits in der allgemeinen Formel (I) dargestellten Ketten bis zu zwei Substituenten aufweisen kann, die aus einem Wasserstoffatom, einem Halogen, einer OH-Gruppe und einem linearen oder verzweigten Alkyl- oder Alkoxyrest mit 1 bis 6 Kohlenstoffatomen ausgewählt ist;
T NO₂ oder NH₂ darstellt;
R₁₆, R₁₇ und R₁₈ unabhängig voneinander ein Wasserstoffatom oder einen linearen oder verzweigen Alkylrest mit 1 bis 6 Kohlenstoffatomen darstellen.

9. Die Verbindungen der allgemeinen Formel (IS') 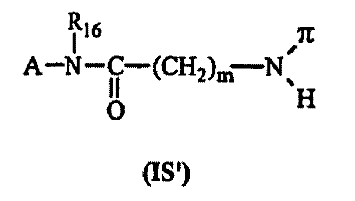 als neue Industrieprodukte, in der
A einen Rest 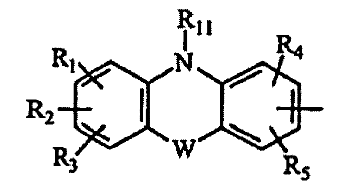 darstellt, in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen oder einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
R₁₁ ein Wasserstoffatom, die OH-Gruppe, einen linearen oder verzweigten Alkyl- oder Alkoxyrest mit 1 bis 6 Kohlenstoffatomen oder einen Rest -COR₁₂ darstellt,
und R₁₂ ein Wasserstoffatom, die OH-Gruppe, einen linearen oder verzweigten Alkylrest mit 1 bis 6 Kohlenstoffatomen darstellt,
oder einen Rest 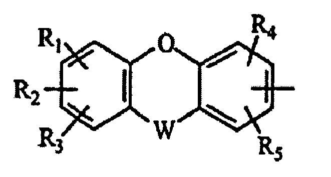 in dem R₁, R₂, R₃, R₄, R₅ unabhängig voneinander ein Wasserstoffatom, ein Halogen, die OH-Gruppe, einen linearen oder verzweigten Alkyl- oder Alkoxyrest mit 1 bis 6 Kohlenstoffatomen oder einen Rest Cyano, Nitro oder NR₆R₇ darstellen,
wobei R₆ und R₇ unabhängig voneinander ein Wasserstoffatom, die OH-Gruppe, einen linearen oder verzweigten Alkyl- oder Alkoxyrest mit 1 bis 6 Kohlenstoffatomen oder eine Gruppe -COR₈ darstellen,
R₈ ein Wasserstoffatom, die OH-Gruppe, einen linearen oder verzweigten Alkyl- oder Alkoxyrest mit 1 bis 6 Kohlenstoffatomen oder NR₉R₁₀ darstellt,
R₉ und R₁₀ unabhängig voneinander ein Wasserstoffatom, die OH-Gruppe oder einen linearen oder verzweigten Alkylrest mit 1 bis 6 Kohlenstoffatomen darstellen,
W nicht existiert oder eine Bindung oder O, S oder NR₁₅ darstellt, worin R₁₅ ein Wasserstoffatom oder einen linearen oder verzweigten Alkylrest mit 1 bis 6 Kohlenstoffatomen darstellt;
π ein Wasserstoffatom oder eine Schutzgruppe vom Carbamat-Typ darstellt;
R₁₆, R₁₇ und R₁₈ unabhängig voneinander ein Wasserstoffatom oder einen linearen oder verzweigten Alkylrest mit 1 bis 6 Kohlenstoffatomen darstellen;
und m eine ganze Zahl von 0 bis 6 darstellt.

10. Ein Produkt der allgemeinen Formel (I) nach einem der Ansprüche 1 bis 7 oder ein pharmazeutisch annehmbares Salz dieses Produkts als Medikament.

11. Pharmazeutische Zusammensetzung, die als Wirkstoff mindestens ein Produkt nach einem der Ansprüche 1 bis 7 oder ein pharmazeutisch annehmbares Salz dieses Produkts enthält.

12. Verwendung eines Produkts der allgemeinen Formel (I) nach einem der Ansprüche 1 bis 7 oder eines pharmazeutisch annehmbaren Salzes dieses Produkts zur Herstellung eines Medikaments, das zur Hemmung der NO-Synthase bestimmt ist.

13. Verwendung eines Produkts der allgemeinen Formel (I) nach einem der Ansprüche 1 bis 7 oder eines pharmazeutisch annehmbaren Salzes dieses Produkts zur Herstellung eines Medikaments, das zur Hemmung der Lipidperoxidation bestimmt ist.

14. Verwendung eines Produkts der allgemeinen Formel (I) nach einem der Ansprüche 1 bis 7 oder eines pharmazeutisch annehmbaren Salzes dieses Produkts zur Herstellung eines Medikaments, das gleichzeitig eine Wirkung der Hemmung der NO-Synthase und der Hemmung der Lipidperoxidation besitzt.
